# EUROPEAN PATENT APPLICATION

(11) **EP 4 574 982 A2**
(43) Date of publication of application: **25.06.2025**
(21) Application number: 25158938.8
(22) Date of filing: 27.11.2020
(51) Int. Cl.: C12N 15/87

(54) **RECOMBINANT POLYPEPTIDES FOR PROGRAMMING EXTRACELLULAR VESICLES**

(30) Priority: 28.11.2019 US 201962941768 P
(62) Divisional of application: 20893234.3
(71) Applicant: McMaster University, Hamilton, Ontario L8S 4L8 (CA); Ottawa Hospital Research Institute, Ottawa, Ontario K1H 8L6 (CA)
(72) Inventor: ILKOW, Carolina, Solange, Ottawa, K1G 5K9 (CA); BELL, John, Cameron, Ottawa, K1G 0K8 (CA); WHELAN, Jack, Timothy, Ottawa, K2P 0W7 (CA); CRUPI, Mathieu, François, Joseph, Ottawa, K1S 5V3 (CA); DUONG, Jessie, Nhan, Ottawa, K2J 5S2 (CA); LICHTY, Brian, Dennis, Brantford, N3T 5L9 (CA); ATHERTON, Matthew, John, Wynnewood, 19096 (US); WANG, Fuan, London, N6G 3R8 (CA); PAUMIER, Yoanna, Poutou, Gloucester, K1T 0E9 (CA); BOULTON, Stephen, Donald, Ottawa, K1B 4L7 (CA); MOHAMMADIAZAD, Mohammadtaha, Ottawa, K1K 2V7 (CA); SINGARAVELU, Ragunath, Ottawa, K1J 1C1 (CA)
(74) Representative: Schlich

(57) **Abstract**

Herein is provided a recombinant tumor-selective viral particle comprising a nucleic acid encoding a recombinant polypeptide for directing an extracellular vesicle (EV) to at least one target cell, said recombinant polypeptide comprising: at least one targeting moiety for directing said EV to said at least one target molecule expressed by said at least one target cell; at least one EV-anchoring polypeptide; and at least one intravesicular polypeptide. The viral particle may be from an oncolytic viruses. Recombinant polypeptides for programming EVs to target particular molecules are also provided. Also described are therapeutic EVs for delivering payload polypeptides (and/or cargo molecules) to target cells, e.g., in vaccine or cell-free "CAR-T"-like applications, along with EVs for recruiting immune cells to target cells in EV-mediated BiTE -like applications. Oncolytic viruses may also be engineered to infect tumor cells and shed programmed EVs, yielding additional therapeutic effects.

## Description

### CROSS REFERENCE TO RELATED APPLICATIONS

This application claims the benefit of priority of U.S. Provisional Patent Application No. 62/941,768 entitled "RECOMBINANT POLYPEPTIDES FOR PROGRAMMING EXTRACELLULAR VESICLES", which was filed November 28, 2019, and which is hereby incorporated by reference. All publications, patents, and patent applications mentioned in this specification and exhibits are herein incorporated by reference to the same extent as if each individual publication, patent, or patent application is specifically and individually indicated to be incorporated by reference.

### FIELD

The present disclosure relates generally delivery of molecules to cells. More particularly, the present disclosure relates to targeted delivery of molecules to cells.

### BACKGROUND

The targeted delivery of therapeutic molecules within biological systems is an important component of disease treatment. However, targeted delivery of molecules to specific cell types remains a challenge due to instability, off-target effects prior to reaching the target cell, toxicity of molecules in other contexts, as well as other issues.

It is, therefore, desirable to provide a stable targeted therapeutic molecule that only treats the target cell.

### SUMMARY

It is an object of the present disclosure to obviate or mitigate at least one disadvantage of previous approaches.

In a first aspect, the present disclosure provides a recombinant tumor-selective viral particle comprising a nucleic acid encoding a recombinant polypeptide for directing an extracellular vesicle (EV) to at least one target cell, said recombinant polypeptide comprising: at least one targeting moiety for directing said EV to said at least one target molecule expressed by said at least one target cell, at least one EV-anchoring polypeptide, and at least one intravesicular polypeptide.

In another aspect, there is provided a recombinant polypeptide for directing an extracellular vesicle (EV) to at least one target cell comprising: at least one targeting moiety for directing said EV to said at least one target molecule expressed by said at least one target cell, at least one EV-anchoring polypeptide, and at least one intravesicular polypeptide.

In one aspect, there is provided a nucleic acid molecule encoding the recombinant polypeptide as herein.

In one aspect, there is provided a vector comprising the nucleic acid as defined herein.

In one aspect, there is provided a recombinant viral genome comprising the nucleic acid as defined herein.

In one aspect, there is provided a viral particle comprising the nucleic acid as defined herein.

In one aspect, there is provided a host cell comprising the nucleic acid as defined, the vector, the recombinant viral genome, or the viral particle as defined herein.

In one aspect, there are provided targeted extracellular vesicles (EVs) comprising the recombinant polypeptide as defined herein.

In one aspect, there is provided a composition comprising the nucleic acid as defined herein, the vector as defined herein, the recombinant viral genome as defined herein, the viral particle as defined herein, or the targeted EVs as defined herein; together with a pharmaceutically acceptable excipient, diluent, or carrier.

In one aspect, there is provided a method of binding a targeting moiety to a target molecule of a target cell comprising contacting said target cell with the targeted EVs as defined herein.

In one aspect, there is provided a method of delivering a payload molecule to a target cell comprising contacting said target cell with the targeted EVs as defined herein.

In one aspect, there is provided a method of delivering a cargo molecule to a target cell comprising contacting said target cell with the targeted EVs as defined herein.

In one aspect, there is provided a method of stimulating an immune response to an antigen comprising administering to a subject the nucleic acid as defined herein, the vector as defined herein, the recombinant viral genome as defined herein, the viral particle as defined herein, or the targeted EVs as defined herein, wherein said target cell comprises an immune cell, and wherein said at least one EV therapeutic payload polypeptide comprises an antigen.

In one aspect, there is provided a method of killing target cells comprising administering to a subject the nucleic acid as defined herein, the vector as defined herein, the recombinant viral genome as defined herein, the viral particle as defined herein, or the targeted EVs as defined herein, wherein said at least one EV therapeutic payload polypeptide comprises a cytotoxic molecule.

In one aspect, there is provided a method of reprogramming immune cells comprising contacting the immune cells with the nucleic acid as defined herein, the vector as defined herein, the recombinant viral genome as defined herein, the viral particle as defined herein, or the targeted EVs as defined herein, wherein the at least one EV therapeutic payload molecule comprises an immunomodulatory molecule.

In one aspect, there is provided a method of directing an immune response to a target disease cell comprising administering to a subject the nucleic acid as defined herein, the vector as defined herein, the recombinant viral genome as defined herein, the viral particle as defined herein, or the targeted EVs as defined herein, wherein said recombinant polypeptide comprises at least two targeting moieties which specifically bind, respectively, to at least two different target molecules, wherein said at least two different target molecules are expressed, respectively, by an immune cell and a disease cell.

In one aspect, there is provided a method of preparing therapeutic targeted EVs for a subject comprising: contacting cells obtained from a subject with the nucleic acid as defined herein, the vector as defined herein, the recombinant viral genome as defined herein, or the viral particle as defined herein, and collecting the targeted EVs.

In one embodiment, there is provided a method of producing targeted EVs, wherein said method comprises expressing the nucleic acid as defined herein in cells, or culturing the host cell as defined herein to produce EVs; and collecting the EVs.

Other aspects and features of the present disclosure will become apparent to those ordinarily skilled in the art upon review of the following description of specific embodiments in conjunction with the accompanying figures.

### BRIEF DESCRIPTION OF THE DRAWINGS

Embodiments of the present disclosure will now be described, by way of example only, with reference to the attached Figures.
**Figure 1** depicts schematic cartoons of the different constructs contemplated by the present disclosure.
**Figure 2** depicts a schematic cartoon of a polypeptide embedded in an extracellular vesicle according to the present invention with a PD1 targeting moiety, LAMP2B transmembrane domain and HA-tag intravesicular polypeptide (panel A) and immunoblots showing successful expression of the polypeptide depicted in panel A from a vaccinia virus platform in whole cell lysates as well as isolated extracellular vesicles (panel B).
**Figure 3** depicts immunoblots showing that the topology of the PD1 targeting moiety in the polypeptide shown in Figure 2 is correct (externally oriented).
**Figure 4** depicts, in panel A, a schematic representation of the competitive binding ELISA experimental design performed to obtain the data in panel B, which shows that the PD1 moiety in the polypeptide as depicted in Figure 2 successfully binds to its PDL-1 receptor.
**Figure 5** depicts, in panel A, a timeline for the data represented in panel B, which shows that the construct of Figure 2, when virally expressed, successfully activates T cells.
**Figure 6** depicts, in panel A, a cartoon schematic showing a bispecific tetraspanin-based construct embedded in an EV targeting two different cell types (one cancer and one immune killing cell), and in panel B, depicts bright-field microscope images showing enhanced cell death when tumor cells are transfected with the construct of panel A and then combined with immune cells.
**Figure 7** depicts a Western blot showing the expression of six different constructs according to the present invention upon cell transfection.
**Figure 8** depicts Western blots showing the expression of five different constructs according to the present invention upon cell transfection.
**Figure 9** depicts a Western blot showing the proper expression of six different constructs according to the present invention upon cell transfection.
**Figure 10** depicts a Western blot showing the proper expression of four different constructs according to the present invention upon cell transfection.
**Figure 11** depicts a Western immunoblot of four different constructs targeted to DEC205 on dendritic cells, showing that small EVs, or targeted EVs, containing a CdaA payload activate the STING pathway in dendritic cells with DEC205 surface molecules.
**Figure 12** depicts a western immunoblot which shows that mouse fibroblast cells without DEC205 on the cell surface do not have the STING pathway activated when treated with small EVs, or targeted EVs containing construct of Figure 11 targeting DEC205 with a CdaA payload.
**Figure 13** depicts a western immunoblot which shows three constructs according to the present invention targeting DEC205 on dendritic cells, showing that the exosomes, or targeted EVs containing the CdaA payload activate the STING pathway in dendritic cells.
**Figure 14** depicts, in panel A, a Western blot showing the proper expression of two constructs according to the present invention, and in panel B, an immunofluorescence image showing expression of a construct according to the present invention targeting dendritic cells.
**Figure 15** depicts an immunofluorescence image showing cell expression of a construct according to the present invention targeting dendritic cells with a rotavirus antigen payload.
**Figure 16** depicts a cartoon schematic illustrating the mechanism of action of the mono-targeting programmed extracellular vesicles according to the present invention which carry a cytotoxic payload for immunotoxin-mediated cell death (apoptosis) as well as oncolysis (virus mediated cell death) for instances where the construct is expressed by a tumor-selective virus.
**Figure 17** depicts schematic drawings of chimeric fusion constructs with single chain variable fragment (scFv) targeting moieties targeting carcinoembryonic antigen (CEA) or carbonic anhydrase IX (CA9) with a VSVG transmembrane domain and mGZMB payload.
**Figure 18** depicts western blots showing proper expression of two constructs according to the present invention expressed from a plasmid or virus and enriched in isolated extracellular vesicles, or targeted EVs, in three different cell types.
**Figure 19A** depicts a bar graph showing reduced cell viability relative to a negative control for two constructs expressed from a vaccinia virus according to the present invention with cytotoxic payloads.
**Figure 19B** depicts bright-field microscope images showing increased cell death in cells that display the target surface molecule on the cell surface that are transfected with constructs targeting these cell surface molecules and carrying a cytotoxic payload according to the present invention.
**Figure 19C** depicts a bar graph showing reduced cell viability relative to a negative control for two constructs carrying cytotoxic payloads according to the present invention compared with untransfected cells, a construct lacking a targeting moiety and a construct lacking the cytotoxic payload.
**Figure 20** depicts a bar graph showing reduced cell viability relative to a negative control for two constructs carrying cytotoxic payloads according to the present invention in two different cell types.
**Figure 21** depicts a cartoon schematic showing the methodology for supernatant transfers.
**Figure 22** depicts bright-field microscope images of cells in a supernatant transfer experiment showing cell death only in MC38-cells which receive the supernatant (extracellular vesicle fraction) containing the construct targeting MC38-cells.
**Figure 23** depicts bright-field microscope images of cells in a supernatant transfer experiment showing cell death only in cells that display the targeted cell surface molecule when treated with supernatants from cells infected by viruses expressing constructs according to the present invention.
**Figure 24** depicts immunofluorescence microscope images of cells in a supernatant transfer experiment showing expression of a construct according to the present invention targeting hCEA with an mCherry payload, showing in the top panel, that CEA negative cells infected with a virus expressing the construct expresses the construct, and that only cells with the CEA target surface marker and which undergo the supernatant transfer in the two lower panels uptake the construct targeting this cell surface molecule.
**Figure 25** depicts a cartoon schematic providing an overview of one aspect of the present invention for producing programmed extracellular vesicles according to the present invention from producer cell lines in vivo, in situ or in vitro.
**Figure 26** depicts cartoon schematics illustrating five different constructs according to the present invention with a tetraspanin-based transmembrane domain and carrying a cytotoxic payload.
**Figure 27** depicts western blots showing successful expression of the five constructs illustrated in Figure 26.
**Figure 28** depicts cartoon schematics illustrating seven different constructs according to the present invention with a CD63 tetraspanin-based transmembrane domain targeting CD19 and CD20 with one payload for six of the illustrated constructs, and two payloads and a Furin cleavage site for one of the seven illustrated constructs.
**Figure 29A** depicts a Western blot showing that a construct according to the present invention with a CTX targeting moiety, VSVG transmembrane domain and Nanoluc^{™} payload is successfully expressed from a plasmid transfected in HEK293T cells.
**Figure 29B** depicts a bar graph showing luminescence in a supernatant transfer experiment where fluorescence is only seen in supernatants of cells expressing construct as shown in Figure 29A.
**Figure 29C** depicts a bar graph showing the supernatants of Figure 29B transferred to six different human glioblastoma cell lines, with luminescence in cells being relative to the proportion of target cell surface marker displayed outside the treated cells and only in constructs which include the appropriate targeting moiety.
**Figure 30** depicts a graph showing that 786-0 cancer cells infected with vaccinia virus (VacV) produce more EVs than uninfected cells.
**Figure 31** depicts Western blots that show that several cancer cell types infected with Vaccinia virus produce more EVs than uninfected cells.
**Figure 32** depicts Western blots showing the expression of four different constructs according to the present invention upon cell transfection and in isolated EV fractions.
**Figure 33** depicts a Western blot showing the expression of a construct according to the present invention upon cell transfection and in isolated EV fractions.
**Figure 34** depicts Western blots showing the expression of four different constructs according to the present invention upon cell transfection and in isolated EV fractions.
**Figure 35A** shows fluorescence microscopy images showing that plasmids containing LDLRT(LDLR-targeting)-VSV- fused to an RNA binding motif can package mRNA coding for blue fluorescent protein (BFP) fused to Nanoluc^{™} (Nluc) and deliver it to recipient cells positive for the LDLR target.
**Figure 35B** Quantitative read-out of the same experiment as 35A with measured Nanoluc^{™} activity in the recipient cells.
**Figure 36** depicts a graphical representation of the cellular viability of fibroblast-activating protein (FAP)-positive pancreatic fibroblasts (PanFib), pancreatic cancer cells (BxPC3), and patient samples (P025, P032) treated with aFAP-VSVG-mGZMB EVs.
**Figure 37** depicts a Western immunoblot showing that PEVs expressing aDEC205-mCD63-CdaA, which targets to DEC205 on dendritic cells and contains a CdaA payload, is capable of activating the STING pathway in dendritic cells.
**Figure 38** depicts a Western immunoblot of three different constructs targeted to MARCO on macrophages, showing that small EVs, or targeted EVs containing a CdaA payload activate the STING pathway in bone-marrow derived macrophages expressing MACRO on their cell surface.
**Figure 39** depicts an assay demonstrating the potency of anti-Marco-linked CdaA PEV constructs in stimulating the Interferon (IFN) signaling pathway.
**Figure 40** depicts a histogram demonstrating increased cell killing of MC38 cells by murine splenocytes (10:1 splenocytes:MC38) in the presence of EVs with αCD3 constructs compared to mock controls.
**Figure 41** depicts a bar graph of the results of a vaccination experiment with naïve EVs, or EVs decorated with aDEC205-VSVGTM-OVA or both aDEC205-CD63D-CdaA-Flag and aDEC205-VSVGTM-OVA demonstrating that a combination of both dendritic cell-targeted antigen [e.g. Ovalbumin (OVA)] and immune adjuvant (e.g. CdaA enzyme) induces immune responses *in vivo.*
**Figure 42** depicts an assay showing dendritic cell-directed PEV constructs that act as immune adjuvants by stimulating the Interferon response

### DETAILED DESCRIPTION

Generally, the present disclosure provides a recombinant tumor-selective viral particle comprising a nucleic acid encoding a recombinant polypeptide for directing an extracellular vesicle (EV) to at least one target cell, said recombinant polypeptide comprising: at least one targeting moiety for directing said EV to said at least one target molecule expressed by said at least one target cell; at least one EV-anchoring polypeptide; and at least one intravesicular polypeptide. The viral particle may be from an oncolytic virus. Also provided is a recombinant polypeptide for directing an extracellular vesicle (EV) to at least one target cell comprising: at least one targeting moiety for directing said EV to said at least one target molecule expressed by said at least one target cell, at least one EV-anchoring polypeptide, and at least one intravesicular polypeptide.

### Viral Particles Comprising EV-Programming Recombinant Polypeptides

In one aspect, there is provided a recombinant tumor-selective viral particle comprising a nucleic acid encoding a recombinant polypeptide for directing an extracellular vesicle (EV) to at least one target cell, said recombinant polypeptide comprising:
- at least one targeting moiety for directing said EV to said at least one target molecule expressed by said at least one target cell,
- at least one EV-anchoring polypeptide, and
- at least one intravesicular polypeptide.

In one embodiment, the recombinant tumor-selective viral particle is of an oncolytic virus.

### Recombinant Polypeptides

In one aspect, there is provided a recombinant polypeptide for directing an extracellular vesicle (EV) to at least one target cell comprising:
- at least one targeting moiety for directing said EV to said at least one target molecule expressed by said at least one target cell,
- at least one EV-anchoring polypeptide, and
- at least one intravesicular polypeptide.

### "Monospecific" Single Transmembrane (TM) Domain Constructs

In one embodiment, said at least one EV-anchoring polypeptide comprises an EV-directed transmembrane polypeptide linked to said at least one targeting moiety.

In one embodiment, said EV-directed transmembrane polypeptide comprises a transmembrane domain from LAMP2b, VSVG, CD81, CD82,or LAMP1,.

In one embodiment, said EV-directed transmembrane polypeptide comprises a transmembrane domain from Junin virus glycoprotein, Lassa fever virus glycoprotein, LCMV (lymphocytic choriomeningitis virus) glycoprotein, SARS-CoV-2 glycoprotein, Tamiami virus glycoprotein, Guanarito virus glycoprotein, Paraná virus glycoprotein, Machupo virus glycoprotein, Sabia virus glycoprotein, or CdaA.

In one embodiment, the EV-directed transmembrane polypeptide comprises a transmembrane domain from a Rhabdovirus glycoprotein.

In one embodiment, the EV-directed transmembrane polypeptide comprises a transmembrane domain from a Arenavirus glycoprotein.

In one embodiment, said at least one target cell comprises a mammalian cell.

In one embodiment, said mammalian cell is a human cell.

In one embodiment, said at least one target cell is a tumor cell, a tumor stromal cell, or an immune cell.

In one embodiment, said tumor stromal cell comprises a cancer-associated fibroblast.

In one embodiment, said immune cell is a T-cell, a B-cell, a natural killer (NK) cell, a dendritic cell, a macrophage, or a neutrophil.

In one embodiment, said immune cell is a macrophage. In one embodiment, said at least one target molecule is macrophage receptor (MARCO).

In one embodiment, said T-cell is a regulatory T-cell or a cytotoxic T cell.

In one embodiment, said at least one target molecule is a cell surface marker or a cell surface receptor.

In one embodiment, said at least one target molecule is a TNF-α family receptor, an integrin, a C-type lectin receptor, a leptin, a carcinoembryonic antigen, a CD antigen, a carbonic anhydrase, FAP, MMP2, DEC205, DC40, CLEC9, CD3, a glycosaminoglycan, a polysaccharide, or a lipid.

In one embodiment, said at least one target molecule comprises a disease-specific cell surface molecule, which is:
- expressed by a disease cell and not by a healthy control cell, or
- expressed in a greater quantity by said disease cell compared to said healthy control cell.

In one embodiment, said disease-specific cell surface molecule comprises a tumor-associated antigen.

In one embodiment, said at least one target molecule comprises DEC205, CLEC9A, CEACAM5, CTLA4, CD3, CD7, CD11c, CD19, CD20, CD22, CD40, CD44, CD206, EGFR, fibroblast activating protein (FAP), CA9, MMP-2, PD-L1, SIRPa, chondroitin sulfate, αv-Integrin, or folate receptor.

In one embodiment, said at least one targeting moiety comprises a receptor ligand, an antibody or a functional fragment thereof, an scFv, a single domain antibody or a DARPin.

In one embodiment, said antibody is a single domain antibody.

In one embodiment, said antibody is a humanized antibody.

In one embodiment, said functional fragment is a Fab' or a F(ab')2.

In one embodiment, said at least one targeting moiety comprises anti-DEC205, anti-Clec9A, anti-FAP, anti-CEA, anti-CA9, anti-CTL4, anti-CD3, anti-CD206, anti-CD19, anti-CD20, anti-CD22, anti-CD44, anti-CD7, SIRPα ectodomain, GE11 peptide, CTX, VAR2Δ,CD40 ligand, CD40-targeting peptide, iRGD, PD1.

In one embodiment, said intravesicular polypeptide may comprise a short amino acid tail for projecting into the intravesicular space. Said intravesicular polypeptide may comprise at least 9 amino acids. Said intravesicular polypeptide may comprise at least 10 amino acids. Said intravesicular polypeptide may comprise at least 11 amino acids. Said intravesicular polypeptide may comprise at least 12 amino acids. Said intravesicular polypeptide may comprise at least 13 amino acids. Said intravesicular polypeptide may comprise at least 14 amino acids. Said intravesicular polypeptide may comprise at least 15 amino acids. Said intravesicular polypeptide may comprise at least 9 amino acids. Said intravesicular polypeptide may comprise 9 to 15 amino acids.

In one embodiment, said intravesicular polypeptide comprises least one EV payload polypeptide linked to said at least one targeting moiety via said EV-anchoring polypeptide. The EV payload polypeptide may comprise, for example, a therapeutic polypeptide, a polypeptide for imaging, a polypeptide for diagnostics, a suicide protein, or a receptor for a biomarker.

In one embodiment, the at least one EV payload polypeptide comprises at least one EV therapeutic payload polypeptide.

### "Monospecific" Tetraspanin Constructs

In one embodiment, said EV-anchoring polypeptide and said intravesicular polypeptide together comprise an EV-directed recombinant tetraspanin comprising said at least one targeting moiety inserted between two transmembrane domains thereof.

In one embodiment, said recombinant tetraspanin comprises or is derived from human CD63 or CD9.

In one embodiment, said at least one target cell comprises a mammalian cell.

In one embodiment, said mammalian cell is a human cell.

In one embodiment, said at least one target cell is a tumor cell, a tumor stromal cell, or an immune cell.

In one embodiment, said tumor stromal cell comprises a cancer-associated fibroblast.

In one embodiment, said immune cell is a T-cell, a B-cell, a natural killer (NK) cell, a dendritic cell, a macrophage, or a neutrophil.

In one embodiment, said immune cell is a macrophage. In one embodiment, said at least one target molecule is macrophage receptor (MARCO).

In one embodiment, said T-cell is a regulatory T-cell or a cytotoxic T cell.

In one embodiment, said at least one target molecule is a cell surface marker or a cell surface receptor.

In one embodiment, said at least one target molecule is a TNF-α family receptor, an integrin, a C-type lectin receptor, a leptin, a carcinoembryonic antigen, a CD antigen, a carbonic anhydrase, FAP, MMP2, DEC205, DC40, CLEC9, CD3, a glycosaminoglycan, a polysaccharide, or a lipid.

In one embodiment, said at least one target molecule comprises a disease-specific cell surface molecule, which is:
- expressed by a disease cell and not by a healthy control cell, or
- expressed in a greater quantity by said disease cell compared to said healthy control cell.

In one embodiment, said disease-specific cell surface molecule comprises a tumor-associated antigen.

In one embodiment, said at least one target molecule comprises DEC205, CLEC9A, CEACAM5, CTLA4, CD3, CD7, CD11c, CD19, CD20, CD22, CD40, CD44, CD206, EGFR, fibroblast activating protein (FAP), CA9, MMP-2, PD-L1, SIRPa, chondroitin sulfate, αv-Integrin, or folate receptor.

In one embodiment, said at least one targeting moiety comprises a receptor ligand, an antibody or a functional fragment thereof, an scFv, a single domain antibody. or a DARPin.

In one embodiment, said antibody is a single domain antibody.

In one embodiment, said antibody is a humanized antibody.

In one embodiment, said functional fragment is a Fab' or a F(ab')2.

In one embodiment, said at least one targeting moiety comprises anti-DEC205, anti-Clec9A, anti-FAP, anti-CEA, anti-CA9, anti-CTL4, anti-CD3, anti-CD206, anti- anti-CD19, anti-CD20, anti-CD22, anti-CD44, anti-CD7, SIRPα ectodomain, GE11 peptide, CTX, VAR2Δ,CD40 ligand, CD40-targeting peptide, iRGD, PD1.

In one embodiment, the recombinant polypeptide further comprises at least one EV payload polypeptide linked to an N- and/or C-terminus of said recombinant tetraspanin. The EV payload polypeptide may comprise, for example, a therapeutic polypeptide, a polypeptide for imaging, a polypeptide for diagnostics, a suicide protein, or a receptor for a biomarker.

In one embodiment, the at least one EV payload polypeptide comprises at least one EV therapeutic polypeptide.

### "Bispecific" Tetraspanin Constructs

In one embodiment, said at least one targeting moiety comprises at least two targeting moieties, wherein said EV-anchoring polypeptide and said intravesicular polypeptide together comprise an EV-directed recombinant tetraspanin comprising four transmembrane domains numbered 1, 2, 3, and 4 from N- to C-terminus, wherein a first of said two targeting moieties is inserted between transmembrane domains 1 and 2, and a second of said two targeting moieties is inserted between transmembrane domains 3 and 4.

In one embodiment, said EV-directed recombinant tetraspanin is derived from human CD63 or CD9.

In one embodiment, said at least two targeting moieties specifically bind to at least two different target molecules.

### Targeting the Same Target Cell

In one embodiment, said at least two different target molecules are expressed by the same target cell.

In one embodiment, said target cell comprises a mammalian cell.

In one embodiment, said mammalian cell comprises a human cell.

In one embodiment, said target cell comprises a tumor cell, a tumor stromal cell, or an immune cell.

In one embodiment, said tumor stromal cell comprises a cancer-associated fibroblast.

In one embodiment, said immune cell is a T-cell, a B-cell, a natural killer (NK) cell, a dendritic cell, a macrophage, or a neutrophil.

In one embodiment, said immune cell is a macrophage. In one embodiment, said at least one target molecule is macrophage receptor (MARCO).

In one embodiment, said T-cell is a regulatory T cell or a cytotoxic T cell.

In one embodiment, each of said at least two target molecules is a cell surface molecule.

In one embodiment, each of said at least two target molecules is a TNF-α family receptor, an integrin, a C-type lectin receptor, a leptin, a carcinoembryonic antigen, a CD antigen, a carbonic anhydrase, FAP, MMP2, DEC205, DC40, CLEC9, CD3, a glycosaminoglycan, a polysaccharide, or a lipid.

In one embodiment, each of said at least two target molecules comprise a disease-specific cell surface molecule, which is:
- expressed by a disease cell and not by a healthy control cell, or
- expressed in a greater quantity by said disease cell compared to said healthy control cell.

In one embodiment, said disease-specific cell surface molecule comprises a tumor-associated antigen.

In one embodiment, each of said at least two target molecules independently comprises DEC205, CLEC9A, CEACAM5, CTLA4, CD3, CD7, CD11c, CD19, CD20, CD22, CD40, CD44, CD206, EGFR, fibroblast activating protein (FAP), CA9, MMP-2, PD-L1, SIRPa, chondroitin sulfate, αv-Integrin, or folate receptor.

In one embodiment, each of said at least two targeting moieties independently comprises a receptor ligand, an antibody or a functional fragment thereof, an scFv, a single domain antibody or a DARPin.

In one embodiment, said antibody is a single domain antibody.

In one embodiment, said antibody is a humanized antibody.

In one embodiment, said functional fragment is a Fab' or a F(ab')2.

In one embodiment, said at least one targeting moiety comprises anti-DEC205, anti-Clec9A, anti-FAP, anti-CEA, anti-CA9, anti-CTL4, anti-CD3, anti-CD206, anti-CD19, anti-CD20, anti-CD22, anti-CD44, anti-CD7, SIRPα ectodomain, GE11 peptide, CTX, VAR2Δ,CD40 ligand, CD40-targeting peptide, iRGD, PD1.

### Targeting Different Target Cells

In one embodiment, said at least two different target molecules are expressed by different target cells.

In one embodiment, said different target cells comprise a disease cell and an immune cell, and wherein said at least two targeting moieties are directed, respectively, to a disease cell surface molecule and an immune cell surface molecule.

In one embodiment, said different target cells comprise a tumor cell and an immune cell, and wherein said at least two targeting moieties are directed, respectively, to a tumor cell surface molecule and an immune cell surface molecule.

In one embodiment, said immune cell is a T cell, and said immune cell surface marker is a T cell surface molecule.

In one embodiment, said T cell is a regulatory T cell or a cytotoxic T cell.

In one embodiment, said immune cell is a natural killer (NK) cell, and said immune cell surface marker is an NK cell surface molecule.

In one embodiment, said immune cell is a B cell, and said immune cell surface marker is a B cell surface molecule.

In one embodiment, said immune cell is a macrophage, and said immune cell surface marker is a macrophage cell surface molecule. In one embodiment, said at least one target molecule is macrophage receptor (MARCO).

In one embodiment, said immune cell is a dendritic cell, and said immune cell an surface marker is a dendritic cell surface molecule.

In one embodiment, said immune cell is a neutrophil, and said immune cell surface marker is a neutrophil cell surface molecule.

In one embodiment, said tumor cell surface molecule comprises a tumor-associated antigen.

In one embodiment, said tumor cell surface molecule comprises one of CEACAM5, CD19, CD20, CD22, EGFR, fibroblast activating protein (FAP), CA9, MMP-2, PD-L1, SIRPα, chondroitin sulfate, αv-Integrin, or folate receptor.

In one embodiment, said at least one targeting moiety comprises an antibody or a functional fragment thereof, an scFv, a single domain antibody or a DARPin.

In one embodiment, said antibody is a single domain antibody.

In one embodiment, said antibody is a humanized antibody.

In one embodiment, said functional fragment is a Fab' or a F(ab')2.

In one embodiment, the recombinant polypeptide further comprising at least one EV payload polypeptide. The EV payload polypeptide may comprise, for example, a therapeutic polypeptide, a polypeptide for imaging, a polypeptide for diagnostics, a suicide protein, or a receptor for a biomarker.

In one embodiment, the at least one EV payload polypeptide comprises at least one EV therapeutic payload polypeptide.

In one embodiment, said EV therapeutic payload polypeptide is linked to said N- and/or C-terminus of said recombinant tetraspanin.

### Payloads

These payloads are intended to embodiment described herein, as appropriate.

In one embodiment, said at least one EV payload polypeptide is linked via a cleavage site for releasing said at least one EV payload polypeptide.

In one embodiment, said at least one EV therapeutic payload polypeptide is linked via a cleavage site for releasing said at least one EV therapeutic payload polypeptide.

In one embodiment, said cleavage site comprises a self-cleavage peptide, a pH-dependent cleavage site, or a site for enzymatic cleavage.

In one embodiment, said EV therapeutic payload polypeptide comprises an active pharmaceutical ingredient (API).

In one embodiment, said EV therapeutic payload polypeptide comprises a cytotoxic molecule.

In one embodiment, said cytotoxic molecule comprises human GZMB R201K, murine GZMB, diphtheria toxin, a PE38 domain from Pseudomonas exotoxin A, or human TRAIL.

In one embodiment, said payload polypeptide comprises an immunomodulatory molecule.

In one embodiment, the immunomodulatory molecule comprises an enzyme that generates an immunogenic molecule.

In one embodiment, said immunomodulatory molecular comprises a STING or ERAdP pathway activator.

In one embodiment, said STING or ERAdP pathway activator comprises a bacterial dinucleotide cyclase.

In one embodiment, said bacterial dinucleotide cyclase comprises CdaA.

In one embodiment, said payload polypeptide comprises an enzyme.

In one embodiment, said payload polypeptide comprises a nucleic acid-binding domain.

In one embodiment, said nucleic acid binding domain comprises an RNA-binding motif.

In one embodiment, the nucleic acid binding domain comprises an RNA binding motif from a Cas13 family member protein. In one embodiment, the RNA binding motif comprises an RNA binding motif from Cas13a. In one embodiment, the RNA binding motif comprises an RNA binding motif from Cas13b. In one embodiment, the RNA binding motif comprises an RNA binding motif from Cas13d.

In one embodiment, the RNA binding motif comprises an RNA binding motif from Pum (Pumilio-homology domain-1).

In one embodiment, the RNA binding motif comprises an RNA binding motif from Stu1 (Staufen-1).

In one embodiment, the RNA binding motif comprises an RNA binding motif from alphavirus capsid protein L72AE.

In one embodiment, the nucleic acid binding comprises an RNA binding motif from the MS2 coat protein (herein "MS2").

In one embodiment, the RNA binding motif comprises an RNA binding motif from VEEV capsid protein.

In one embodiment, said RNA binding motif comprises a nucleic acid ligand system.

In one embodiment, said payload polypeptide comprises an antigen.

In one embodiment, said antigen is a tumor-associated antigen.

In one embodiment, said antigen is from a pathogen.

In one embodiment, said EV therapeutic payload polypeptide further comprises an adjuvant.

In one embodiment, said adjuvant comprises a STING or ERAdP pathway activator.

In one embodiment, said STING or ERAdP pathway activator comprises a bacterial dinucleotide cyclase.

In one embodiment, said bacterial dinucleotide cyclase comprises CdaA.

In one embodiment, said at least one EV therapeutic payload polypeptide is linked to at least one further EV payload polypeptide.

In one embodiment, said at least one EV therapeutic payload polypeptide is linked to said at least one further EV payload polypeptide by a cleavage site.

In one embodiment, said at least one EV therapeutic payload polypeptide is separated from said at least one further EV payload polypeptide by at least two EV transmembrane domains.

### Nucleic Acid Molecules

In one aspect, there is provided a nucleic acid molecule encoding the recombinant polypeptide as herein.

In one embodiment, said nucleic acid further encodes a separate EV cargo molecule.

In one embodiment, said EV cargo molecule comprises a nucleic acid.

In one embodiment, said nucleic acid comprises an RNA.

In one embodiment, the RNA comprises a target sequence from a sequence in Table 12. In one embodiment, the recombinant polypeptide comprises an RNA binding motif from a sequence in Table 12 corresponding to the target sequence of the cargo.

In one embodiment, said RNA comprises an mRNA, an miRNA, or an shRNA.

In one embodiment, said EV cargo molecule comprises a polypeptide.

In one aspect, there is provided a nucleic acid molecule encoding the recombinant polypeptide as defined herein and comprising the EV payload as defined herein.

In one aspect, there is provided a nucleic acid molecule encoding the recombinant polypeptide as defined herein and comprising the EV therapeutic payload as defined herein.

In one embodiment, said nucleic acid further encodes a separate EV cargo molecule.

In one embodiment, said EV cargo molecule comprises a nucleic acid.

In one embodiment, said nucleic acid comprises an RNA.

In one embodiment, said RNA comprises an mRNA, an miRNA, or an shRNA.

In one embodiment, said EV cargo molecule comprises a polypeptide.

### Vectors

In one aspect, there is provided a vector comprising the nucleic acid as defined herein.

In one aspect, there is provided a vector comprising the nucleic acid as defined herein, wherein the recombinant polypeptide comprises an EV therapeutic payload.

### Recombinant Viral Genomes

In one aspect, there is provided a recombinant viral genome comprising the nucleic acid as defined herein.

In one embodiment, the viral genome is from a Lentivirus, the Tian Tan strain of Vaccinia virus, or Adeno-associated Virus (AAV).

In one embodiment, the viral genome is from a virus that is tumor-selective.

In one embodiment, said viral genome is from an oncolytic virus.

In one embodiment, said oncolytic virus is vesicular stomatitis virus (VSV), Vaccinia virus, Herpes virus simplex 1 (HSV-1), Herpes virus 2 (HSV-2), adenovirus.

In one aspect, there is provided a recombinant viral genome comprising the nucleic acid as defined herein, wherein the recombinant polypeptide comprises an EV therapeutic payload.

In one embodiment, the viral genome is from a virus that is tumor-selective.

In one embodiment, said viral genome is from an oncolytic virus.

In one embodiment, said oncolytic virus is vesicular stomatitis virus (VSV), Vaccinia virus, Herpes virus simplex 1 (HSV-1), Herpes virus 2 (HSV-2), adenovirus.

### Viral Particles

In one aspect, there is provided a viral particle comprising the nucleic acid as defined herein.

In one embodiment, said viral particle is of a Lentivirus, the Tian Tan strain of Vaccinia virus, or Adeno-associated Virus (AAV).

In one embodiment, said viral particle is of a virus that is tumor-selective.

In one embodiment, said viral particle is of an oncolytic virus.

In one embodiment, said oncolytic virus is vesicular stomatitis virus (VSV), Vaccinia virus, Herpes virus simplex 1 (HSV-1), Herpes virus 2 (HSV-2), adenovirus.

In one aspect, there is provided a viral particle comprising the nucleic acid as defined herein, wherein the recombinant polypeptide comprises an EV therapeutic payload.

In one embodiment, said viral particle is of a virus that is tumor-selective.

In one embodiment, said viral particle is of an oncolytic virus.

In one embodiment, said oncolytic virus is vesicular stomatitis virus (VSV), Vaccinia virus, Herpes virus simplex 1 (HSV-1), Herpes virus 2 (HSV-2), adenovirus.

### Host Cells

In one aspect, there is provided a host cell comprising the nucleic acid as defined, the vector, the recombinant viral genome, or the viral particle as defined herein.

In one embodiment, the host cell is a prokaryotic cell.

In one embodiment, the host cell is a eukaryotic cell.

In one embodiment, the host cell is a yeast cell or an insect cell.

In one embodiment, the host cell is a mammalian cell.

In one embodiment, the host cell is a human cell.

In one embodiment, the host cell is an immune cell.

In one embodiment, the host cell is a B cell, a T cell, a dendritic cell, a macrophage or a neutrophil.

In one embodiment, the host cell is a regulatory T cell or a cytotoxic T cell.

In one embodiment, said host cell further encodes a separate EV cargo molecule.

In one embodiment, said EV cargo molecule comprises a nucleic acid.

In one embodiment, said nucleic acid comprises an RNA.

In one embodiment, the nucleic acid binding domain comprises an RNA binding motif from a Cas13 family member protein. In one embodiment, the RNA binding motif comprises an RNA binding motif from Cas13a. In one embodiment, the RNA binding motif comprises an RNA binding motif from Cas13b. In one embodiment, the RNA binding motif comprises an RNA binding motif from Cas13d.

In one embodiment, the RNA binding motif comprises an RNA binding motif from Pum (Pumilio-homology domain-1).

In one embodiment, the RNA binding motif comprises an RNA binding motif from Stu1 (Staufen-1).

In one embodiment, the RNA binding motif comprises an RNA binding motif from alphavirus capsid protein L72AE.

In one embodiment, the nucleic acid binding comprises an RNA binding motif from the MS2 coat protein (herein "MS2").

In one embodiment, the RNA binding motif comprises an RNA binding motif from VEEV capsid protein.

In one embodiment, the recombinant polypeptide comprises one of the above-described RNA binding motifs and the EV cargo comprise a cognate RNA target sequence for the RNA binding motif. Examples of RNA binding motifs and cognate target sequences are provided in the sequences of Table 12.

In one embodiment, said RNA comprises an mRNA, an miRNA, or an shRNA.

In one embodiment, said EV cargo molecule comprises a polypeptide.

In one aspect, there is provided a host cell comprising the nucleic acid as defined, the vector, the recombinant viral genome, or the viral particle as defined herein, wherein the recombinant polypeptide comprises an EV therapeutic payload.

In one embodiment, the host cell is a prokaryotic cell.

In one embodiment, the host cell is a eukaryotic cell.

In one embodiment, the host cell is a yeast cell or an insect cell.

In one embodiment, the host cell is a mammalian cell.

In one embodiment, the host cell is a human cell.

In one embodiment, the host cell is an immune cell.

In one embodiment, the host cell is a B cell, a T cell, a dendritic cell, a macrophage or a neutrophil.

In one embodiment, the host cell is a regulatory T cell or a cytotoxic T cell.

In one embodiment, said host cell further encodes a separate EV cargo molecule.

In one embodiment, said EV cargo molecule comprises a nucleic acid.

In one embodiment, said nucleic acid comprises an RNA.

In one embodiment, said RNA comprises an mRNA, an miRNA, or an shRNA.

In one embodiment, said EV cargo molecule comprises a polypeptide.

### Targeted Extracellular Vesicles (EVs)

In one aspect, there are provided targeted extracellular vesicles (EVs) comprising the recombinant polypeptide as defined herein.

In one embodiment, the targeted EVs further comprise a separate EV cargo molecule.

In one embodiment, said EV cargo molecule comprises a nucleic acid.

In one embodiment, said nucleic acid comprises an RNA.

In one embodiment, the RNA comprises a target sequence from a sequence in Table 12. In one embodiment, the recombinant polypeptide comprises an RNA binding motif from a sequence in Table 12 corresponding to the target sequence of the cargo.

In one embodiment, said RNA comprises an mRNA, an miRNA, or an shRNA.

In one embodiment, said EV cargo molecule comprises a polypeptide.

In one embodiment, said EV cargo molecule comprises an API.

In one embodiment, the targeted EVS are exosomes.

In one embodiment, the targeted EVS are microvesicles.

In one embodiment, the targeted EVS are ectosomes.

In one embodiment, the targeted EVS are apoptotic bodies.

In one embodiment, the targeted EVS are virus-like particles.

In one embodiment, the targeted EVS are macrovesicles.

In one embodiment, the targeted EVS are oncosomes.

In one embodiment, the targeted EVS are gesicles.

In one aspect, there are provided targeted extracellular vesicles (EVs) comprising the recombinant polypeptide as defined herein, wherein the recombinant polypeptide comprises an EV therapeutic payload.

In one embodiment, the targeted EVs further comprise a separate EV cargo molecule.

In one embodiment, said EV cargo molecule comprises a nucleic acid.

In one embodiment, said nucleic acid comprises an RNA.

In one embodiment, said RNA comprises an mRNA, an miRNA, or an shRNA.

In one embodiment, said EV cargo molecule comprises a polypeptide.

In one embodiment, said EV cargo molecule comprises an API.

In one embodiment, the targeted EVS are exosomes.

In one embodiment, the targeted EVS are microvesicles.

In one embodiment, the targeted EVS are ectosomes.

In one embodiment, the targeted EVS are apoptotic bodies.

### Pharmaceutical Compositions

In one aspect, there is provided a composition comprising the nucleic acid as defined herein, the vector as defined herein, the recombinant viral genome as defined herein, the viral particle as defined herein, or the targeted EVs as defined herein; together with a pharmaceutically acceptable excipient, diluent, or carrier.

In one aspect, there is provided a composition comprising the nucleic acid as defined herein, the vector as defined herein, the recombinant viral genome as defined herein, the viral particle as defined herein, or the targeted EVs as defined herein; together with a pharmaceutically acceptable excipient, diluent, or carrier, wherein the recombinant polypeptide comprises an EV therapeutic payload.

### Methods and Uses

### Binding a Target

In one aspect, there is provided a method of binding a targeting moiety to a target molecule of a target cell comprising contacting said target cell with the targeted EVs as defined herein.

In one aspect, there is provided a use of the targeted EVs as defined herein for binding a targeting moiety to a target molecule of a target cell.

In one aspect, there are provided the targeted EVs as defined herein for use in binding a targeting moiety to a target molecule of a target cell.

### Delivering a Payload

In one aspect, there is provided a method of delivering a payload molecule to a target cell comprising contacting said target cell with the targeted EVs as defined herein.

In one aspect, there is provided a use of the targeted EVs as defined herein for delivering a payload molecule to a target cell.

In one aspect, there are provided the EVs as defined herein for use in delivering a payload molecule to a target cell.

### Delivering a Cargo

In one aspect, there is provided a method of delivering a cargo molecule to a target cell comprising contacting said target cell with the targeted EVs as defined herein.

In one aspect, there is provided a use of the targeted EVs as defined herein for delivering a cargo molecule to a target cell.

In one aspect, there are provided the targeted EVs as defined herein for use in delivering a cargo molecule to a target cell.

### Stimulating an Immune Response

In one aspect, there is provided a method of stimulating an immune response to an antigen comprising administering to a subject the nucleic acid as defined herein, the vector as defined herein, the recombinant viral genome as defined herein, the viral particle as defined herein, or the targeted EVs as defined herein, wherein said target cell comprises an immune cell, and wherein said at least one EV therapeutic payload polypeptide comprises an antigen.

In one embodiment, said antigen comprises a disease cell-specific antigen.

In one embodiment, said antigen comprises a tumor-specific antigen

In one embodiment, said antigen is from a pathogen.

In one embodiment, said at least one EV therapeutic payload polypeptide further comprises an adjuvant.

In one aspect, there is provided a use, for stimulating an immune response to an antigen, of the nucleic acid as defined herein, the vector as defined herein, the recombinant viral genome as defined herein, the viral particle as defined herein, or the targeted EVs as defined herein, wherein said target cell comprises an immune cell, and wherein said at least one EV therapeutic payload polypeptide comprises an antigen.

In one embodiment, said antigen comprises a disease cell-specific antigen.

In one embodiment, said antigen comprises a tumor-specific antigen

In one embodiment, said antigen is from a pathogen.

In one embodiment, said at least one EV therapeutic payload polypeptide further comprises an adjuvant.

In one aspect, there is provided the nucleic acid as defined herein, the vector as defined herein, the recombinant viral genome as defined herein, the viral particle as defined herein, or the targeted EVs as defined herein, for use in stimulating an immune response to an antigen, wherein said target cell comprises an immune cell, and wherein said at least one EV therapeutic payload polypeptide comprises an antigen.

In one embodiment, said antigen comprises a disease cell-specific antigen.

In one embodiment, said antigen comprises a tumor-specific antigen

In one embodiment, said antigen is from a pathogen.

In one embodiment, said at least one EV therapeutic payload polypeptide further comprises an adjuvant.

### Killing Target Cells

In one aspect, there is provided a method of killing target cells comprising administering to a subject the nucleic acid as defined herein, the vector as defined herein, the recombinant viral genome as defined herein, the viral particle as defined herein, or the targeted EVs as defined herein, wherein said at least one EV therapeutic payload polypeptide comprises a cytotoxic molecule.

In one embodiment, said cytotoxic molecule comprises human GZMB R201K, murine GZMB, diphtheria toxin, a PE38 domain from Pseudomonas exotoxin A, or human TRAIL.

In one embodiment, said target cell comprises a disease cell.

In one embodiment, said disease cell is a tumor cell.

In one aspect, there is provided a use, for killing target cells, the nucleic acid as defined herein, the vector as defined herein, the recombinant viral genome as defined herein, the viral particle as defined herein, or the targeted EVs as defined herein, wherein said at least one EV therapeutic payload polypeptide comprises a cytotoxic molecule.

In one embodiment, said cytotoxic molecule comprises human GZMB R201K, murine GZMB, diphtheria toxin, a PE38 domain from Pseudomonas exotoxin A, or human TRAIL.

In one embodiment, said target cell comprises a disease cell.

In one embodiment, said disease cell is a tumor cell.

In one aspect, there is the nucleic acid as defined herein, the vector as defined herein, the recombinant viral genome as defined herein, the viral particle as defined herein, or the targeted EVs as defined herein, for use in killing target cells, wherein said at least one EV therapeutic payload polypeptide comprises a cytotoxic molecule.

In one embodiment, said cytotoxic molecule comprises human GZMB R201K, murine GZMB, diphtheria toxin, a PE38 domain from Pseudomonas exotoxin A, or human TRAIL.

In one embodiment, said target cell comprises a disease cell.

In one embodiment, said disease cell is a tumor cell.

### Reprogramming Immune Cells

In one aspect, there is provided a method of reprogramming immune cells comprising contacting the immune cells with the nucleic acid as defined herein, the vector as defined herein, the recombinant viral genome as defined herein, the viral particle as defined herein, or the targeted EVs as defined herein, wherein the at least one EV therapeutic payload molecule comprises an immunomodulatory molecule.

In one embodiment, said immunomodulatory molecular comprises a STING or ERAdP pathway activator.

In one embodiment, said STING or ERAdP pathway activator comprises a bacterial dinucleotide cyclase.

In one embodiment, said bacterial dinucleotide cyclase comprises CdaA.

In one embodiment, said immune cells comprise B cells, a T cells, NK cells, dendritic cells, macrophages, or neutrophils. In one embodiment, said immune cells comprise macrophages.

In one embodiment, said immunomodulatory molecule comprises a STING pathway activator, said immune cells comprise macrophages, and said at least one target molecule is macrophage receptor (MARCO).

In one aspect, there is provided a use, for reprogramming immune cells, of the immune cells with the nucleic acid as defined herein, the vector as defined herein, the recombinant viral genome as defined herein, the viral particle as defined herein, or the targeted EVs as defined herein, wherein the at least one EV therapeutic payload molecule comprises an immunomodulatory molecule.

In one embodiment, said immunomodulatory molecular comprises a STING or ERAdP pathway activator.

In one embodiment, said STING or ERAdP pathway activator comprises a bacterial dinucleotide cyclase.

In one embodiment, said bacterial dinucleotide cyclase comprises CdaA.

In one embodiment, said immune cells comprise B cells, a T cells, NK cells, dendritic cells, macrophages, or neutrophils. In one embodiment, said immune cells comprise macrophages.

In one embodiment, said immunomodulatory molecule comprises a STING pathway activator, said immune cells comprise macrophages, and said at least one target molecule is macrophage receptor (MARCO).

In one aspect, there is provided the immune cells with the nucleic acid as defined herein, the vector as defined herein, the recombinant viral genome as defined herein, the viral particle as defined herein, or the targeted EVs as defined herein, for use in reprogramming immune cells, wherein the at least one EV therapeutic payload molecule comprises an immunomodulatory molecule.

In one embodiment, said immunomodulatory molecular comprises a STING or ERAdP pathway activator.

In one embodiment, said STING or ERAdP pathway activator comprises a bacterial dinucleotide cyclase.

In one embodiment, said bacterial dinucleotide cyclase comprises CdaA.

In one embodiment, said immune cells comprise B cells, a T cells, NK cells, dendritic cells, macrophages, or neutrophils. In one embodiment, said immune cells comprise macrophages.

In one embodiment, said immunomodulatory molecule comprises a STING pathway activator, said immune cells comprise macrophages, and said at least one target molecule is macrophage receptor (MARCO).

### Directing an Immune Response to a Target

In one aspect, there is provided a method of directing an immune response to a target disease cell comprising administering to a subject the nucleic acid as defined herein, the vector as defined herein, the recombinant viral genome as defined herein, the viral particle as defined herein, or the targeted EVs as defined herein, wherein said recombinant polypeptide comprises at least two targeting moieties which specifically bind, respectively, to at least two different target molecules, wherein said at least two different target molecules are expressed, respectively, by an immune cell and a disease cell.

In one embodiment, said disease cell comprises a tumor cell.

In one embodiment, one of said at least two different target molecules comprises a tumor-associated antigen.

In one embodiment, said immune cell comprises a T-cell, a B-cell, a natural killer (NK) cell, a dendritic cell, a macrophage, or a neutrophil.

In one embodiment, said immune cell is a T cell.

In one embodiment, said immune cell is a regulatory T cell or a cytotoxic T cell.

In one embodiment, said immune cell is an NK cell.

In one aspect, there is provided a use, for directing an immune response to target disease cells, of the vector as defined herein, the recombinant viral genome as defined herein, the viral particle as defined herein, or the targeted EVs as defined herein, wherein said recombinant polypeptide comprises at least two targeting moieties which specifically bind, respectively, to at least two different target molecules, wherein said at least two different target molecules are expressed, respectively, by an immune cell and a disease cell.

In one embodiment, said disease cell comprises a tumor cell.

In one embodiment, one of said at least two different target molecules comprises a tumor-associated antigen.

In one embodiment, said immune cell comprises a T-cell, a B-cell, a natural killer (NK) cell, a dendritic cell, a macrophage, or a neutrophil.

In one embodiment, said immune cell is a T cell.

In one embodiment, said immune cell is a regulatory T cell or a cytotoxic T cell.

In one embodiment, said immune cell is an NK cell.

In one aspect, there is provided the vector as defined herein, the recombinant viral genome as defined herein, the viral particle as defined herein, or the targeted EVs as defined herein, for use in directing an immune response to target disease cells, wherein said recombinant polypeptide comprises at least two targeting moieties which specifically bind, respectively, to at least two different target molecules, wherein said at least two different target molecules are expressed, respectively, by an immune cell and a disease cell.

In one embodiment, said disease cell comprises a tumor cell.

In one embodiment, one of said at least two different target molecules comprises a tumor-associated antigen.

In one embodiment, said immune cell comprises a T-cell, a B-cell, a natural killer (NK) cell, a dendritic cell, a macrophage, or a neutrophil.

In one embodiment, said immune cell is a T cell.

In one embodiment, said immune cell is a regulatory T cell or a cytotoxic T cell.

In one embodiment, said immune cell is an NK cell.

### Preparation of Therapeutic Targeted EVs

In one aspect, there is provided a method of preparing therapeutic targeted EVs for a subject comprising:
- contacting cells obtained from a subject with the nucleic acid as defined herein, the vector as defined herein, the recombinant viral genome as defined herein, or the viral particle as defined herein, and
- collecting the targeted EVs.

In one embodiment, said cells are tumor cells.

In one embodiment, said cells are immune cells.

In one embodiment, said immune cells comprises T cells, B cells, natural killer (NK) cells, dendritic cells, macrophages, or neutrophils.

In one aspect, there is provided a use, for preparing therapeutic targeted EVs for a subject comprising, of the nucleic acid as defined herein, the vector as defined herein, the recombinant viral genome as defined herein, or the viral particle as defined herein.

In one embodiment, said cells are tumor cells.

In one embodiment, said cells are immune cells.

In one embodiment, said immune cells comprises T cells, B cells, natural killer (NK) cells, dendritic cells, macrophages, or neutrophils.

In one aspect, there is provided the nucleic acid as defined herein, the vector as defined herein, the recombinant viral genome as defined herein, or the viral particle as defined herein for use in preparing therapeutic targeted EVs for a subject.

In one embodiment, said cells are tumor cells.

In one embodiment, said cells are immune cells.

In one embodiment, said immune cells comprises T cells, B cells, natural killer (NK) cells, dendritic cells, macrophages, or neutrophils

### Production Methods

In one embodiment, there is provided a method of producing targeted EVs, wherein said method comprises expressing the nucleic acid as defined herein in cells, or culturing the host cell as defined herein to produce EVs; and collecting the EVs.

### Definitions & Embodiments

The following definitions are provided to facilitate understanding of the terms used herein.

By a **"tumor-selective"** virus is meant a virus that preferentially grows or replicates in tumor cells.

By **"oncolytic virus"** is meant any one of a number of viruses that have been shown, when active, to replicate and kill tumor cells *in vitro* or *in vivo.* These viruses may naturally oncolytic viruses, or virus that have been modified to produce or improve oncolytic activity. Oncolytic viruses include Rhabdoviruses. Rhabdoviruses include: Carajas virus, Chandipura virus, Cocal virus, Isfahan virus, Piry virus, Vesicular stomatitis Alagoas virus, BeAn 157575 virus, Boteke virus, Calchaqui virus, Eel virus American, Gray Lodge virus, Jurona virus, Klamath virus, Kwatta virus, La Joya virus, Malpais Spring virus, Mount Elgon bat virus, Perinet virus, Tupaia virus, Farmington, Bahia Grande virus, Muir Springs virus, Reed Ranch virus, Hart Park virus, Flanders virus, Kamese virus, Mosqueiro virus, Mossuril virus, Barur virus, Fukuoka virus, Kern Canyon virus, Nkolbisson virus, Le Dantec virus, Keuraliba virus, Connecticut virus, New Minto virus, Sawgrass virus, Chaco virus, Sena Madureira virus, Timbo virus, Almpiwar virus, Aruac virus, Bangoran virus, Bimbo virus, Bivens Arm virus, Blue crab virus, Charleville virus, Coastal Plains virus, DakArK 7292 virus, Entamoeba virus, Garba virus, Gossas virus, Humpty Doo virus, Joinjakaka virus, Kannamangalam virus, Kolongo virus, Koolpinyah virus, Kotonkon virus, Landjia virus, Manitoba virus, Marco virus, Nasoule virus, Navarro virus, Ngaingan virus, Oak- Vale virus, Obodhiang virus, Oita virus, Ouango virus, Parry Creek virus, Rio Grande cichlid virus, Sandjimba virus, Sigma virus, Sripur virus, Sweetwater Branch virus, Tibrogargan virus, Xiburema virus, Yata virus, Rhode Island, Adelaide River virus, Berrimah virus, Kimberley virus, Maraba virus, Bovine ephemeral fever virus, or engineered variants thereof.

**"Extracellular vesicles"** (EVs) are cell-derived membranous structures, including exosomes, microvesicles, virus-like particles, macrovesicles, oncosomes, gesicles, and apoptotic bodies. These extracellular vesicles generally are categorized based on their size, specific markers, cellular origin and biogenesis processes. Exosomes are 30-160 nm vesicles of endosomal-origin released from the cell upon fusion of a multivesicular body (MVB) membrane with the plasma membrane. Exosomes are produced by every cell type and their release can be induced by a variety of stimuli, including stress, hypoxia, cell death, and viral infection. Classical microvesicles (also known as microparticles) are 100nm-1µm vesicles released from the cell by shedding of the plasma membrane. Cancer cells can also secrete larger microvesicles (>1 µm) called oncosomes, which only differ from classical microvesicles in regard to their size. Like exosomes, microvesicle release can be induced by stress and viral infection, and their contents are heterogeneous. Apoptotic bodies are large EVs that are released from apoptotic cells by blebbing and range in size from 200 nm to 5 µm. These phosphatidylserine and Annexin V-coated EVs contain cytoplasmic contents from the dying cell. Traditionally, EVs that pelleted at 100,000g were referred to as exosomes, but in fact this pellet contains a combination of microvesicles and exosomes. Though their biogenesis pathways are distinct, exosomes and microvesicles have many similarities and are difficult to distinguish from one another once released from the cell. Recently, the International Society for Extracellular Vesicles suggested the term Small EVs (sEVs) should be used for particles less than 200nm in size, while the term Large EVs (IEVs) should be used for particles greater than 200 nm.

The terms **"programmed EVs"** (PEVs) and **"targeted EVs"** are used synonymously herein to refer to EVs comprising the recombinant polypeptide, as defined herein, and therefore having an engineered acquired affinity (provided by the targeting moiety) for a target molecule.

By **"recombinant"** is meant a nucleic acid or polypeptide molecule that contains segments of different origins, such as (but not limited to) the products of genetic engineering through recombinant DNA technology.

By **"EV-anchoring polypeptide"** is meant a polypeptide that tethers the recombinant polypeptide to an EV membrane.

By **"EV-directed transmembrane polypeptide"** is meant the portion of a transmembrane protein that spans the entirety of a phospholipid bilayer membrane of an EV, and which innately targets (is trafficked to) EV membranes.

Proteins containing such EV-directed transmembrane domains can originate from viruses (e.g. VSVG), or originate in cells (e.g. CD63 and lamp2b). Membrane spanning domains may be single pass or may pass through the membrane multiple times, such as four times (quadruple pass, or tetraspanin).

Single pass and tetraspanin domains can be engineered via linker sequences to carry a single or multiple payloads, and single pass domains can be similarly engineered to carry a single or multiple targeting moieties in tandem.

Likewise, by **"EV-directed tetraspanin"** is meant a subset of tetraspanins that are trafficked to EV membranes. Tetraspanins are a family of membrane proteins found in all multicellular eukaryotes, and also referred to as the transmembrane 4 superfamily (TM4SF) proteins. They have four transmembrane alpha-helices and two extracellular domains, one short extracellular domain or loop, and one longer extracellular domain/loop. Although several protein families have four transmembrane alpha-helices, tetraspanins are defined by conserved amino acid sequences including four or more cysteine residues in the EC2 domain, with two in a highly conserved 'CCG' motif.

Tetraspanins can be engineered to carry up to 2 targeting moieties, and up to 2 payloads directly, or more if linked together.

**Table 1** provides some examples of proteins that specifically direct to, and are enriched in, EV membranes. These examples include single pass and tetraspanin domains.

**Table 1: Examples of Proteins Comprising EV-directed Transmembrane Domains**

| | Protein Name (abbreviation) | Origin (virus/cell) | Single pass or Tetraspanin | NCBI sequence ID for Glycoprotein or Genome (see also Table 13) |
|---|---|---|---|---|
| D1m | CD63 (Murine) | Cell | Tetraspanin | MGI:99529 |
| D1h | CD63 (Human) | Cell | Tetraspanin | NG_008347.1 |
| D2 | CD9 | Cell | Tetraspanin | NG_055677 |
| D3 | LAMP2B | Cell | Single | DQ895288.2 |
| D7 | VSVG-TM | Virus | Single | J02428.1 |
| D8 | CD81 | Cell | Tetraspanin | NG_023386.1 |
| D9 | CD82 | Cell | Tetraspanin | NG_023234.1 |
| D10 | LAMP1 | Cell | Single | NC_000013.11 |
| D11 | JUNIN virus glycoprotein TM | Cell | Single | NP_899218.1 |
| D12 | LASSA fever virus glycoprotein TM | Cell | Single | AIT17400.1 |
| D13 | LCMV glycoprotein TM | Cell | Single | AAX49341.1 |
| D14 | SARS-CoV-2 glycoprotein TM | Cell | Single | See Table 13 |
| D15 | Tamiami virus glycoprotein TM Domain | Cell | Single | AAN32955.1 |
| D16 | Guanarito virus glycoprotein TM Domain | Cell | Single | NP_899210.1 |
| D17 | Machupo virus glycoprotein TM Domain | Cell | Single | NP_899212.1 |
| D18 | Sabia virus glycoprotein TM Domain | Cell | Single | YP_089665 |
| D19 | Parana virus glycoprotein TM Domain | Cell | Single | AAN32957.1 |
| D20 | CdaA TM Domain | Cell | Single | See Table 13 |

By **"derived from",** in the context of a recombinant tetraspanin, it would be understood that the native tetraspanin is modified to include exogenous sequences, such as a targeting moiety inserted into one or both extracellular loop(s) and/or a payload linked to the tetraspanin.

By **"targeting moiety"** is meant a molecule capable of binding to a target molecule with sufficient affinity and specificity so as to be able to target EVs to a target cell expressing the target molecule. Non-limiting examples of targeting moieties include antibodies, functional fragments thereof, engineered fragments thereof, ligands (which target receptors), designed ankyrin repeat proteins (DARPins) (which bind target proteins), and domains that mediate specific protein-protein interactions. It would be understood that the targeting moiety of the recombinant polypeptide is, in the context of an EV, intended to be externally-orientated.

**Table 2** sets for some example targeting moieties.

**Table 2: Example Targeting Moieties**

| **ID** | **Name** | **Description (Target name)** | **Application** |
|---|---|---|---|
| T1 | Anti-DEC205 scFv | Targets DEC205 | Vaccination and tailored delivery/activation of dendritic cells |
| T2 | Anti-CLEC9A scFv | Targets CLEC9A | Vaccination and tailored delivery/activation of dendritic cells |
| T3 | Anti-CEACAMS scFv | Targets CEACAM5 | Tailored delivery of therapeutic payloads and or cargoes to cancer cells (e.g. Colorectal cancer) |
| T4 | Anti-CTLA4 scFv (9D9 Clone) | Targets CTLA4 | Tailored delivery of payloads and/or cargoes to immune cells displaying CTL4 (e.g. Treg cells) |
| T5 | Anti-CD19 scFv | Targets CD19 | Tailored delivery of therapeutic payloads and/or cargoes to cancer cells expressing CD19 |
| T6 | Anti-CD20 scFv | Targets CD20 | Tailored delivery of therapeutic payloads and/or cargoes to cancer cells expressing CD20 |
| T7 | Anti-FAP scFv | Targets the and murine fibroblast activating protein (FAP) | Tailored delivery of therapeutic payloads and/or cargoes to cancer associated fibroblasts and some cancer cells that express FAP (e.g. Pancreatic cancer) |
| T8 | Anti-CA9 scFv (7D12 clone-1) | Targets CA9 | Tailored delivery of therapeutic payloads and/or cargoes to cancer associated fibroblasts and some cancer cells that express CA9 (e.g. Cancer cells in hypoxic environments) |
| T9 | Anti-CA9 scFv (7D12 clone-2) | Targets CA9 | Tailored delivery of therapeutic payloads and/or cargoes to cancer associated fibroblasts and some cancer cells that express CA9 (e.g. Cancer cells in hypoxic environments) |
| T10 | Anti-CTLA4 scFv | Targets CTLA4 | Tailored delivery of payloads and/or cargoes to immune cells displaying CTL4 (e.g. Treg cells) |
| T11 | Chlorotoxin | Targets human and murine cancer cells by binding to various surface proteins that are enriched in malignant cells (e.g. MMP-2, Annexin A2, etc.). | Tailored delivery of payloads and/or cargoes to tumor cells |
| T12 | PD1 ectodomain | Binds mouse PD-L1 | Targets PD-L1 expressing cancer and immune cells. Could act as a "a nucleic acid ligand system." or could target payload and/or cargoes for specific delivery |
| T13 | SIRPa ectodomain | N-Terminal V-set Ig domain of SIRPa (residues 1-118)-F6 variant. This variant binds more efficiently to mouse and human CD47 | CD47, also known as the "don't-eat-me" signal, is a cell surface protein that transmits an anti-phagocytic signal to macrophages upon engaging with its receptor signal regulatory protein α (SIRPα). Molecules that antagonize the CD47-SIRPα interaction by binding to CD47, such as anti-CD47 antibodies and the engineered SIRPα variant CV1, have been shown to facilitate macrophage-mediated antitumor responses. |
| T14 | VAR2 domain of Plasmodium falciparum protein, VAR2CSA, | Targets chondroitin sulfate modifications found on the surface of cancer cells and placenta. | In the placenta, VAR2CSA binds a distinct type of chondroitin sulfate (CS) glycosaminoglycan (GAG) chain called CS A (CSA) The minimal CS binding region of VAR2CSA consists of the Duffy Binding Ligand-like (DBL) 2X domain with flanking interdomain (ID) regions. This domain binds CS with remarkably high specificity and affinity |
| T15 | Anti-CD3 | Binds on CD3 on T cells | In the context of EV-BITEs, binding tom CD3 can be used to target cytotoxic T cells against tumor cells. In examples in which only anti-CD3 decorated EVs are used, these EVs bind to T cells expressing CD3 and enhance their tumor killing activity. |
| T16 | Anti-CD7 | Binding to the pan-T cell surface protein CD7, a surface antigen present on the majority of human T cells. | The CD7 receptor is rapidly internalized after being engaged by a targeting moiety, it can be exploited for the targeted delivery of toxin conjugates to T cell lymphomas and leukemias or targeted delivery of payloads and/or cargoes to T cells to fight diseases. |
| T17 | CD40 ligand | CD40 | Vaccination and tailored delivery/activation of dendritic cells |
| T18 | CD40-targeted peptide | CD40 | Vaccination and tailored delivery/activation of dendritic cells |
| T19 | Anti-CD11c | CD11c | Vaccination and tailored delivery/activation of dendritic cells |
| T20 | Anti-CD206 | CD206 | Tailored delivery of payloads and/or cargoes to M2 macrophages |
| T21 | CD206-targeting peptide | CD206 | Tailored delivery of payloads and/or cargoes to M2 macrophages |
| T22 | GE11 | EGFR | Tailored delivery of payloads and/or cargoes to tumor cells expressing EGFR |
| T23 | iRGD (integrin-binding peptide) | αv-Integrin | Tailored delivery of payloads and/or cargoes to tumor cells expressing Integrin |
| T24 | Anti-CD22 | CD22 | Targeting CD22 on precursor B-cell acute lymphoblastic leukemia (BCP-ALL) and other Cd22+ malignancies for tailored delivery of therapeutic payloads and/or cargoes |
| T25 | Anti-CD44 | CD44, which is highly expressed in high tumorigenic and metastatic hepatocellular cancer stem cells (CSCs) | Anti-CD44 decorated-EVs can deliver payloads and/or cargoes to CSCs. Interestingly, the anti-CD44 antibody itself can induce the apoptosis of CD90⁺ hepatocellular carcinoma stem cells Conceivably, an anti-CD44-decorated EVs could directly induce CSC death, along with their delivery role. |
| T26 | LDLR (low density lipoprotein receptor) targeting peptide | LDLR | LDLR targeting peptide (LDLRT) refers to the endogenous targeting ectodomain of the VSV glycoprotein. and can bind to LDLR expressed on the surface of cells |

A **"single domain antibody"** (sdAbs), also known as a nanobody, is an antibody fragment consisting of a single monomeric variable antibody domain. Like a whole antibody, it is able to bind selectively to a specific antigen. With a molecular weight of only 12-15 kDa, single-domain antibodies are much smaller than common antibodies composed of two heavy chains and two light chains. sdABs are produced by immunization of dromedaries, camels, llamas, alpacas or sharks, or can be engineered from common IgGs with four chains.

By **"functional fragment"** is meant a portion of an antibody that maintains the paratope (comprising the complementary determining regions or CDRs) and is capable of binding to the same target molecule as the parent antibody from which is it derived. Examples include Fab and F(ab')2 fragments.

By **"engineered fragment"** is meant a recombinant polypeptide derived from a parent antibody and retaining the paratope, thus being able to bind to the same target molecule as the parent antibody. An example is a single-chain variable fragment (scFv), which is a fusion protein of the variable regions of the heavy (V_{H}) and light chains (V_{L}) of immunoglobulins, connected with a short linker peptide, of typically 10 to about 25 amino acids.

**"DARPins"** are repeat proteins comprising several repeating structural domains (generally 4 to 6 repeats) of usually 33 amino acids. DARPins can be selected and used as alternative scaffolds for specific targeting because they can bind to their target antigens with high affinity and specificity. A key advantage of using DARPins compared to monoclonal antibodies is that DARPins generally possess low molecular weights, containing between 40 to 100 amino acid residues. For example, HER2 is frequently overexpressed in breast cancer cells. DARPins binding to the extracellular domains of HER2 can be selected and used to direct therapeutic EVs towards malignant cells expressing HER2.

By **"target molecule"** is meant a molecule to which the targeting moiety binds. Such molecules may be cell surface molecules, such as, e.g., polypeptides, lipids, or polysaccharides that can be specifically bound by the targeting moiety.

By **"target** cell" is meant a cell that expresses the target molecule that is bound by the targeting moiety, and to which the payload (if applicable) and/or cargo (if applicable) is/are directed.

By **"intravesicular polypeptide"** is meant the polypeptide portion of the recombinant polypeptide that extends internally to the EV. It will be understood that the intravascular polypeptide may comprise a short polypeptide (e.g. of at least 9 amino acids) that projects into the intravesicular space. However, in other configurations described herein, the it will be understood that the intravascular polypeptide may comprise an EV payload polypeptide. In yet other configurations the EV-directed transmembrane domain and the intravascular polypeptide may together comprise an EV-directed recombinant tetraspanin, which may or may not comprise at least one EV payload polypeptide, which may be linked to the N- and/or C-terminus.

**"Monotargeted"** indicates that a population of EVs is targeted to a target molecule. However, where **"at least one"** target is specified, it will be understood that this is also intended to encompass EVs directed to more than one target molecule, so that the EVs are minimally monotargeted.

Likewise, **"bispecific"** means that an EV targets two target molecules. Where "at least two" is specified, it will be understood that this is also intended to encompass EVs directed to more than two target molecules, such that the EVs are minimally bispecific.

By **"cell surface molecule"** is meant any molecule that is anchored or otherwise associated with a cell surface to permit targeting of the cell by the recombinant polypeptide via the targeting moiety. Such molecules may include, for example, polypeptides, polysaccharides, or lipids (including polysaccharide and lipid modifications to polypeptides). Examples include integral membrane proteins, peripheral membrane proteins, and modifications thereof.

A **"cell surface marker"** is a cell surface molecule particular to (or enriched in) a particular cell type. A cell surface marker or a combination of cell surface markers may be unique to a given cell type, or cell state (such as a disease state).

By **"tumor stroma"** is meant cells in the tumor environment other than cancer cells *per se,* such as, e.g., cancer associated fibroblasts.

By **"tumor-associated antigen"** (TAA) is meant any immunogen that is associated with tumor cells, and that is either absent from or less abundant in healthy cells or corresponding healthy cells (depending on the application and requirements). For instance, the tumor associated antigen may be unique, in the context of the organism, to the tumor cells. A TAA may be, for example, a tumor-specific mutation, an aberrantly spliced protein, an oncofetal antigen, or an endogenous retroviral protein. A TAA may be a neoantigen comprising neoepitope. Neoantigens are newly formed (non-autologous) antigens that have not been previously recognized by the immune system, and can arise, e.g., from tumor mutations.

The terms **"payload"** and **"cargo"** are used differentially here. The former are part of the recombinant polypeptide, while the latter are intended to be separate molecules to be carried in the EVs.

By **"EV payload polypeptide"** is meant any polypeptide that is part of the recombinant polypeptide itself, and that would therefore be co-encoded by the same nucleic acid molecule. EV payload polypeptides include any polypeptides for which EV loading or EV-mediated targeting or delivery would be desirable.

By **"EV therapeutic payload polypeptide"** is meant a therapeutic polypeptide that is part of the recombinant polypeptide itself, and that would therefore be co-encoded by the same nucleic acid molecule. Categories of payloads include (but are not limited to) cytotoxic molecules (e.g. GZMB variants, Diphtheria Toxin, pe38 (domain from pseudomonas exotoxin A), or TRAIL), immune reprogramming molecules (e.g. STING or ERAdP pathway activators, such as bacterial cyclases), enzymes, nucleotide binding domains, and antigens (such as tumor antigens or antigens from infectious pathogens, such as Dengue virus, Malaria, or Rotavirus). Non-limiting examples are presented in **Table 3.**

**Table 3: Example Payloads**

| **ID** | **Name** | **Description** | **Application** |
|---|---|---|---|
| P1 | Nanoluc^{™} | Luciferase protein | Evaluation of EV production and uptake by luciferase assays/Control |
| P2 | mCherry | Fluorescent protein | Detection of EVs by flow cytometry and immunofluorescence/Control |
| P3 | PE38 | Cytotoxic protein including only domain 3 of PE38 | Enhancement of cancer cell death |
| P4 | Diphtheria Toxin | Cytotoxic protein that includes the domain for translocation from endosomes to the cytoplasm (sequence from IL2-DT approved drug) | Enhancement of cancer cell death |
| P5 | Human GZMB R201K | Cytotoxic protein with a point mutation to provide resistance to serpin B9-mediated degradation of granzyme B | Enhancement of cancer cell death |
| P6 | Human TRAIL | Cytotoxic protein | Enhancement of cancer cell death |
| P7 | Ovalbumin | Antigen | Proof-of-Concept-disease (cancer)-specific antigen vaccine |
| P8 | Murine GZMB | Cytotoxic protein | Enhancement of cancer cell death |
| P9 | CdaA | STING pathway activator | Activation of STING in dendritic cells to stimulate cytokine release, cross-presentation of antigens to T cells, maturation of dendritic cells, and T cell-mediated cancer cell death |
| P10 | mtbDISA | STING pathway activator | Activation of STING in dendritic cells to stimulate cytokine release, cross-presentation of antigens to T cells, maturation of dendritic cells, and T cell-mediated cancer cell death |
| P13 | Rotavirus VP6 | Antigen | Infectious disease vaccine |
| P13a | Cas13a RNA-binding motif (RBM) | RNA binding protein | facilitate the loading of RNA species (e.g. miRNAs, mRNAs, long-non-coding RNAs) into EVs |
| P13b | Cas13b RBM | (as above) | (as above) |
| P13c | Cas13d RBM | (as above) | (as above) |
| P13d | Pum RBM | (as above) | (as above) |
| P13e | Stu1 RBM | (as above) | (as above) |
| P13f | Alphavirus capsid protein L72AE RBM | (as above) | (as above) |
| P13g | MS2 coat protein RBM | (as above) | (as above) |
| P13h | VEEV capsid protein RBM | (as above) | (as above) |
| P14 | CY5.5 | Tumor Imaging Payload | Can be used as a control construct, and can be used in vivo and in patient care for tumor imaging Cy5.5 is visible under near IR light |
| P15 | Dopachrome tautomerase, DCT (mouse) | Antigen | Proof-of-Concept-disease (cancer)-specific antigen vaccine and preclinical mouse studies |
| P16 | DCT (human) | Antigen | Cancer-specific antigen vaccine |

By **"EV cargo",** in contrast, is meant a molecule to be carried in the EV, but that is otherwise separate from the recombinant polypeptide that directs the EV to a target. Accordingly, the cargo may be encoded by the same or a different nucleic acid that encodes the recombinant polypeptide (in the case of the latter they would be understood to be expressed as separately polypeptides). It is envisaged, for example, that host cells manipulated to express the recombinant polypeptide could separately encode (or be modified to express) the cargo (or vice versa). Being a separate molecule to the recombinant polypeptide, cargo molecule need not be polypeptides. For example, the cargo molecule could be a small molecule, e.g. a small molecule drug or imaging agent. The cargo could be a nucleic acid, such as an mRNA, miRNA, shRNA, or siRNA. Nucleic acids could be preferentially loaded into vesicles, for example, in embodiments in which the payload comprises a nucleic acid binding domain. Such binding domains may be sequence-specific, binding to a sequence motif within the nucleic acid molecule. Cargo molecules could also comprise polypeptides, such as cytotoxic molecules, immune reprogramming molecules, enzymes, or antigens.

The term **"linked"** indicates that two moieties are covalently linked, though such linkage need not be direct. For example, if "A" and "B" are "linked", it would be understood that the linkage could comprise additional amino acids residues or polypeptides. Likewise, linked **"via"** a feature, such as a linker polypeptide or payload, indicates that the feature lies between (and separates) "A" and "B" in the context of the recombinant polypeptide. However, neither "A" nor "B" need be directly attached to the intervening feature.

By **"adjuvant"** will be understood a molecule that potentiates the immune response to an antigen and/or modulates it towards the desired immune response.

Where nucleic acid and amino acid molecules are referred to herein, it will be appreciated the embodiments encompassing sequence variants thereof are also expressly contemplated. For example, such sequence variants may be for polypeptides (or nucleic acid molecule encoding polypeptides) that retain substantially the same function as the parent molecule from which they are derived, or the same function. Such sequence variants may be at least 70% identical to the parent molecule. They may be at least 80% identical to the parent molecule. They may be at least 90% identical to the parent molecule. They may be at least 95% identical to the parent molecule. They may be at least 96% identical to the parent molecule. They may be at least 97% identical to the parent molecule. They may be at least 98% identical to the parent molecule. They may be at least 99% identical to the parent molecule. Sequence variants contemplated herein may comprise conservative amino acid substitutions (or nucleic acid sequence changes encoding them). Sequence variants contemplated herein may comprise silent mutations.

### EXAMPLES

The following Examples outline embodiments of the invention and/or studies conducted pertaining to the invention. While the Examples are illustrative, the invention is in no way limited the following exemplified embodiments.

### Introduction to the Examples

Attempts have been made to program EVs to carry or deliver therapeutics using multiple molecules, mechanisms and means, which can be expensive, required to be ex vivo, and time-consuming (such as via electroporation), and which are not shelf-stable and lack validation.

There is therefore a need for a means to deliver molecules to cells, including, but not limited to, therapeutics and toxins, to targeted cells in vivo, or prepared simply and continually in vivo, in vitro, or ex vivo, in a simple, inexpensive, stable way.

Recombinant peptides have been designed for targeted delivery of molecules to cells.

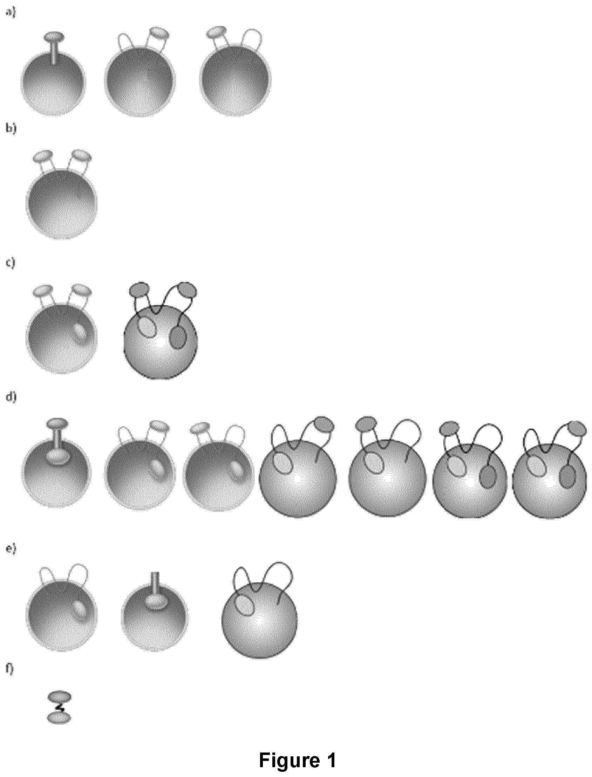

**Figure 1** provides an overview of example construct configurations contemplated herein:

Figure 1, panel (a) - Single target - no payload

Figure 1, panel (b) - Two-targets - no payload

Figure 1, panel (c) - Two-targets - with payload

Figure 1, panel (d) - Single target - with payload

Figure 1, panel (e) - Control - no targets, single payload

Figure 1, panel (f) - Control - no transmembrane domain

### Example 1

### Mono-Targeting - no payload (two-part construct)

Application: Blocking or activating function of cell surface receptors.

How these platforms work: They can act as competitive binding or blocking drugs.

Advantages: Stability and lack of immunogenicity. In cases that the PEV is produced from a virus platform, this has the potential for in-situ delivery. Incidentally, the approach is also more cost-effective due to these solutions.

Delivery/manufacturing modalities: Viral-based platforms (e.g. Vaccinia virus, lentivirus, adeno-associated virus [AAV], VSV, HSV-1, etc.). Plasmids (e.g. pcDNA 3.1) and free PEVs.

**Example 1(a):** Programmed cell death protein 1 (PD1) is a surface protein preferentially expressed in immune cells such as T, B, NK cells, and myeloid-derived dendritic cells. Upon engagement with its ligand, PD-L1, PD1 transmits immune inhibitory signals. Molecules (monoclonal antibodies) that antagonize the PD1:PD-L1 interaction by binding to PD1 and PD-L1 have been shown to facilitate T cell-mediated killing of tumor cells. While these strategies are showing positive results in some clinical indications, these approaches tend to yield a large amount of wasted antigen that do not make it to their final destination/use (e.g. exosomes expressing PD-L1). These factors reduce bioavailability and increase the risk of toxicities of anti-PD1 and anti-PD-L1 monoclonal antibodies.

As such, presently contemplated is a PEV targeting PD1 with PD-L1 as the targeting moiety, thereby blocking the function. Targeting moiety: PD-L1 (blocking application/adjuvant for ICIs).

Payloads: None or optional.

Transmembrane domain (TD domain): All the examples listed in Table 1 could be used.

**Example 1(b):** Similarly, T cells could be activated via the CD3 surface protein using a CD3 targeting moiety, such as an anti-CD3 antibody (T cell activation/engager application).

Payloads: None or optional.

Transmembrane domain (TD domain): All the examples listed in Table 1 could be used.

**Table 4: Example Constructs**

| **Full Name** | **Targeting Molecule** | **Payload** | **TM domain** | **Activity** |
|---|---|---|---|---|
| PD-L1-targeted, PD1-ectodomain Lamp2b-only | PD1 | NONE | Lamp2b | Blocking PD1 |
| PD-L1-targeted, PD1-ectodomain VSVG-only | PD1 | NONE | VSVG | Blocking PD1 |
| anti-CD3 | CD3 | NONE | VSVG | Activation of T cells |

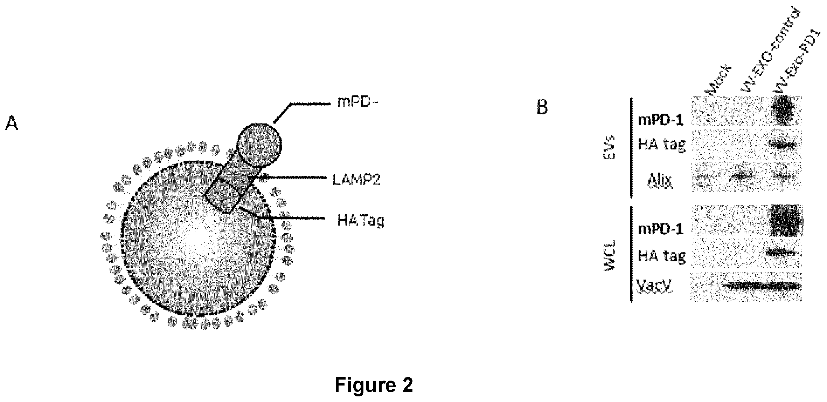

Results: Data for PD1 targeting can be seen in **Figures 2 to 5****.**

**Figure 2****:** An oncolytic vaccinia virus (VV or VacV) can program EVs with a "chimeric blocking construct" encoding the ectodomain of PD1. A VV backbone was created to express a PEV construct that allows for programming EVs to display the murine PD-1 ectodomain (mPD-1) on the surface of EVs. These EVs displaying PD-1 can specifically bind to tumor cells expressing on the surface PD-L1 in mice.

**Figure 2A****:** Schematic of the "chimeric blocking construct" whereby mPD-1 is displayed on the extracellular surface of the PEV and acts as the targeting moiety. The mPD-1 is fused to a transmembrane LAMP2B domain, which helps shuttle the construct into PEVs. Of note, LAMP2b is commonly enriched in EVs. This entire PEV construct is HA-tagged on the intracellular portion of the chimeric transgene construct, which allows for the tracking and visualization of the PEV.

**Figure 2B****:** Human renal cell carcinoma 786-0 cells were mock-infected or infected with VV-Exo-control (This control construct expresses the LAMP2B transmembrane domain and the HA tag on the C-terminus of the construct but the EV-targeting moiety was replaced by the FLAG tag), or with VV-Exo-PD1 (PEV construct shown in panel Figure 2A, MOI (multiplicity of infection) of 1 for 48h). After 48 hours of infection, cells were harvested and lysed for immunoblot analysis. Similarly, extracellular vesicles were isolated from the culture media by a serial centrifugation method. Then, western blot analysis of the EV fractions and whole cell lysates (WCL) were conducted using antibodies for mPD-1 and the HA tag (to confirm proper chimeric transgene expression). The EV marker Alix, and the VV antigen A27L were also tested to properly show EV isolation and VV infection, respectively.

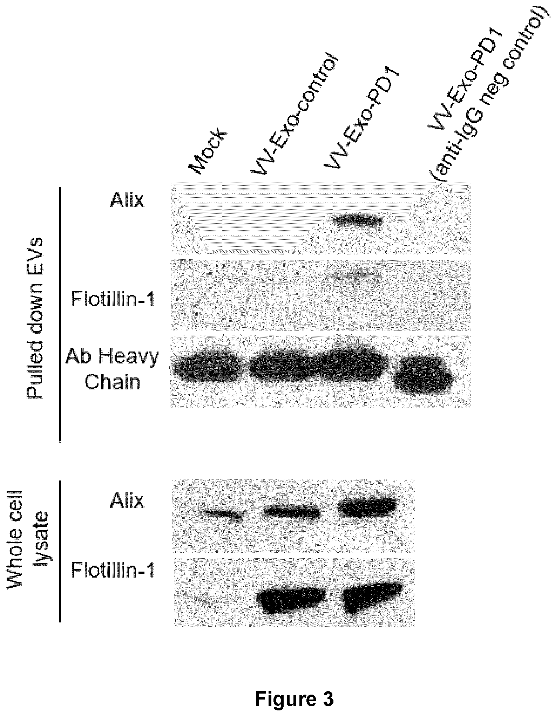

**Figure 3****:** Shows that PEVs expressing the construct shown in Figure 2A can be isolated using anti-PD1 antibodies, with the immunoblot showing that the chimeric protein construct integrates into EVs to form PEVs with the targeting moiety on the exterior. The molecules bound to the anti-PD1 antibodies express Alix and Flotillin (EV markers), indicating that they are integrated with EVs. An oncolytic vaccinia virus (VV) can program infected cell derived-EVs with a "chimeric blocking construct" displaying PD1 on their surface. Using the VV platforms expressing the chimeric PEV construct and its control described above in **Figure 2****,** 786 cancer cells were infected at a MOI of 1 for 48 hours and then cell lysates and EVs were isolated. The image in this figure shows the immunoblotting of the collected EV fractions following immunoprecipitation (IP) pull-down of the intact EVs using anti-PD1 antibodies. The originating WCLs are also shown. The EVs and WCLs were collected from mock or infected cells (MOI of 1 for 48h). The membranes were also probed for the EV markers, Alix and Flotillin-1. Detection of the heavy chains of the antibodies due to cross-reactivity was used as a loading control for each condition.

It is noted that that only the EVs derived from cells infected with a VV expressing the mPD-1-LAMP2B-HA tag construct were pulled down with anti-PD1 antibodies. This data shows that the construct is not only expressed and incorporated into EVs, but also the 'topology or orientation" of the PEV constructs in the EVs is as expected.

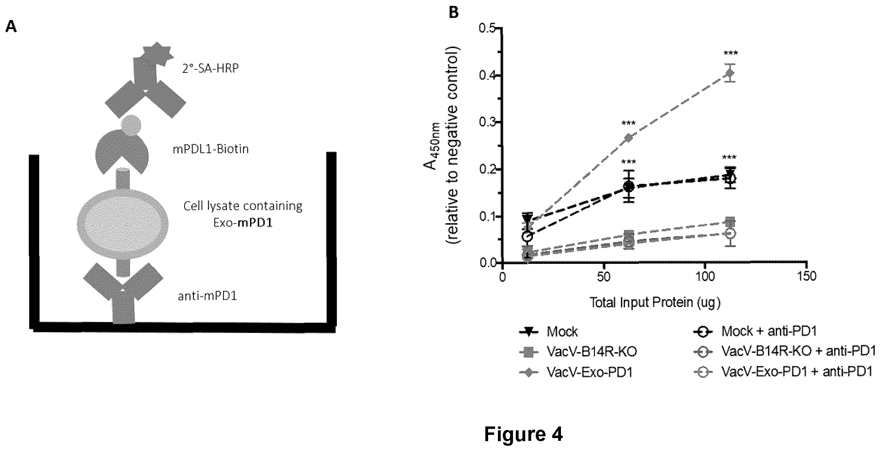

**Figure 4** shows that VV can program EVs with a "PEV blocking construct" to display PD1 that specifically binds to its binding partner, PD-L1.

**Figure 4A****:** Schematic representation of the competitive binding ELISA set-up performed to obtain the data shown in panel B: SA- Streptavidin; HRP- horseradish peroxidase. In this experimental set-up, an mPD-L1 protein conjugated to biotin binds mPD-1 from the Exo-PD1 construct with B14R as a control, resulting in fluorescence signal that can be quantified; samples lacking mPD-1 expression (due to absence of the Exo-PD1 chimera) should therefore not bind the mPD-L1, resulting in little to no fluorescence signal. This shows a cartoon schematic of a competitive binding Enzyme-linked immunosorbent assay (ELISA) experiment used to obtain the data shown in Figure 4B. Here, cell lysates are washed over well plates covered in anti-mPD1 antibody. Therefore, constructs displaying mPD1 will bind to the plates. The plates are washed so that only bound constructs remain, and are then incubated with mPD-L1, the natural binding ligand of PD1, fused with biotin. After this incubation, 2-SA-HRP (2 represents that this is the secondary antibody wash, the first being the PD1) is then washed over the plates, which interacts with biotin and creates a quantifiable fluorescent signal. As such, well plates that display functional PD-1 on the exterior of cell lysate EVs should show fluorescence, as shown in Figure 4b.

**Figure 4B****:** To demonstrate whether the Exo-PD1 chimera can bind mPD-L1, the VV platforms expressing the chimeric PEV construct and its control described herein in **Figure 2** were used. Mouse colorectal CT26 cancer cells were infected at a MOI of 10 for 48 hours and then cell lysates were prepared. Cell lysates were used in the adapted competitive binding ELISA as illustrated in panel A. For this binding ELISA, the samples for each condition were first subjected to Bicinchoninic Acid Protein Assay (BCA) analysis to determine the protein concentration. Based on this analysis, known total input protein concentrations were used for each condition. For each virus treatment, an additional experimental condition (a negative control) was included in which the lysates were pre-incubated with the anti-mPD-1 antibody for 2 hours prior to addition to the ELISA set-up; this was done to block the interaction between the mPD-1 (of the Exo-PD1 chimera) and mPD-L1, to ensure that any fluorescence observed was due to this interaction. Absorbance readings for each condition relative to the negative control were plotted as a function of the total input protein in the CT26 cell lysate samples as indicated. Additional conditions were included in which the cell lysate samples were pre-incubated for 2 hours with an anti-mPD-1 antibody. The results shown in panel B demonstrate that the greatest fluorescence signal observed was for the VV-Exo-PD1 condition. It is also important to note that the fluorescence signal for the VV-Exo-PD1 condition is lost when the lysates are pre-incubated with an anti-mPD-1 antibody (negative control); thus, validating that the signal observed is due to mPD-1 binding to mPD-L1. This is further confirmed by the observation that for the mock infected and VV control virus conditions (with and without anti-PD-1 antibody), there is little to no difference in absorbance. ***P<0.001. This Figure shows fluorescence by concentration of input protein, showing that Vaccinia Virus expressing PD1 (without simultaneous expression of anti-PD1) shows increased fluorescence with increased concentration of protein.

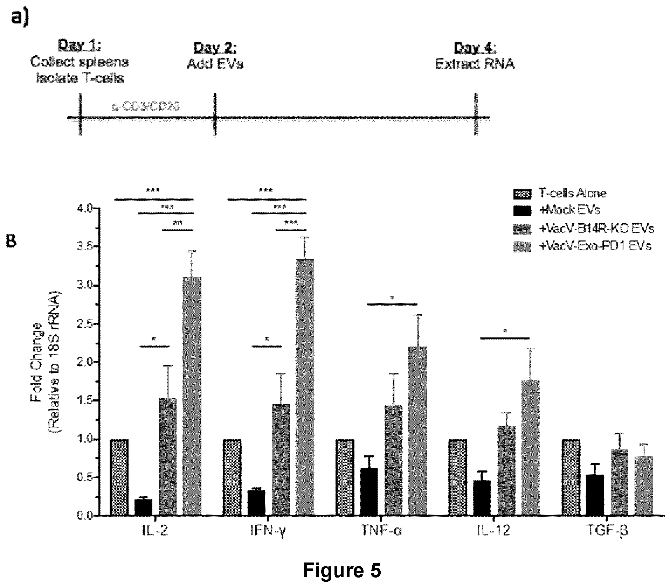

**Figure 5** provides a schematic timeline for an experimental time-course, and data showing that PEVs expressing PD-1 successfully bind to PD-L1 on T cells, thereby activating various mRNA immune markers in these T cells.

**Figure 5****, panel a):** Schematic representation of the experimental time-course setup for the qPCR analysis of T cell activation upon PEV treatment shown in panel B. In brief, T cells were isolated from mouse spleens; the cells were then subjected to overnight activation by anti-CD3e and anti-CD28 antibodies. In parallel, EVs were collected from CT26 cells that were either mock-infected, infected with the VV-Exo-control virus, or infected with the VV-Exo-PD1 virus (described in **Figure 2****)** at an MOI of 10 for 48 hours. EVs were purified by differential centrifugation for each condition, and then transferred to the activated T-cells for 48 hours. Then, RNA was extracted from the T-cells and subjected to qPCR analysis. An additional experimental condition in which no EVs were transferred to the activated T-cells was also included (T-cells alone), as well as analysis of TGF-β mRNA levels -this marker functions as a non-targeting control, since it is not one of the cytokines known to be altered by the PD1:PD-L1 inhibitory axis. It is important to mention that prior to the EV transfer, the EVs for each condition were normalized, such that an equal concentration was used for each condition.

**Figure 5****, panel B:** Data showing that the Exo-PD1 EVs are able to activate various immune markers at the mRNA level in murine T-cells. qPCR analysis of mRNA collected from murine T-cells following treatment with equal concentrations of EVs. EVs were collected from CT26-WT cells treated as indicated in the graph (mock-infected cells or cells infected with a control VV virus or VV-exo-PD1). The immune T cell activation markers used in this study are: IL-2, IFN-y, TNF-α, and IL-12. TGF-β was included as a non-target control. *P<0.05, **P<0.01, ***P<0.001.

### Example 2

### Multi-targeting two-part constructs - (EV-BiKe and EV-BiTe) (no payload)

Background: In nature, the Major Histocompatibility Complex (MHC) is required for T cells to recognize and kill tumor cells. However, most tumors downregulate the expression of the (MHC) to escape immune attack. One existing strategy in the art to circumvent the tumor's escape mechanism is by way of engineered bi-specific antibodies which draw T-cells and Tumor cells to close proximity. These bi-specific antibodies are also referred to as Bi-specific T cell Engagers or BiTEs.

These BiTEs are able to mediate the T cell's capacity to recognize and kill tumor cells in an MHC independent fashion. BiTEs consist of linked variable chain antibody fragments directed against the T cell antigen CD3 and a specific tumor-associated antigen (TAA). Similarly, Bi-specific NK cell engagers or BiKEs can mediate simultaneous binding to an activating receptor on NK cells and a surface tumor antigen to thus promote NK cell-dependent killing of tumor cells. Although existing BiKE and BiTE technologies are promising, many that are currently in clinical development have issues with associated toxicity during systemic administration, drug stability issues (short half-life), and challenges to reaching high enough local concentrations to be effective in most solid cancers

Application: PEV constructs with two targeting moieties: one that recognizes T (or NK) cell targets, and the other targeting tumor cells (cancer cell or CAFs).

How these platforms work: These PEVs will promote the synapsis between T cells and tumor cells or between NK cells and tumor cells, thus promoting the directed killing of tumor cells by these immune cell types.

Advantages: Displaying BiTEs and BiKEs in a PEV format is more stable than the bi-specific antibody constructs.

Special Features: Generally, payload-less - the PEV construct itself is a stable bi-specific cell engager bringing T or NK cells closer to cancer cells. These PEVs can be produced in vivo or ex vivo.

Delivery modalities: Using OVs as delivery vehicles in patients to secrete BiTEs and BiKEs in the infected cancer cell. As such, the PEV is delivered to the exact site where needed, and therefore likely to be effective at picomolar concentrations. i.e., lower dose treatment than the current bi-specific antibody approaches. Viral-based platforms such as: Vaccinia virus (abbreviated as VacV or VV), lentivirus, adeno-associated virus [AAV], VSV, HSV-1, etc. could be used.

Plasmids (e.g. pcDNA 3.1) for preparing the virus and infecting cells, as well as for manufacturing isolated PEVs are also contemplated.

Targeting moieties: Single chain variable fragments or nanobodies as described above. These bind to:
- Tumor cells: surface tumor antigen targets (e.g. anti-CEA, anti-CA9, anti-FAP, etc.); and either
   - T cells: molecules that bind to T cells (e.g. CD3 target, via an anti-CD3 scFV targeting moieties) , or
   - NK cells: molecules that bind to NK cell receptor targets (e.g. anti-CD16 or anti-NKG2D)

### Payloads: None

Transmembrane Domain: All the examples listed in Table 1 could be used. Examples included here are with tetraspanin proteins, however single pass TM proteins may be used "multimerization technology" (see special features for details).

**Table 5: Bi-specific EV Targeting Constructs**

| **Full Name** | **Targeting Molecule(s)** | **Payload** | **TM domain** | **Activity** |
|---|---|---|---|---|
| Bi-specific EV targeting human CEA and engaging human T cells (through CD3) | α-hCEA, α-hCD3 | NONE | mCD63 | Activation of T cells to kill tumour cells |
| Bi-specific EV targeting human CEA and engaging murine T cells (through CD3) | α-hCEA, α-mCD3 | NONE | mCD63 | Activation of T cells to kill tumour cells |
| Bi-specific EV targeting murine and human FAP and engaging murine T cells (through CD3) | α-mhFAP, α-mCD3 | Flag | mCD63 | Activation of T cells to kill tumour cells |

### Results:

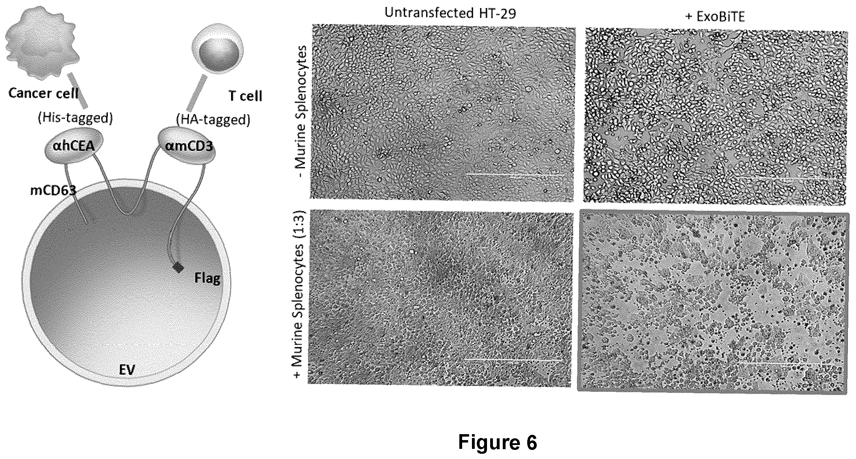

**Figure 6****:** An EXO-bite PEV construct targeting CEA in the surface of cancer cells and CD3 on the surface of T cells leads to enhance cancer cell death. HT-29 cells were transfected with ExoBiTE constructs (i.e. anti-CEA+anti-CD3-CD63 construct that displays antibodies recognizing CEACAM5 and CD3 on the surface of cancer cells and T cells, respectively, a cartoon showing the Exo-bite construct and its topology in EVs is shown in the left panel) or left untransfected. Then cells were co-incubated or not with mouse splenocytes (1:3 ratio) for 48 hours. Note that HT-29 cells transfected with Exo-bite construct and then co-cultured with splenocytes lead to enhanced (~50%) cell death. See panel boxed in bold.

**Figure 6****, left panel** shows a cartoon schematic of an example of a PEV with a tetraspanin-based chimeric construct targeting T cells and cancer cells. **Figure 6****, right panel** shows that there is enhanced cell death when cells are transfected with the bispecific PEVs drawing T cells to the cancer cells.

### Example 3

### Mono-targeting - Immunologic adjuvant payloads

Background/Context: Pharmacologic stimulation of innate immune processes represents an attractive strategy to achieve multiple therapeutic outcomes such as inhibition of virus replication, boosting antitumor immunity, and enhancing vaccine immunogenicity. The platforms described herein may represent effective means to augment and prolong the cellular and tumoral immune responses evoked by infectious disease and cancer vaccines, respectively.

Application: Immunologic adjuvants (e.g. STING or ERAdP activators, which generate immunogenic molecules that stimulate the immune system) payloads can be specifically delivered to antigen presenting cells (APCs) such as dendritic cells (DCs) by targeting specific DC surface molecules.

How these platforms work: Antigen presenting cells (APC), such as DCs exhibit a largely immature or immunologically tolerizing phenotype (not yet functionally ready to accept presented-antigens, or serving to suppress immune responsiveness). Delivery of immunologic adjuvants (i.e. STING or ERAdP pathway activators, e.g. bacterial dinucleotide cyclases such as CdaA and MtbDisa which are c-di-AMP cyclases, and VCA0848, which is a c-di-GMP cyclase, or mouse/human cGAS) to DCs via PEVs may result in activation of STING and/or ERAdP, which enhances DC antigen presentation capacity, and increases expression of T cell co-stimulatory molecules, thereby boosting the APC activity. In some instances, these platforms can be used in combination with vaccine approaches.

Advantages: Stability, less off-target toxicity, tailored delivery.

Targeting moieties: The targets are antigen presenting cell-surface molecules, including but not limited to CD40, a TNF-α family receptor, DEC-205, a C-type lectin receptor and CD11c, an integrin receptor, by way of targeting moieties including specific monoclonal antibodies, scFvs, single domain antibodies, nanobodies (i.e. anti-DEC205, anti-Clec9A, anti-CD11c, anti-lectin receptor). Peptides and ligands represent a suitable alternative to antibodies as active targeting agents (e.g. CD40 ligand or CD40-targeted peptide).

Payloads: Bacteria dinucleotide cyclases (i.e. CdaA, etc.) (Note: these payloads are enzymes, thus these examples indicate that functionally active enzymes could also be delivered by PEVs).

Transmembrane domain: All the examples listed in **Table 1** could be used. Thus far, all our examples are built with VSV-G and CD63.

Delivery/manufacturing modalities: Viral-based platforms such as, Vaccinia virus, lentivirus, adeno-associated virus [AAV], VSV, HSV-1, etc. could be used. Plasmids (e.g. pcDNA 3.1) for preparing recombinant virus and transfecting cells, as well as manufactured and isolated PEVs are also contemplated.

**Table 6: PEV-targeting Dendritic Cells with Payload to Activate STING or ERAdP**

| **Full Name** | **Targeting Molecule** | **Payload** | **TM domain** | **Activity** |
|---|---|---|---|---|
| Murine CLEC9a-targeted, VSVG-CdaA | α-mCLEC9a | CdaA | VSVG | Activation of APCs |
| Murine DEC205-targeted, VSVG-CdaA | α-mDEC205 | CdaA | VSVG | Activation of APCs |
| Murine DEC205-targeted, VSVG-XX | α-mDEC205 | XX | VSVG | |
| Murine DEC205-targeted, VSVG-XX | α-mDEC205 | XX | VSVG | |
| Murine DEC205-targeted, mCD63-CdaA | α-mDEC205 | CdaA | CD63 | Activation of APCs |

### Results:

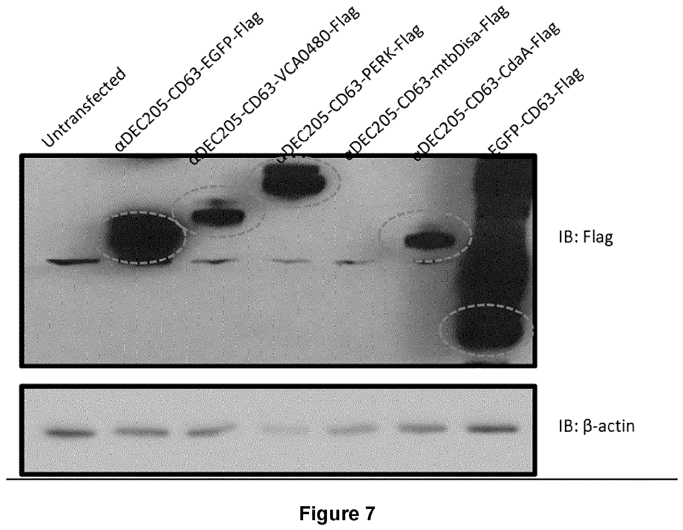

**Figures 7 to 10** provide Western blots showing construct expression in cells and EVs from pcDNA3.1 plasmids and from VV. Note that HEK293T (human embryonic kidney) cells were chosen as an example simply due to ease of transfection, and are representative of any number of cell types. Note that these figures also represent constructs that act as cancer vaccines, as described below.

**Figure 7****:** Various chimeric PEVs constructs are properly expressed upon cell transfection. HEK293T cells were left untransfected or transfected with indicated CD63 plasmids (all constructs are Flag-tagged in their C-terminus) containing an anti-DEC205 targeting moiety and different payloads [inserted in the second loop of CD63]. Cells lysates were collected at 24hrs and immunoblotted and probed for indicated antibodies (anti-Flag, or anti-beta-actin as loading control). Red ovals show the desired bands.

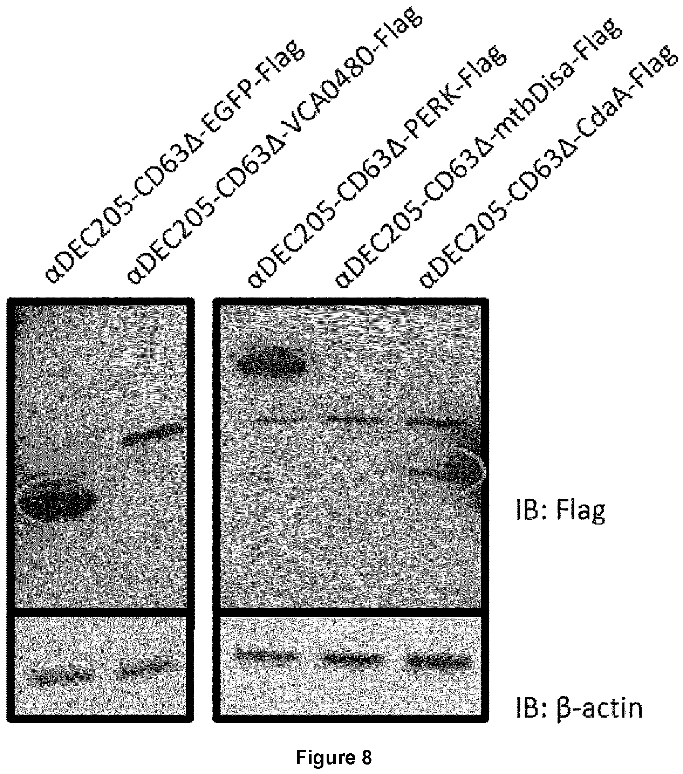

**Figure 8****:** Various chimeric PEVs constructs are properly expressed upon cell transfection. HEK293T cells were untransfected or transfected with indicated CD63 plasmids (all constructs are Flag-tagged in their C-terminus) containing an anti-DEC205 targeting moiety and different payloads [inserted in the first loop of CD63]. Cells lysates were collected at 24hrs and immunoblotted for indicated antibodies (anti-Flag, or anti-beta-actin as loading control). Red ovals show the desired bands.

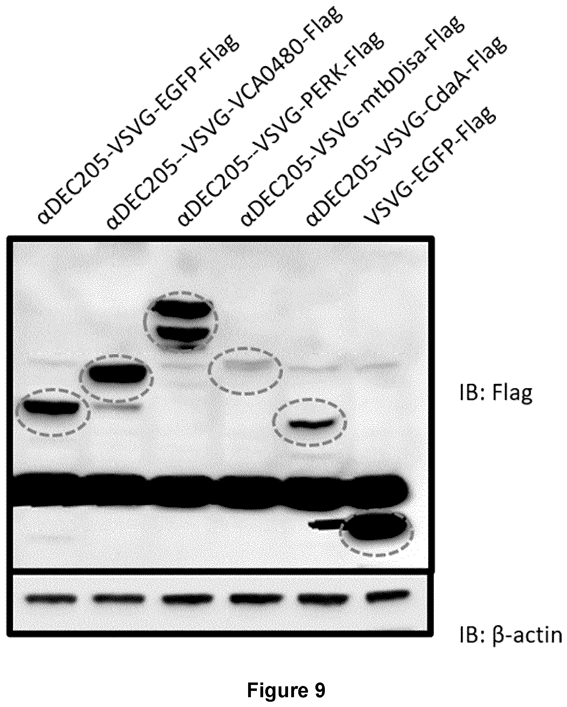

**Figure 9****:** Various chimeric PEVs constructs are properly expressed upon cell transfection. HEK293T cells were left untransfected or transfected with indicated pcDNA3.1 plasmids (all constructs are Flag-tagged in their C-Terminus) containing an anti-DEC205 targeting moiety a VSV-G TM domain, and different payloads. Cells lysates were collected at 24hrs and immunoblotted and probed for indicated antibodies (anti-Flag, or anti-beta-actin as loading control). Red ovals show the desired bands.

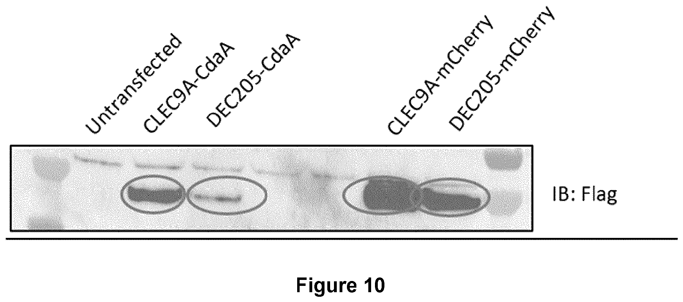

**Figure 10****:** Various chimeric PEVs constructs are properly expressed upon cell transfection. HEK293T cells were left untransfected or transfected with indicated pcDNA3.1 plasmids (ectodomain-negative & Flag-tagged) containing an anti-DEC205 or anti-CLEC9A targeting moiety (VSV-G) and different payloads (CdaA or mCherry). Cells lysates were collected at 24hrs and immunoblotted and probed for indicated antibodies (anti-Flag, or anti-beta-actin as loading control). Red ovals show the desired bands.

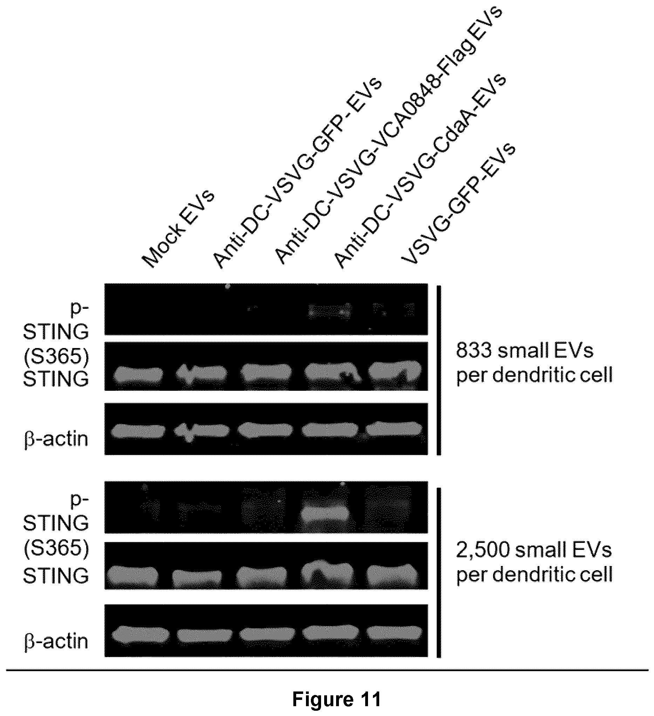

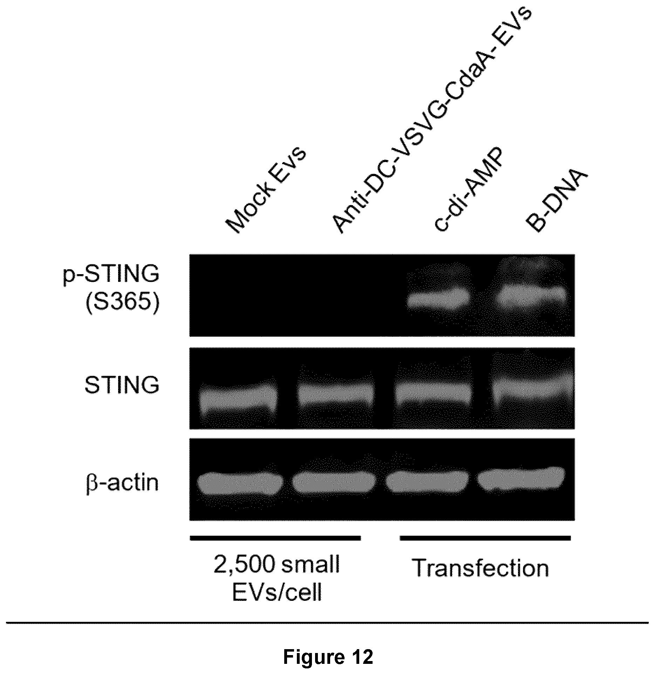

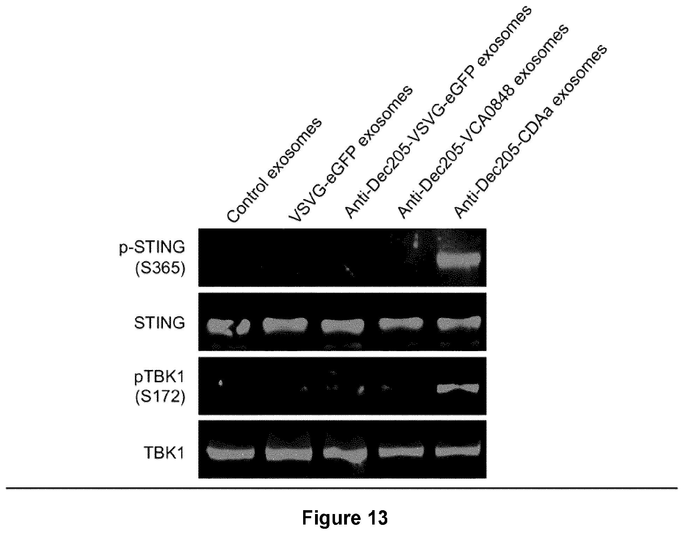

**Figure 11 to 13** show 3 panels of western immunoblots showing that isolated PEVs containing chimeric constructs that target Dec205, with a VSVG transmembrane domain and CdaA payload (STING and/or ERAdP pathway activator) successfully activate STING or ERAdP in dendritic cells in a dose dependent fashion (Fig 11) but do not activate STING in non-target murine fibroblasts (Figure 12, L929 cells with c-di-AMP and B-DNA positive controls). This dinucleotide cyclase delivery to DCs leads to activation of the STING signalling axis as indicated by phosphorylation of TBK1 (Fig 13). These figures show that STING phosphorylation occurs (activation) in DCs.

Isolated PEVs containing anti-DEC205-VSVG-CdaA chimeric constructs lead to STING activation in a dose dependent fashion. The STING (stimulator of interferon genes) pathway contributes to the activation of antigen presenting cells, including DCs. STING activation is mediated by its phosphorylation. In DCs, activation of STING is important for IFN-β expression and IL-12 production as well as for the surface expression of the activation markers CD40 and CD86. The role of the cGAS-STING pathway is important in pathogen detection and in cancer immunity. STING activation, as well as ERAdP activation, appear to be an essential component in the recruitment of immune cells to the tumor microenvironment, which is paramount to immune clearance of the tumor. STING activation provides an adjuvant function during vaccination as well.

The data shown here demonstrates that only EVs decorated with anti-DEC205-VSVG-CdaA constructs can activate the STING-TBK1-IRF3 signaling axis in primary murine dendritic cells (**Figure 11**) but not mouse fibroblasts (**Figure 12**), which do not express DEC205 in the cell surface but are able to respond to classic STING agonists c-di-AMP and B-DNA, following 24h treatment by the indicated amounts of EVs isolated from HEK293T cells transfected with pcDNA3.1 plasmids encoding the indicated PEV constructs and controls. Note that herein STING activation is demonstrated using an antibody that recognizes phosphorylated STING at serine 365. Loading controls include total STING and b-actin. **Figure 13****:** Activation of STING-TBK1-IRF3 signaling axis in primary mouse DCs following 24h treatment by EVs isolated from HEK293T cells transfected with VSVG plasmids as indicated. STING and TBK1 activation by phosphorylation is demonstrated by western blot using phospho-specific antibodies. Total levels of STING and TBK1 are also shown as loading controls. STING has been identified as a critical signaling molecule required for the detection of cytosolic nucleic acids, particularly dsDNAs derived from pathogens and viruses as well as endogenous second messengers, such as cyclic-di-GMP and -AMP). These events result in the production of innate immune response genes through the activation (phosphorylation) IRF3 and NF-κB pathways by cellular kinases, including TBK1. TBK1 activity is regulated by auto-phosphorylation of its Ser172. Note that in panel C only PEVs carrying anti-DEC205-VSVG-CdaA constructs not only lead to STING activation (phosphorylation) but also lead to the phosphorylation (=activation) of a downstream molecule (TBK1) in mouse dendritic cells.

### Example 4

### Mono-targeting - Cancer Vaccine Payloads

Background/Context: Tumor-associated antigens and/or immune reprograming moieties (e.g. STING or ERAdP pathway activators) can be specifically delivered to surface molecules on APCs, such as dendritic cells via PEVs. This construct would express a targeting moiety to target PEVs to DCs (dendritic cells) and it could concomitantly carry one or multiple payloads.

Application: These platforms will represent effective means to elicit robust tumor antigen-specific immunity.

How these platforms work: DCs exhibit a largely immature or tolerizing phenotype. Tumor antigen delivery via PEVs (as payloads or cargo), in conjunction with co-administration of an adjuvant (DC maturation stimuli such as agonistic anti-CD40 mAbs, poly(I:C), cytosine-phosphate-guanine (CpG), lipo-polysaccharide (LPS), or toll-like receptor 7/8 (TLR7/8) agonists) or targeted co-delivery of PEVs containing STING or ERAdP pathway activators as described above (e.g. Bacterial dinucleotide cyclases such as CdaA and MtbDisa which are c-di-AMP cyclases, and VCA0848, which is a c-di-GMP cyclase) results in enhanced tumor-associated antigen presentation capacity and increased expression of T cell costimulatory molecules

Tumor-associated antigens alone or in combination with adjuvants or in combination with immune reprograming moieties (e.g. STING or ERAdP pathway activators) can be specifically delivered to surface molecules on dendritic cells via PEVs

Targeting moieties: Targets: Antigen presenting cell-surface molecules, including CD40, a TNF-α family receptor, DEC205, a C-type lectin receptor (CLEC9) and CD11c, an integrin receptor, are targeted by targeting moieties including specific monoclonal antibodies, scFvs, single domain antibodies, nanobodies (i.e. anti-DEC205, anti-Clec9A, anti-CD11c), ligands or targeted peptides (e.g. CD40 ligand or CD40-targeted peptide).

Payloads: Specific tumor-associated antigens (For proof-of-concept in mouse tumor models: DCT and OVA are being explored). Human tumor-associated antigens relevant for clinical testing can be used (e.g. HPV-E6 and E7, NY-ESO-1, etc.). Cancer-specific neoantigens can also be used.

Concomitant expression of specific disease cell antigens is contemplated, such as tumor-associated/specific antigens (e.g. OVA, DCT, mERKm9 etc.). Also, adjuvant molecules such as a STING or ERAdP activator could be concomitantly delivered with disease-specific antigens or tumor-associated/specific antigens

Transmembrane domain: All the examples listed in **Table 1** could be used. Thus far, all our examples are built with VSV-G.

Delivery/manufacturing modalities: Viral-based platforms such as, Vaccinia virus, lentivirus, adeno-associated virus [AAV], VSV, HSV-1 etc. could be used. Plasmids (e.g. pcDNA 3.1) for transfecting cells, as well as manufactured isolated PEVs are also contemplated.

**Table 7: Dendritic Cell Targeting with Antigen Payloads (Cancer Vaccine Applications)**

| **Full Name** | **Targeting Molecule** | **Payload** | **TD domain** | **activity** |
|---|---|---|---|---|
| Murine CLEC9a-targeted, VSVG-OVA | α-mCLEC9a | OVA | VSVG | Direct antigen delivery |
| Murine DEC205-targeted, VSVG-OVA | α-mDEC205 | OVA | VSVG | Direct Antigen delivery |
| Murine DEC205-targeted, VSVG-DCT | α-mDEC205 | DCT | VSVG | Direct Antigen delivery |
| Murine DEC205-targeted, VSVG-mERKm9 | α-mDEC205 | mERKm9 | VSVG | Direct Antigen delivery |

### Results:

**Figures 7 to 9** provide Western blots showing construct expression in cells and EVs from a pcDNA3.1 vector plasmid after transfection .

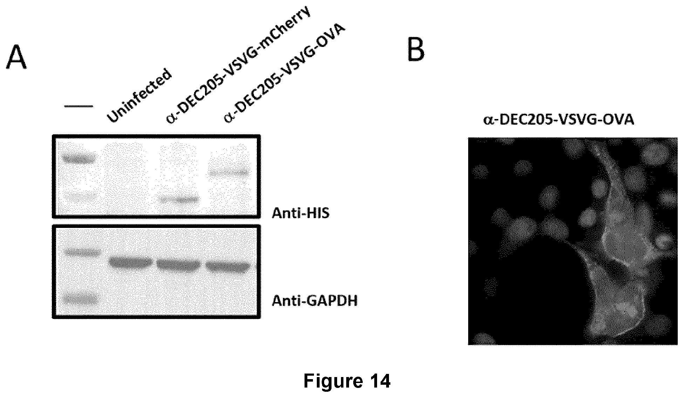

**Figure 14: Figure 14****, panel A** is a western blot showing expression of PEVs targeting dendritic cells (targeting moiety- anti-Dec205), with a VSVG-based transmembrane domain and mCherry (control) or OVA (cancer target). **Figure 14****, panel B** is an immunofluorescence image showing cell expression of anti-DEC205-VSVG-OVA upon transfection of a plasmid encoding this construct. These figures shows that a chimeric PEV construct targeting DEC205 and carrying the ovalbumin (OVA) antigen is properly expressed upon cell transfection.

**Figure 14****, panel A:** HEK293T cells were transfected with indicated pcDNA 3.1 plasmids encoding an anti-DEC205-VSVG-OVA or an anti-DEC205-VSVG-mCherry control construct. Note that both constructs are HIS tagged in the N-terminus. Cell lysates were collected at 24hrs post-transfection and prepared for immunoblotting with specific antibodies against the HIS tag and the loading control GAPDH.

**Figure 14****, panel B:** HEK293T cells were transfected with indicated pcDNA 3.1 plasmids encoding an anti-DEC205-VSVG-OVA construct. Note that both constructs are HIS tagged in the N-terminus. Cell were fixed at 24hrs post-transfection and prepared for immunofluorescence staining with anti-HIS antibodies (Green). Nuclei were stained with DAPI (blue).

### Example 5

### Mono-targeting - Infectious Disease Vaccine payloads

Background/Context: Pathogen-specific antigens and/or immune reprograming moieties (e.g. STING or ERAdP pathway activators) can be specifically delivered to surface molecules on dendritic cells via PEVs. This construct would express a targeting moiety to tailor PEVs to DCs and it could concomitantly carry multiple payloads.

Application: These platforms will represent effective means to elicit robust pathogen-specific antigen-driven immunity.

How these platforms work: Similar to above, DCs exhibit a largely immature phenotype. Pathogen-specific antigen delivery via PEVs, in conjunction with co-administration of adjuvant (DC maturation stimuli such as agonistic anti-CD40 mAbs, poly(I:C), cytosine-phosphate-guanine (CpG), lipo-polysaccharide (LPS), or toll-like receptor 7/8 (TLR7/8) agonists) or targeted co-delivery of PEVs containing STING or ERAdP pathway activators (e.g. Bacterial dinucleotide cyclase, such as CdaA and MtbDisa which are c-di-AMP cyclases, and VCA0848, which is a c-di-GMP cyclase) results in enhanced pathogen-specific antigen presentation capacity and increased expression of T cell costimulatory molecules

Targeting moieties: Targets include Antigen presenting cell-surface molecules, including CD40, a TNF-α family receptor, DEC-205, a C-type lectin receptor and CD11c, an integrin receptor, are targeted by means of targeting moieties such as specific monoclonal antibodies, scFvs, single domain antibodies, nanobodies (i.e. anti-DEC205, anti-Clec9A, anti-CD11c), ligands or targeted peptides (e.g. CD40 ligand or CD40-targeted peptide).

Payloads: Pathogen-specific antigens [e.g. Dengue PM & E antigens, Malaria CS30, Rotavirus VP6, etc.). Concomitant expression of specific infectious disease-associated antigens with adjuvant molecules such as STING or ERAdP activator could be pursued to boost vaccination activity.

Transmembrane domain: All the examples listed in **Table 1** could be used. Thus far, all our examples are built with VSV-G.

Delivery/manufacturing modalities: Viral-based platforms (e.g. Vaccinia virus, lentivirus, adeno-associated virus [AAV], VSV, etc.). Plasmids (e.g. pcDNA 3.1) and free PEVs.

**Table 8: Dendritic Cell Targeting with Antigen Payloads (Vaccine Applications)**

| **Full Name** | **Targeting Molecule** | **Payload** | **TD domain** | **Activity** |
|---|---|---|---|---|
| Murine CLEC9a-targeted, VSVG-VP6 | α-mCLEC9a | VP6 | VSVG | Direct antigen delivery |
| Murine CLEC9a-targeted, VSVG-E-prM-DENV2 | α-mCLEC9a | E-prM-DENV2 | VSVG | Direct antigen delivery |
| Murine DEC205-targeted, VSVG-VP6 | α-mDEC205 | VP6 | VSVG | Direct antigen delivery |
| Murine DEC205-targeted, VSVG-E-prM-DENV2 | α-mDEC205 | E-prM-DENV2 | VSVG | Direct antigen delivery |
| Murine DEC205-targeted, VSVG-CS30 | α-mDEC205 | Cs30 | VSVG | Direct antigen delivery |

### Results:

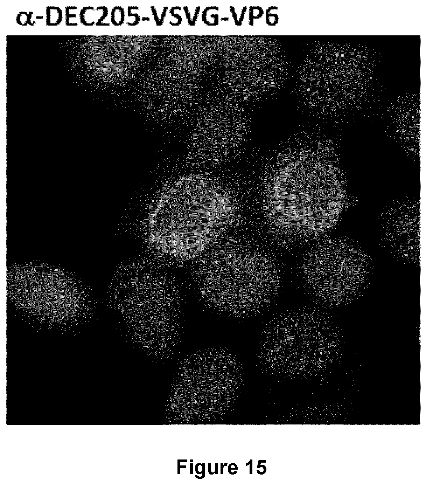

**Figure 15** is an immunofluorescence image showing cell expression of anti-DEC205-VSVG-VP6, which is a rotavirus antigen, upon transfection of a plasmid encoding this construct. A chimeric PEV construct targeting DEC205 and carrying the rotavirus antigen (VP6) is properly expressed upon cell transfection. HEK293T cells were transfected with indicated pcDNA 3.1 plasmids encoding an anti-DEC205-VSVG-VP6 construct. After 24 hours, cells were fixed and prepared for immunofluorescence staining with anti-HIS antibodies (Green). Nuclei were stained with DAPI (blue). Note that both the anti-DEC205-VSVG-VP6 construct is HIS tagged in the N-terminus.

### Example 6

### Mono-targeting - Immune Reprogramming Payloads

Background/Context: Immune reprograming molecules (e.g. cytokines, miRNAs) can be specifically delivered as payloads or cargoes to surface molecule targets on specific immune cell populations via PEVs. This construct would express a targeting moiety to tailor PEVs to specific-immune cell populations and it could concomitantly carry multiple payloads.

Application: These platforms represent effective means to reprogram or educate (e.g. activate, phenotype change, etc.) immune cells to play specific functions and thus fight inflammatory diseases and cancer. In addition, these PEVs could be used to augment the visibility (immunogenicity) of cancer cells to immune cells (e.g. promoting immunogenic cell death).

### Example 6(a): M1/M2 imbalance

How these platforms work: Immune-suppressive M2 macrophages will be turned into immune-boosting M1 macrophages that are ready to engulf tumor cells. Also, certain subsets of macrophages are important in causing inflammatory diseases such as asthma, atherosclerosis, rheumatoid arthritis, osteoarthritis, endometriosis, diabetes type 1 and 2, and obesity. Macrophage reprograming can be done with PEVs.

Targeting moieties: Single chain variable fragments or a binding peptide for CD206 (Mannose receptor) can be used to specifically target M2 macrophages (also known as tumor-promoting macrophages).

Payloads: either payload-less with cargo, or payload being an RNA-binding motif to specifically capture cargo that modifies macrophage polarization to reduce inflammatory gene expression through RNAi, as multiple genes can be downregulated simultaneously. Cargo targets may include inflammatory mediators such as cytokines (e.g., TNF-α, IL-6, IL-1β), chemokines (e.g., CCL2, CCL3, CCL5), and transduction targets involved in promoting inflammation, such as members of the NF-κB signaling cascade. miRNA cassettes targeting IκBα, siRNA directed toward mitogen-activated protein kinase4 4 (Map4k4) reduced systemic inflammation by reducing Tnf-α mRNA in macrophages.

### Example 6(b): Treg reprogramming:

How these platforms work: Regulatory T cells (Tregs) are known to restrict the function of effector T cells. In the context of cancer, Tregs are powerful inhibitors of anti-tumor immunity and the presence of these cells in the tumor microenvironment leads to tumor growth. Directed targeting of regulatory molecules in Tregs with PEVs will lead to the conversion of these cells into IFNg-secreting effector cells (cancer-fighting cells).

Targeting moieties: Single chain variable fragments directed to CTL4 (cytotoxic T-lymphocyte-associated antigen 4) on the surface of immune suppressive T cells.

Payloads: either payload-less with cargo, or payload being an RNA-binding motif to specifically capture cargo that convert immunosuppressive regulatory T cells (Tregs) into cancer fighting T cell by downregulating CARMA1 and/or MALT1. For example, a miRNA cassettes containing a shRNA against CARMA 1 and/or MALT1. T cell activation. These miRNA cassettes may be EV-directed miRNA cassettes with or without RNA sequences corresponding to the payload RNA-binding motif recognition site. Alternatively, these miRNA cassettes may be regular non-EV directed cassettes which include an RNA sequence corresponding to the payload's RNA-binding motif recognition site.

Transmembrane Domain: All the examples listed in **Table 1** could be used.

Delivery/manufacturing modalities: Viral-based platforms (e.g. Vaccinia virus, lentivirus, adeno-associated virus [AAV], VSV, etc.). Plasmids (e.g. pcDNA 3.1) and free PEVs.

### Example 6(c): T cell activation

How these platforms work: Decreased T cell function has been described in chronic viral, bacteria and parasitic infections and in cancer. CD3-targeting PEVs can be used to stimulate the activity of disease-fighting T cells in the immune system.

Targeting moieties: T cell activation: Single chain variable fragments or single-domain antibodies targeting CD3 on T cells.

Payloads: The CD3 targeting construct is payless- Engaging CD3 in T cells may be sufficient to activate them and mobilize them to kill cancer cells. Anti-CD3 monoclonal antibodies (mAbs) initiate signals which result in activation of T lymphocytes through the T-cell receptor (TCR), involving the phosphatidylinositol pathway, activation of PKC, and increasing intracellular calcium (Cai2+).

### Example 7

### Mono-targeting and Multi-targeting - EV-CAR-like, with cytotoxic payload

Background/Context: These provide an EV that functions like a targeted cytotoxic T cell (akin to CAR-T therapy, but removing the T cell from the equation). This enables the killing of highly immunosuppressive, immunologically "cold" tumors and MHC-I deficient cancers by our PEVs.

Application: PEVs with a cytotoxic function, used as a drug to target specific tumor cell types as described in the examples below. Other cell types could be contemplated.

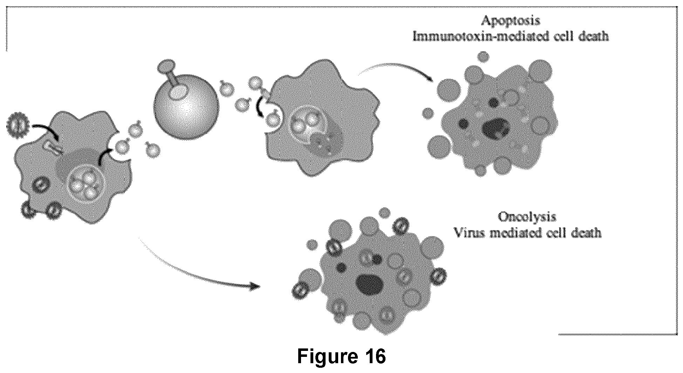

How these platforms work: **Figure 16** depicts the proposed mechanism of action of the mono-targeted EVs carrying a cytotoxic payload through a viral-based platform. While vaccinia virus is depicted here with a mono-targeting moiety, this can be extended to other delivery/manufacturing modalities such as other viruses (lentivirus, adeno-associated virus, vesicular stomatitis virus, etc.), through plasmid expression (i.e. pcDNA3.1) and free PEVs. In brief, the engineered vaccinia virus would infect a cancer cell. In the infected cell, the viral genome is transcribed and translated to create more viral progeny which would be released to infect adjacent tumor cells resulting in oncolysis or viral-mediated cell death. In parallel, the transgene, which has been encoded into the viral genome, would also be translated by the infected cell. This transgene would be comprised of a targeting moiety (purple) fused to a transmembrane linker domain (gray) which would carry a cytotoxic payload (green). The transmembrane domain, i.e. VSVG, preferentially shuttles the construct into PEVs, such that the targeting domain is on the extracellular surface of the PEV, while the payload is sequestered intracellularly. These PEVs are then secreted by the infected cell. Through the extracellular targeting moiety, the PEVs then bind to the target antigen on adjacent cancer cells resulting in uptake of these PEVs, and release of their cytotoxic payload into the recipient cell resulting in death of the antigen-positive target cell.

Advantages: not autologous, stable, specifically targeted, can be virally delivered or shelf-stably produced.

Targeting moieties: Single chain variable fragments or single domain antibodies (i.e., anti-CD19, anti-CD20, anti-CD22, anti-EGFR, anti-FAP, anti-CEA, anti-CA9) or through targeting peptides [i.e. MMP2-targeted chlorotoxin (CTX), proteoglycan-targeted VAR2Δ (VAR2Δ also named as VAR2CSA, binds to a distinct type chondroitin sulfate (CS) exclusively expressed in the placenta and also found on a high proportion on cancer cells), GE11 peptide, which targets with high affinity EGFR].

Payloads: Cytotoxic payloads such as murine granzyme B (mGZMB), human granzyme B (hGZMB R201K) - note that the R201K mutation is to confer resistance against the endogenous human granzyme B inhibitor-, diphtheria toxin (DT), TRAIL (a cytokine that causes cell death primarily in tumor cells), and the truncated pseudomonas exotoxin 38 (PE38).

Transmembrane Domain: All the examples listed in **Table 1** could be used.

Delivery/manufacturing modalities: Viral-based platforms (e.g. Vaccinia virus, lentivirus, adeno-associated virus [AAV], VSV, etc.). Plasmids (e.g. pcDNA 3.1) and free PEVs.

**Table 9: Example Constructs**

| **Full Name** | **Targeting Molecule(s)** | **Payload** | **TM domain** | **Activity** |
|---|---|---|---|---|
| Anti-CD19+anti-CD20-CD63-mGZMB | α-hCD20, α-hCD19 | mGZMB | mCD63 | Bi-specific mCD63-positive PEVs concomitantly targeting human CD19 and CD20 and delivering mGZMB |
| anti-CD20-CD63-mGZMB | α-hCD20 | mGZMB | mCD63 | Mono-targeted mCD63-positive EV binding human CD20 and delivering mGZMB |
| Anti-CD19-CD63-mGZMB | α-hCD19 | mGZMB | mCD63 | Mono-targeted mCD63-positive EV binding human CD19 and delivering mGZMB. |
| Anti-CD19-VSVG-PE38 | α-hCD19 | PE38 | VSVG | Mono-targeted VSVG-positive EV binding human CD19 and delivering PE38. |
| CTX-VSVG-mGZMB | CTX | mGZMB | VSVG | CTX peptide targeting molecule (binds to MMP proteins, such as MMP2, which are highly expressed in cancer cells). |
| CTX- VSVG-hGZMB R201K | CTX | hGZMB R201K | VSVG | CTX peptide targeting molecule (binds to MMP proteins, such as MMP2, which are highly expressed in cancer cells). |
| CTX VSVG-Diphtheria Toxin | CTX | DT | VSVG | CTX peptide targeting molecule (binds to MMP proteins, such as MMP2, which are highly expressed in cancer cells). |
| Anti-CA9-VSVG-mGZMB | α-mhCA9 | mGZMB | VSVG | PEVs targeting murine & human CA9, a surface molecule often upregulated in multiple cancer cells and CAFs and at the same time delivering mGZMB. |
| | | | | |
| Anti-CA9-VSVG-hGZMB R201K | α-mhCA9 | hGZMB R201K | VSVG | PEVs targeting murine & human CA9, a surface molecule often upregulated in multiple cancer cells and CAFs and at the same time delivering hGZMB R201K. |
| anti-CA9-VSVG-Diphtheria Toxin | α-mhCA9 | DT | VSVG | PEVs targeting murine & human CA9, a surface molecule often upregulated in multiple cancer cells and CAFs and at the same time delivering DT. |
| anti-CA9 mCD63-mGZMB | α-mhCA9 | mGZMB | CD63 | PEVs targeting murine & human CA9, a surface molecule often upregulated in multiple cancer cells and CAFs and at the same time delivering mGZMB. |
| anti-CEA-VSVG-mGZMB | α-mhCEA | mGZMB | VSVG | PEVs targeting murine & human CEA, a surface molecule often upregulated in multiple cancer cells and at the same time delivering mGZMB. |
| anti-CEA- VSVG-hGZMB R201K | α-mhCEA | hGZMB R201K | VSVG | PEVs targeting murine & human CEA, a surface molecule often upregulated in multiple cancer cells and at the same time delivering mGZMB. |
| anti-CEA-VSVG-Diphtheria Toxin | α-mhCEA | DT | VSVG | PEVs targeting murine & human CEA, a surface molecule often upregulated in multiple cancer cells and at the same time delivering DT. |
| anti-CEA- mCD63-mGZMB | α-mhCEA | mGZMB | CD63 | PEVs targeting murine & human CEA, a surface molecule often upregulated in multiple cancer cells and at the same time delivering mGZMB. |
| VAR2Δ-VSVG-mGZMB | VAR2Δ | mGZMB | VSVG | PEVs targeting Proteoglycans normally present on the surface of multiple cancer cells and concomitantly delivering mGZMB. |
| VAR2Δ-VSVG-hGZMB R201K | VAR2Δ | hGZMB R201K | VSVG | PEVs targeting Proteoglycans normally present on the surface of multiple cancer cells and concomitantly delivering mGZMB. |
| VAR2Δ-VSVG-Diphtheria Toxin | VAR2Δ | DT | VSVG | PEVs targeting Proteoglycans normally present on the surface of multiple cancer cells and concomitantly delivering mGZMB. |
| GE11-VSVG-mGZMB | GE11 | mGZMB | VSVG | PEVs targeting human EGFR on the surface of cancer cells and delivering mGZMB. |
| GE11-VSVG-hGZMB R201K | GE11 | hGZMB R201K | VSVG | PEVs targeting human EGFR on the surface of cancer cells and delivering hGZMBR201K. |
| GE11-VSVG-Diphtheria Toxin | GE11 | DT | VSVG | PEVs targeting human EGFR on the surface of cancer cells and delivering DT. |
| Anti-FAP-VSVG-mGZMB | α-mhFAP | mGZMB | VSVG | PEVs targeting human and mouse FAP on the surface of CAFs and delivering mGZMB. |
| Anti-FAP-VSVG-hGZMB R201K | α-mhFAP | hGZMB R201K | VSVG | PEVs targeting human and mouse FAPon the surface of CAFs and delivering hGZMBR201K. |
| Anti-FAP-VSVG-Diphtheria Toxin | α-mhFAP | DT | VSVG | PEVs targeting human and mouse FAPon the surface of CAFs and delivering DT. |
| PD1-VSVG-TRAIL | Mouse PD1 actodomain | TRAIL | VSVG | PEVs targeting mouse PD-L1 on the surface of tumor cells and concomitantly delivering TRAIL. |
| Murine CTLA4-targeted, VSVG-mGZMB | α-m9D9 | mGZMB | VSVG | T-regs targeting with cytotoxic payload |
| Murine CTLA4-targeted, VSVG-hGZMB R201K | α-m9D9 | hGZMB R201K | VSVG | T-regs targeting with cytotoxic payload |
| Murine CTLA4-targeted, VSVG-Diphtheria Toxin | α-m9D9 | DT | VSVG | T-regs targeting with cytotoxic payload |
| Murine CTLA4-targeted, mCD63-mGZMB | α-m9D9 | mGZMB | CD63 | T-regs targeting with cytotoxic payload |

### Results:

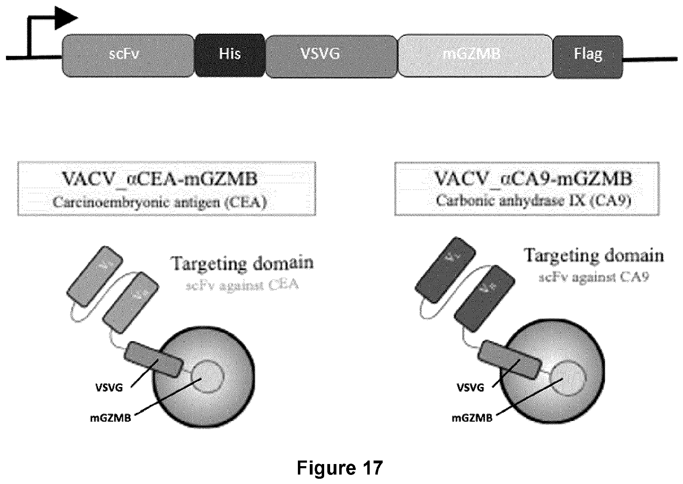

**Figure 17** shows schematic drawings of chimeric fusion constructs with single chain variable fragment (scFv) targeting moieties targeting carcinoembryonic antigen (CEA) or carbonic anhydrase IX (CA9) with a VSVG transmembrane domain and mGZMB payload. Purple, targeting domain of the construct through a single chain variable fragment (scFv) comprised of the heavy and light chains, or single domain antibodies, or nanobodies or other targeting modalities; grey, single-pass transmembrane linker domain (VSV-G); green, granzyme B payload. Note that the targeting moiety can also comprise of a peptide other than an scFv, single domain antibody or nanobody, such as the MMP2-targeted chlorotoxin, or the proteoglycan-targeted VAR2Δ. His and Flag serve the function of tags for visualization and tracking the expression of the chimeric constructs.

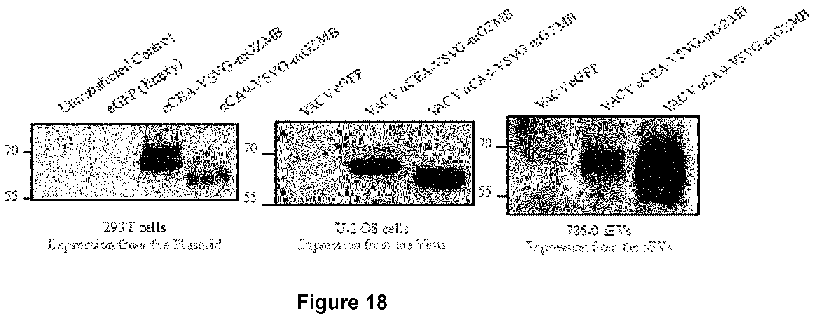

**Figure 18** shows immunoblots showing the successful expression of the constructs depicted in **Figure 17** from a pcDNA3.1 plasmid upon transfection of HEK 293T cells, viral infection of human osteosarcoma U2OS cells, and further in small EVs isolated from the virus-infected 786-0 human renal cell adenocarcinoma cells. The negative control used in these experiments is eGFP expressed in the place of the chimeric construct sequence. This shows the successful expression of the PEVs by plasmid and by virus in transfected and infected cells, respectively, and that they are successfully embedded in EVs derived from transfected and infected cells. All blots were probed with an anti-granzyme B antibody. The blots show expression of the anti-CEA-VSVG-mGZMB and the anti-CA9-VSVG-mGZMB in:

Leftmost blot - 293T cells transfected with pcDNA3.1 plasmids encoding the constructs.

Middle blot - U2OS human osteosarcoma cells infected with vaccinia virus (VACV) encoding these constructs

Rightmost blot - small extracellular vesicles (sEVs) isolated from 786-0 human renal cell adenocarcinoma cells infected with the viruses.

As expected, no signal is picked up in cells infected by the VACV-eGFP which does not express any granzyme B protein.

These blots show that not only are the chimeric granzyme B fusion constructs expressed by the plasmid, but that they are also successfully sorted and packaged in the sEVs through the transmembrane VSVG linker.

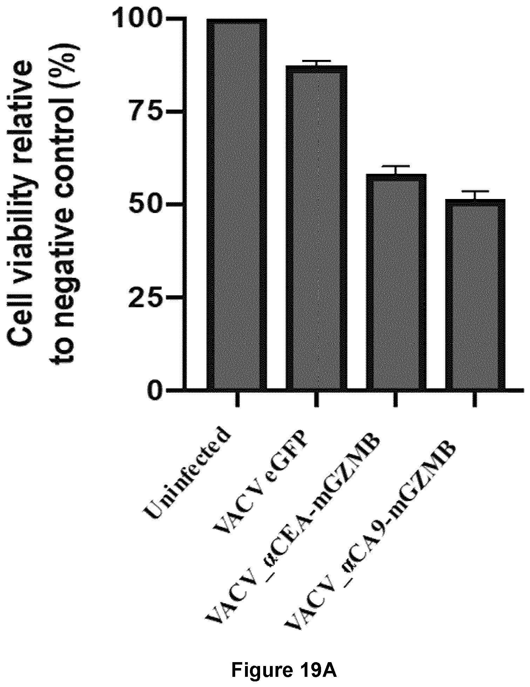

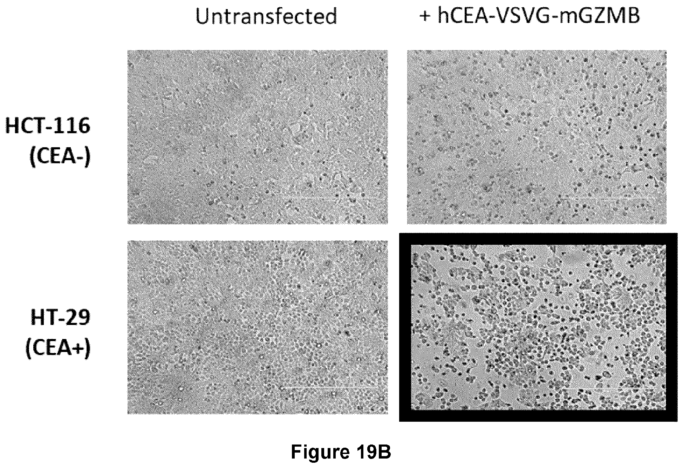

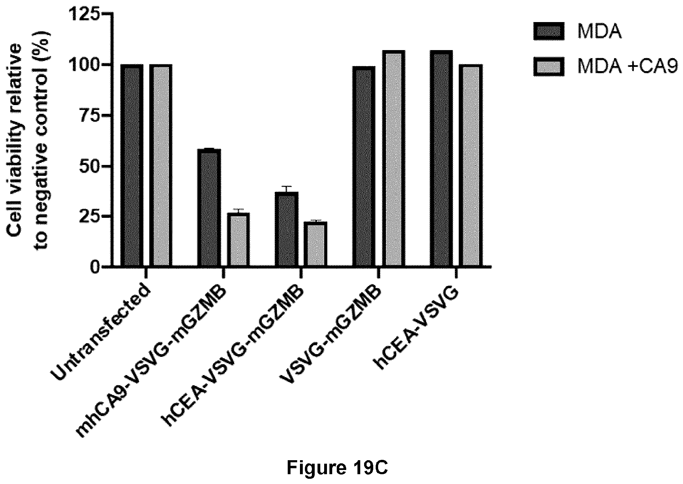

**Figure 19A****,** **19B****, and** **19C** shows that cancer cells displaying CEA or CA9 that are virally infected by vaccinia virus expressing the PEVs of **Figures 17** **and** **18** have enhanced cell death, which is mediated by the cytotoxic payload carried by the PEVs targeting CEA or CA9 on the recipient cells, respectively.

**Figure 19A****:** Cytotoxicity of vaccinia viruses encoding either human CEA or CA9 mono-targeted derived-PEVs carrying a cytotoxic granzyme B payload, in a human colon cancer cell line (HT-29) known to express high levels of CEA and CA9 on their surface. In brief, HT-29 cells were infected with the respective viruses at an MOI of 0.1. Cell viability was assessed by Alamar Blue ^{™} viability assay at 48 hours post-infection (n=4 biological replicates). While the eGFP control virus did induce cell death, there was a considerably greater degree of cell death following infection by the CEA-targeted and the CA9-targeted viruses in this cell line. This was to be expected, given the fact that HT-29s express cell-surface levels of both the CEA and CA9 antigen.

**Figure 19B****:** hCEA-VSVG-mGZMB transfected HCT116 cells produce PEVs in the supernatant upon transfection, but do not die as they do not express CEACAM5, which is required for the EV uptake. In contrast, transfected HT-29 cells, which express CEACAM5, produce EVs in the supernatant upon transfection but the majority (~60%) of the cells die from uptaking the EVs that contain GZMB.

**Figure 19C****:** Quantification of cell viability of MDA-MB-231 and MDA-MB-231-CA9 overexpressing cells that die from taking up hCEA-VSVG-mGZMB and CA9-VSVG-mGZMB containing-PEVs that they produce upon plasmid transfection of the indicated cell lines. Cells do not die upon exposure to PEV controls that lack mGZMB or an antibody for targeting.

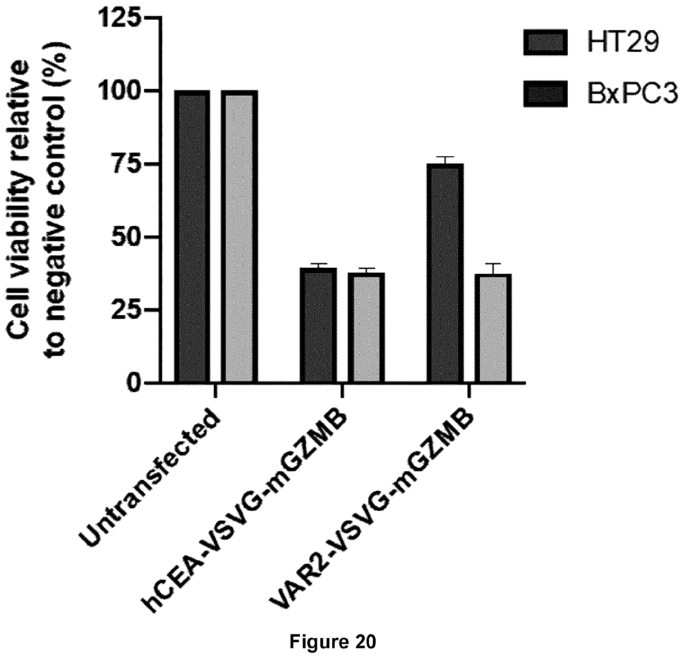

**Figure 20** shows that two cancer cell lines show enhanced cell death upon exposure to PEVs displaying VAR2Δ and carrying mGZMB. Cells were transfected with plasmids expressing VAR2-VSVG-mGZMB chimeric constructs. Quantification of cell viability of HT-29 (a human colorectal cancer cell line) and BxPC3 cells (a human pancreatic cancer cell line) that die from taking up VAR2-VSVG-mGZMB containing-PEVs that they produce upon plasmid transfection.

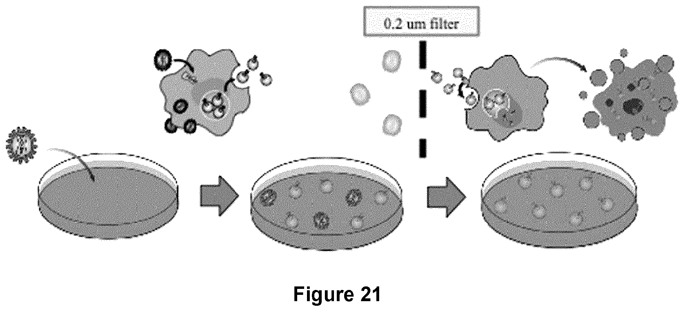

**Figure 21** is a schematic showing the methodology for supernatant transfers (See Figure 6 for data and results). In brief, 786-0 cells are seeded on Day 0 such that they will reach confluency the following day. Cells are then infected with (1) VACV-eGFP (2) VACV αCEA-VSVG-mGZMB, or (3) VACV αCA9-VSVG-mGZMB at an MOI=0.1. These cells are considered the producer cell lines for the EVs. The media on the cells are then replaced with DMEM + 10% exosome-depleted FBS 2 hours post-infection. Plates are then incubated at 37oC + 5% CO2 for 48 hours at which point which allows for replication of the viruses and production of the chimeric granzyme B constructs and their subsequent packaging and secretion in the EVs. After 48 hours, the supernatant is collected and spun down at 2000xg for 20 minutes at 4°C to remove cellular debris and dead cells. The supernatant is then passed through a 0.2 µm filter to remove vaccinia virus by size exclusion such that the filtered supernatant is free of virus and contains only EVs. The supernatant is then transferred to a recipient cell line.

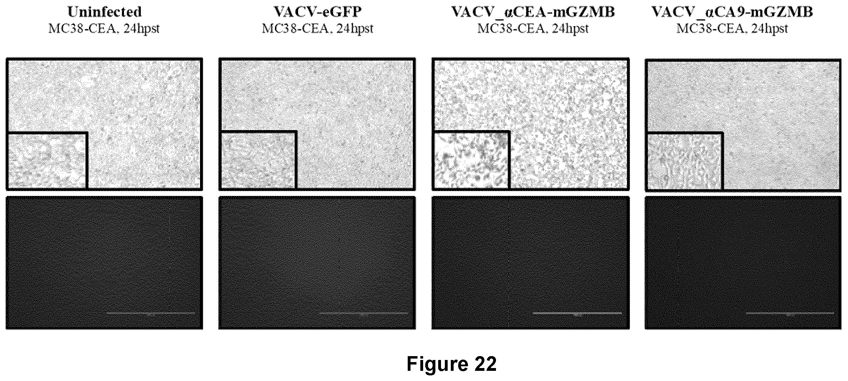

**Figure 22** shows the results of a supernatant transfer experiment as described above for 2 PEV constructs (Vaccinia virus expressing anti-CEA-mGZMB with a VSVG transmembrane domain, and vaccinia virus expressing anti-CA9-mGZMB with a VSVG transmembrane domain) The controls are uninfected cells and Vaccinia virus expressing eGFP. Following the methodology described in **Figure 21****.** All PEV constructs express eGFP. There are three controls (uninfected, eGFP only and anti-CA9 construct). The only MC38-CEA cells (mouse colorectal cancer cell line genetically engineered to express human CEA) with observable cell death are those which received the supernatant containing the hCEA-VSVG-mGZMB PEVs (3^{rd} panel), as compared with the leftmost two panels which are controls MC38 cells that received the mock (uninfected and eGFP only) and the CA9-targeted PEVs (4^{th} panel from the left- note that MC38-CEA do not express human CA9). The green channel is shown to demonstrate that no infectious viral particles passed through during the filtration step (eGFP expression from all vectors) and that the observable results are a result of the supernatant transfer alone. The fourth panel further demonstrates that the PEV targeting CA9 do not have an effect because the target cells (MC38-CEA) do not have CA9 expressed on the surface.

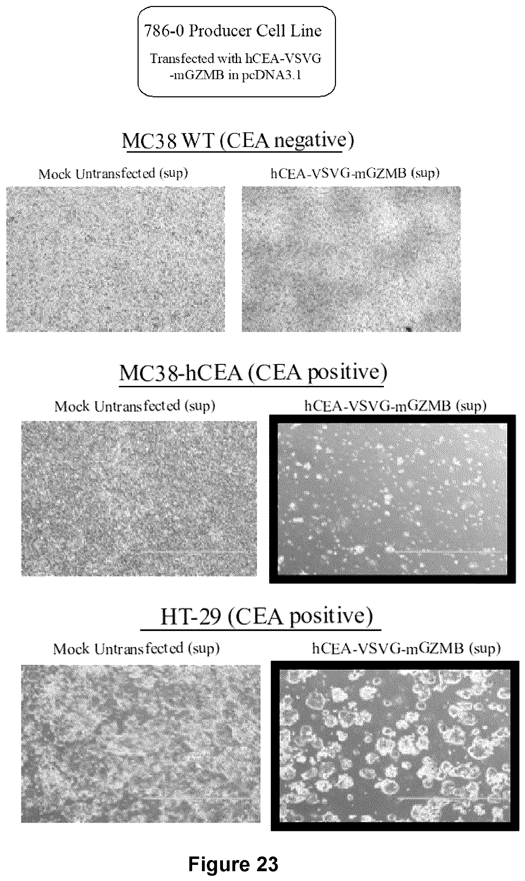

**Figure 23** shows another supernatant transfer experiment with supernatants from 786-0 cells transfected with an anti-CEA-VSVG-mGZMB plasmid, or supernatants from untransfected 786-0 cells as negative controls. The supernatants are transferred on the MC38 cells that are wild type (CEA-negative) or that expressed CEA, as well as HT-29 cells expressing CEA. 786-0 cells are used as the PEV producer cell line and transfected with the hCEA-VSVG-mGZMB plasmid. Twenty four hours later, supernatant (i.e. containing PEVs) is transferred onto cell lines that either express or don't express the target antigen, in this case hCEA. MC38 WT cells are CEA negative and showed no significant difference following reception of mock supernatant from untransfected 786-0 cells vs. supernatant from 786-0 cells transfected with hCEA-VSVG-mGZMB plasmid. Both MC38-hCEA and HT-29 cells, which are CEA-positive cell lines, demonstrated significant cell death upon receiving supernatant from 786-0s transfected with hCEA-VSVG-mGZMB compared to mock supernatant suggesting specificity of the targeting moiety in the PEVs (in the supernatant) to the target cell.

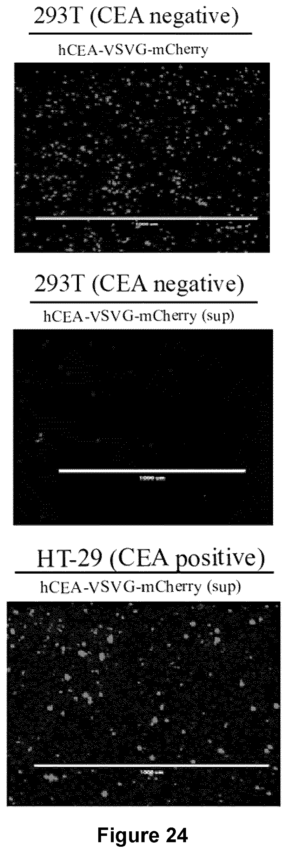

**Figure 24** shows supernatant transfer experiments with mCherry as the payload in PEVs targeting CEA on cells that are either CEA negative or CEA positive. 293T cells that are CEA negative were transfected with hCEA-VSVG-mCherry encoding-plasmids to produce hCEA-VSVG-mCherry PEVs (top panel). Supernatants from these cells were collected and used to treat fresh 293T (CEA-negative, middle panel) cells and HT-29 (CEA positive, bottom panel) cells for twenty four hours. CEA positive cells showed significant mCherry signal, suggesting that the PEVs were incorporated by the target cells only (CEA positive).

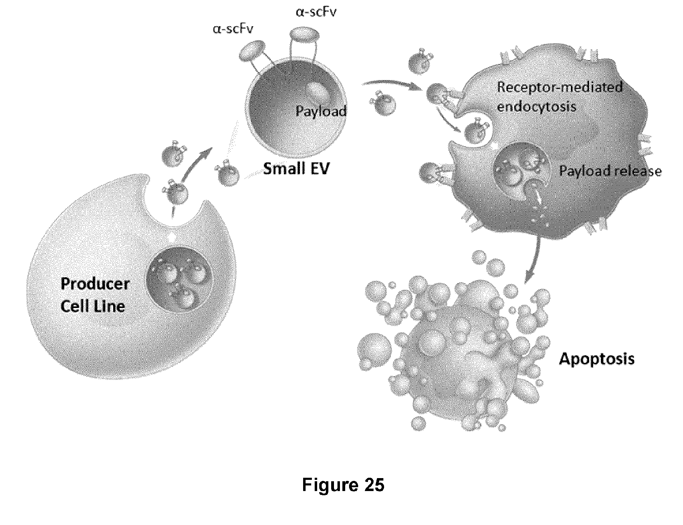

**Figure 25** shows a schematic cartoon diagram providing an overview of EV-CAR (chimeric antigen receptor) platform where the PEVs are produced from donor cells. A producer cell line that has been made to stably express the EV-CAR construct (such as through retroviral transduction) will generate EVs carrying the desired construct. In this example, the construct is made up of a CD63 transmembrane domain scaffold (tetraspanin) with at least one single chain variable fragment (scFv) targeting moiety specific to tumor associated antigens (TAAs) (e.g. CD19, CD20), and a cytotoxic payload (e.g. granzyme B). The PEVs generated by the producer cell line can be isolated and administered to patients. When the PEVs (EV-CARs) reach the tumor cell, recognition of the TAA yields receptor-mediated endocytosis to engulf the PEV. Following uptake, the PEVs release the chimeric construct/EV contents and therefore the cytotoxic payload, resulting in induction of apoptosis in the tumor cell. The Figure description provides a specific example for targeting a cancer cell.

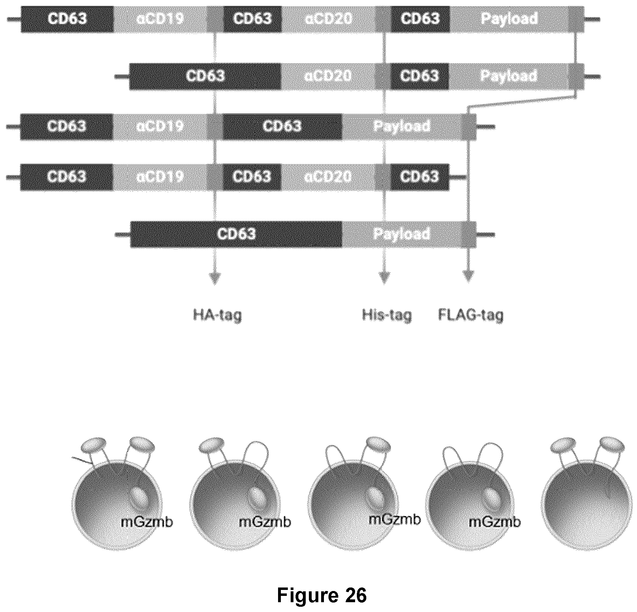

**Figure 26** shows schematic diagrams of different constructs with a tetraspanin CD63 transmembrane domain, with: one construct having two targeting moieties and a single payload, two constructs having one targeting moiety and a single payload, with the targeting moiety positioned at different positions in the construct, one construct with two targeting moieties and no payload, and a control construct with no targeting moieties and a single payload.

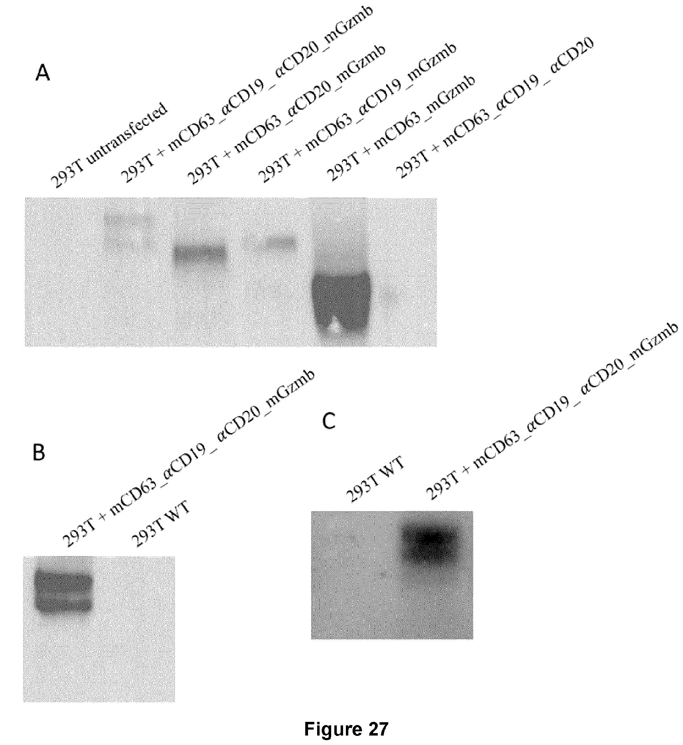

**Figure 27** shows western blots probed for granzyme B to demonstrate successful protein expression of full-length constructs depicted in **Figure 26** expressed by plasmids.

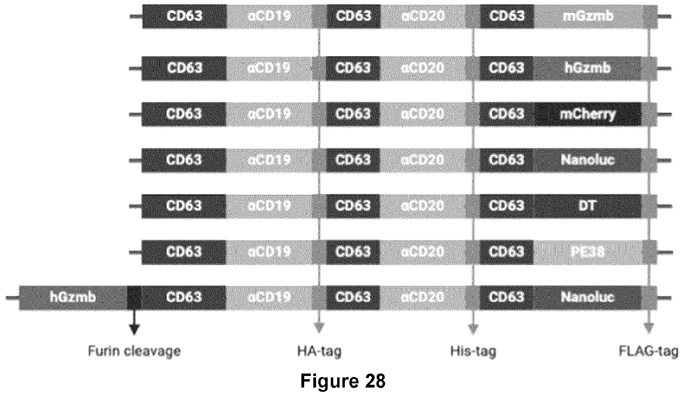

**Figure 28** shows schematic diagrams of a variety of bispecific tetraspanin-based chimeric constructs that have been prepared and are in the midst of being verified experimentally. These include 6 different payloads, and one construct with a FURIN cleavable site for a second payload on the other side of the construct.

### Example 8

### Mono-targeting & Multi-targeting - Tumor Cell Programming Payloads

Background/Context: Reprograming moieties can be specifically delivered as free cargoes (therapeutic miRNAs, mRNAs) or by binding to RNA binding proteins/domains payloads (e.g. RNA binding proteins/domains MS2, CAS13, or others), linked to surface molecule targets on specific tumor (e.g. immune cell populations, CAFs or cancer cells) via PEVs. This construct would express a targeting moiety to tailor PEVs to the desire cell type and it could concomitantly carry a single or multiple payloads and/or these constructs can be combined with specific cargoes with corresponding sequences.

Application: These platforms represent effective means to reprogram or educate (e.g. activate, phenotype change, etc.) tumor resident cells to play specific functions and thus fight cancer.

How these platforms work: For example, these PEVs could be used to augment the visibility (immunogenicity) of cancer cells to immune cells (e.g. promoting immunogenic cell death) or could be used to re-program T cell as CAR-T cells in situ in the tumor microenvironment.

Special Features: "nucleic acid ligand system" between a "RNA binding payloads (e.g. MS2, CAS13) and a therapeutic RNA molecule cargo (i.e. mRNAs, IncRNAs, microRNAs) containing the "matching" RNA binding motif (RNA ligand domain) bound by the RNA binding payload.

Targeting moieties: All the examples listed above.

Payloads: RNA binding proteins or their RNA-binding motifs (e.g. Cas13, MS2 coat protein, Staufen-1, human Pumilio-homology domain-1).

Transmembrane domain: All the examples listed in **Table 1** could be used.

**Delivery/manufacturing modalities:** Viral-based platforms (e.g. Vaccinia virus, lentivirus, adeno-associated virus [AAV], VSV, etc.). Plasmids (e.g. pcDNA 3.1) and free PEVs.

### Results:

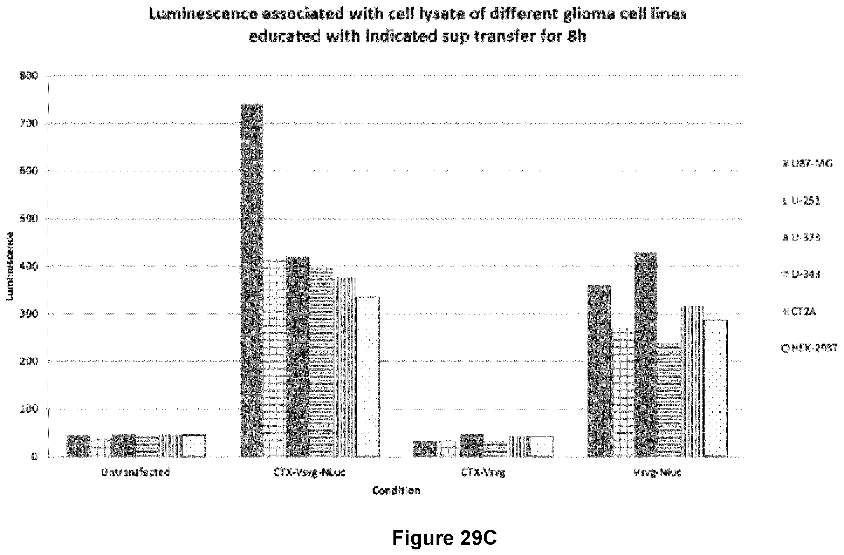

**Figures 29C** shows that PEVs containing CTX-VSVG-Nanoluc^{™} are able to specifically re-program receiving cells to "light up" during an enzymatic reaction (Nanoluc^{™} is an enzyme) with luciferin (substrate). Note that Nanoluc^{™} is an enzyme and this data shows not only the targeted delivered of Nanoluc^{™} via PEVs but also the delivery of a functional enzyme.

**Figure 13** shows that the CdaA enzyme can be specifically delivered to DCs via PEVs and that, once in the recipient cells, the enzyme is functionally active.

### Example 9

### Cargo co-expression (can be combined with various PEV constructs)

Background/Context: The term "cargo" is defined herein as a molecule that is co-expressed with but it is not part of the chimeric protein construct. As such, cargo can be included/co-expressed whether or not there is a payload in the construct. Cargo can be nucleic acids or proteins that are preferentially directed to EVs, and/or RNAs that may include a special sequence recognized by a specific RNA-recognizing payload included in the PEV construct.

Application: Can be for any application where it might be suitable to target molecules to the target cell, particularly if they cannot maintain activity/function in the PEV construct. In other cases, could promote specific delivery of an EV cargo molecule.

Special Features: RNA can either have an RNA-binding motif recognition site to bind to RNA-binding motif payload or have an EV-directing motif. Proteins may be preferentially directed to EVs by way of specific sequences that are known in the art to target them.

Targeting moieties: Various, depending on situation

Payload: RNA-binding motif, for instances where the cargo has RNA-binding motif recognition sequence.

To produce EVs that are tailored and concomitantly carry specific nucleic acid molecules of interest (cargos; e.g. microRNAs and mRNAs) a PEV can be designed to carry a payload that contains 1 or more RNA-binding domains (RBDs). The RNA cargo can display the binding motif recognized by the RBD (also referred to as the RNA ligand domain) and thus specifically interact and be carried by the PEV containing RBDs ("RNA nucleic acid ligand system" system). For example, well characterized RNA-binding domains found in cellular RNA binding proteins such as the RRM, KH, cold shock domain (CSD), and the zinc finger CCHC domains could be used. Similarly, RNA-binding domains found in Viral coat or capsid proteins (e.g. the MS2 bacteriophage coat protein) or bacterial RNA-binding Cas proteins (e.g. Cas13) can be designed as part payloads in PEV constructs that function as RNA-carrying or nucleic acid ligand systems. The cognate RNA binding ligands will be included in the RNA cargo molecules.

Transmembrane domain: the PEV can contain any transmembrane domain according to the other categories outlined in this document- cargos are not part of the presently described chimeric construct

**Table 10: Example Constructs**

| **Cargo type** | **Example** | **Description** |
|---|---|---|
| mRNA | mRNA for anti-cd19 and or CD22 | T cell can be reprogramed in situ in the tumor to function as CAR-T cells by programing them via EVs load with RNA binding-PEVs carrying for example anti-CD19 mRNA molecules. |
| miRNA | miRNA targeting PD-L1 | Specific downregulation of the mRNA targets |
| | miRNA targeting ARID1A | |

### Example 10

### Control Constructs

These constructs are used as controls for various experiments in multiple categories. Some of these are independently proof of concept constructs, e.g. functional payload delivery (mCherry or Nanoluc^{™}) or placement of targeting molecules within tetraspanin transmembrane domains for a single target.

**Table 11: Proof-of-concept PEV Constructs - Controls**

| **Full Name** | **Targeting Molecule** | **Payload** | **TM Domain** | **Activity** |
|---|---|---|---|---|
| CTX-VSVG-mCherry | CTX | mCherry | VSVG | MMP2-targeted PEVs carrying a fluorescent marker |
| CTX- VSVG-Nanoluc^{™} | CTX | Nanoluc^{™} | VSVG | MMP2-targeted PEVs carrying a luminescence enzyme marker |
| Anti-CD19+anti-CD20-CD63-mCherry | α-hCD20, α-hCD19 | mCherry | mCD63 | Bi-specific PEV targeting human CD19 and CD20 and carrying the red fluorescent marker, mCherry |
| Anti-CD19+anti-CD20-CD63-Nanoluc^{™} | α-hCD20, α-hCD19 | Nanoluc^{™} | mCD63 | Bi-specific PEV targeting human CD19 and CD20 and carrying the bioluminescent marker, Nanoluc^{™} |
| Anti-mCLEC9a- VSVG-mCherry | α-mCLEC9a | mCherry | VSVG | PEV targeting CLEC9a and carrying the red fluorescent marker, mCherry |
| anti-mCLEC9a- VSVG-Nanoluc^{™} | α-mCLEC9a | Nanoluc^{™} | VSVG | PEV targeting CLEC9a and carrying the red fluorescent marker, Nanoluc^{™} |
| Anti-mDEC205- VSVG-mCherry | α-mDEC205 | mCherry | VSVG | PEV targeting mouse DEC205 and carrying the red fluorescent marker, mCherry |
| Anti-mDEC205- VSVG-Nanoluc^{™} | α-mDEC205 | Nanoluc^{™} | VSVG | PEV targeting mouse DEC205 and carrying the bioluminescent marker, Nanoluc^{™} |
| Anti-mCTLA4- VSVG-mCherry | α-m9D9 | mCherry | VSVG | PEV targeting CTL4 and carrying the red fluorescent marker, mCherry |
| Anti-mCTLA4- VSVG-Nanoluc^{™} | α-m9D9 | Nanoluc^{™} | VSVG | PEV targeting CTL4 and carrying the bioluminescent marker, Nanoluc^{™} |
| Anti-CA9-VSVG-mCherry | α-mhCA9 | mCherry | VSVG | PEV targeting human CA9 and carrying the red fluorescent marker, mCherry |
| Anti-CA9-VSVG-Nanoluc^{™} | α-mhCA9 | Nanoluc^{™} | VSVG | PEV targeting human CA9 and carrying the bioluminescent marker, Nanoluc^{™} |
| anti-CEA-VSVG-mCherry | α-mhCEA | mCherry | VSVG | PEV targeting human CEA and carrying the fluorescent marker, mCherry |
| anti-CEA- VSVG-Nanoluc^{™} | α-mhCEA | Nanoluc^{™} | VSVG | PEV targeting human CEA and carrying the bioluminescent marker, Nanoluc^{™} |
| VAR2Δ-VSVG-mCherry | VAR2Δ | mCherry | VSVG | PEV targeting oncofetal chondroitin sulfate and carrying the red fluorescent marker, mCherry |
| VAR2Δ-VSVG-Nanoluc^{™} | VAR2Δ | Nanoluc^{™} | VSVG | PEV targeting oncofetal chondroitin sulfate and carrying the bioluminescent marker, Nanoluc^{™} |
| GE11-mCherry | GE11 | mCherry | VSVG | PEV targeting EGFR and carrying the fluorescent marker, mCherry |
| GE11-VSVG-Nanoluc^{™} | GE11 | Nanoluc^{™} | VSVG | PEV targeting EGFR and carrying the bioluminescent marker, Nanoluc^{™} |
| Anti-CD19+anti-CD20-CD63 | α-hCD20, α-hCD19 | mCD63 | NONE | Bi-specific PEV targeting human CD19 and CD20 and carrying no payload |
| anti- FAP-VSVG-mCherry | α-mhFAP | mCherry | VSVG | PEV targeting the fibroblast activating protein (FAP) and carrying the fluorescent marker, mCherry |
| anti-FAP-VSVG-Nanoluc^{™} | α-mhFAP | Nanoluc^{™} | VSVG | PEV targeting. FAP and carrying the bioluminescent marker, Nanoluc^{™} |

### Results:

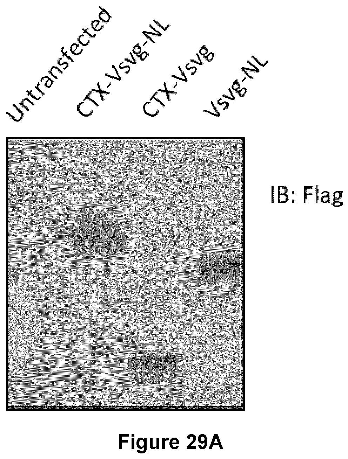

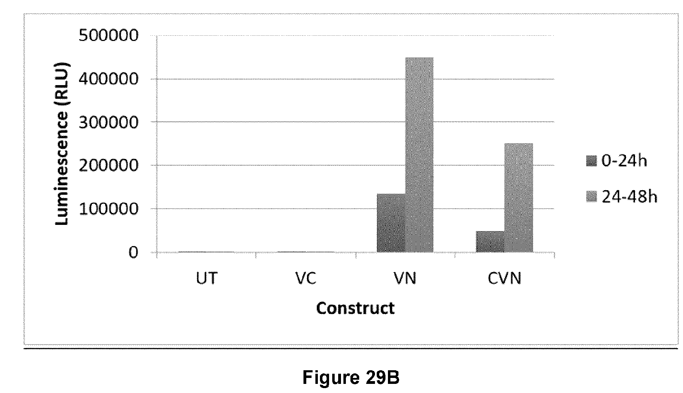

**Figures 29A****,** **29B****, and** **29C** are proof of concept, showing that PEVs targeting MMP-2 on the surface of cancer cells (PEV targeting moiety: CTX) can deliver a functional payload, in this case the enzyme Nanoluc^{™}.

**Figure 29A****:** HEK293T cells were untransfected or transfected with indicated PEV plasmids (Flag-tagged) with or without chlorotoxin (CTX) targeting moiety and a reporter payload (Nanoluc^{™}). All constructs contain the VSVG transmembrane domain and a FLAG tag in the C-terminus. Cells lysates were collected 24 h post-transfection and immunoblotted for indicated antibodies (Flag, or beta-actin as loading control). Data shows expression of the desired experimental construct (CTX-VSVG- Nanoluc^{™}) and its respective negative controls [VSVG-Nanoluc^{™} (no targeting moiety) or CTX-VSVG (no payload)].

**Figure 29B****:** HEK293T cells were transfected as indicated in (A) and cultured in EV-depleted media. After 24 h and 48h, supernatants were collected and luminescence levels were measured using a luminometer and a standard Luciferase detection assays. Abbreviations: UT: untransfected; VC: CTX-VSVG construct; VN: VSVG- Nanoluc^{™} construct; CVN: CTX-VSVG- Nanoluc^{™} construct. Note that Nanoluc^{™} signal is only detected in supernatants (containing EVs) derived from cells transfected with the VSVG- Nanoluc^{™} and CTX-VSVG-Nanoluc^{™} constructs.

**Figure 29C****:** Supernatants collected from transfected HEK293T cells at 48hrs post-transfection were used to treat various human glioblastoma cell lines. After 8 hours of conditioned media transfer, luminescence in cell lysates was measured using a Nanoluc^{™} substrate (luciferase assay) and a luminometer. Note that uptake of CTX-VSVG-Nanoluc^{™}-displaying EVs was particularly enhanced in U87MG cells. This cell line expresses high levels of MMP-2 (the protein that CTX binds to).

### Example 10

### Viral Infection Increases EV Secretion

It has been demonstrated that virus infection (e.g. Vaccinia virus infection) increases small EV secretion.

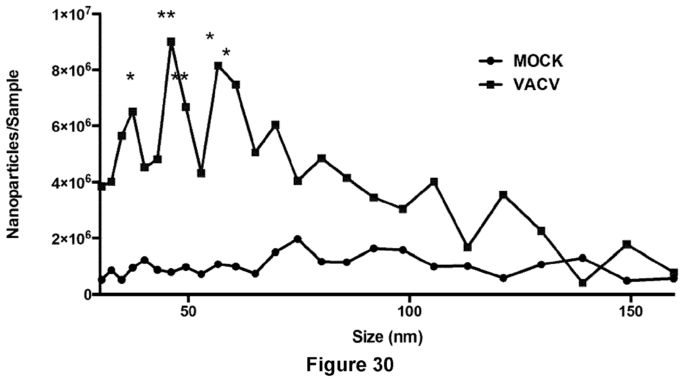

**Figure 30** depicts a graph showing that total small EVs produced from 786-0 cancer cell line uninfected (mock) or infected with vaccinia virus (VacV) were analyzed using a nanoparticle tracking analysis system (ZetaView^{®} software). **P<0.05, **P<0.01.*

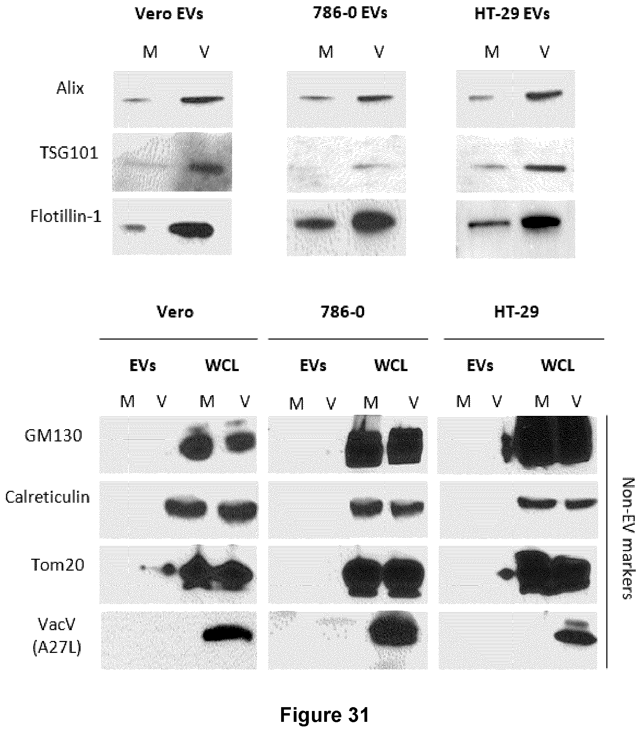

**Figure 31** depicts Western blot analysis of EVs and whole cell lysates (WCLs) from Vero, 786-0, and HT-29 cells following mock infection (M), or CopWT infection (VACV) (V) at an MOI of 1, 48 hours post-infection. The membranes were probed for EV markers (Alix, TSG101, and Flotillin-1), cellular/non-EV markers (GM130, Calreticulin, Tom20), and the A27L protein of VacV.

### Example 11

### Alternative viral glycoproteins as EV-directed transmembrane polypeptides

### Results:

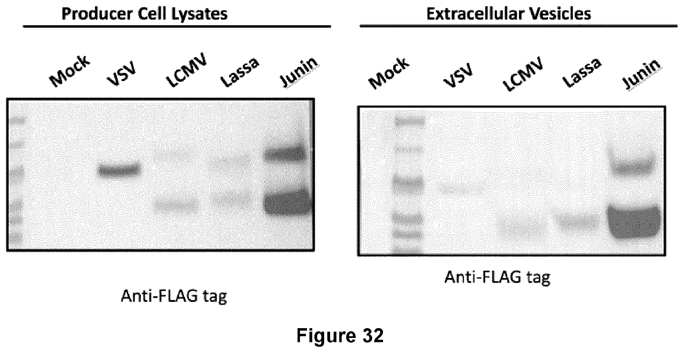

**Figure 32** depicts Western blots showing the expression of four different constructs upon cell transfection and in isolated EV fractions, showing that the viral glycoproteins (G) derived from VSV, LCMV (lymphocytic choriomeningitis virus), Lassa (Lassa fever virus), and Junin virus (also known as Argentinian mammarenavirus) can be used as the transmembrane domain to shuttle payloads (Flag tag) into EVs. In brief, HEK293 T cells were transfected with pCDNA 3.1 plasmids expressing VSV-G, LCMV-G, Lassa virus-G, Junin virus-G, or empty vector as control (mock). After 48 hours, cell lysates were collected using RIPA buffer and EVs were collected from the supernatant of transfected cells using EXO-quick-TC reagent (System Biosciences) as per the manufacture's protocol. Then, cell lysates and purified EVs were SDS-PAGE and western blotted with anti-Flag antibody. Of note, all glycoprotein constructs display a Flag tag in their C-terminus for easy detection.

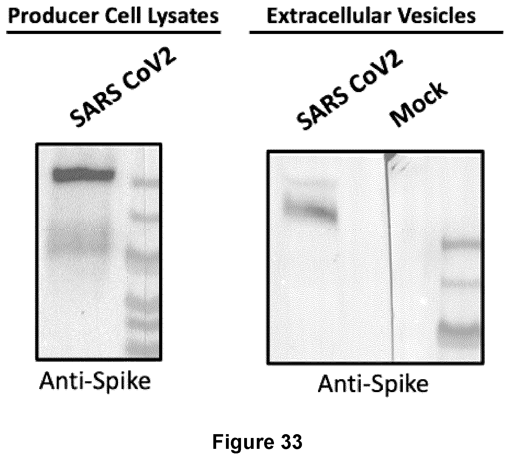

**Figure 33** depicts a Western blot showing the expression of a construct upon cell transfection and in isolated EV fractions, showing that the viral glycoprotein derived from SARS-CoV-2 can be used as the transmembrane domain to shuttle payloads into EVs. In brief, HEK293 T cells were transfected with a pcDNA 3.1 plasmid expressing the SARS-CoV-2 Spike protein or an empty vector as control (mock). After 48 hours, cell lysates were collected using RIPA buffer and EVs were collected from the supernatant of transfected cells using EXO-quick-TC reagent (System Biosciences) as per the manufacture's protocol. Then, cell lysates and purified EVs were SDS-PAGE and western blotted with anti-Spike antibody.

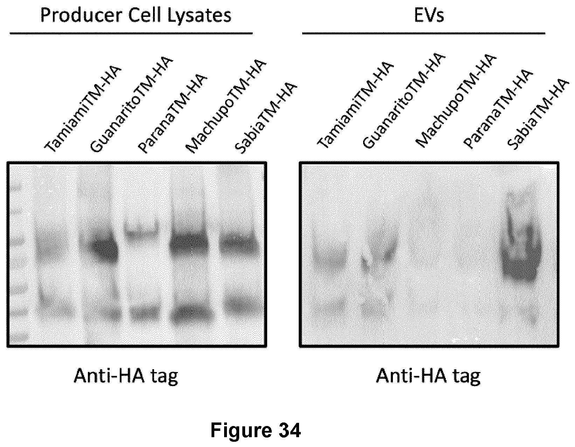

**Figure 34** depicts Western blots showing the expression of four different constructs upon cell transfection and in isolated EV fractions, showing that the viral glycoproteins derived from the Tamiami, Guanarito, Paraná, Machupo, and Sabia viruses can be used as the transmembrane domain to shuttle payloads (Flag tag) into EVs. In brief, HEK293 T cells were transfected with pcDNA 3.1 plasmids expressing Tamiami virus-G, Guanarito virus-G, Paraná virus-G, Machupo virus-G, Sabia virus G or empty vector as control (mock). After 48 hrs, cell lysates were collected using RIPA buffer and EVs were collected from the supernatant of transfected cells using EXO-quick-TC reagent (System Biosciences) as per the manufacture's protocol. Then, cell lysates and purified EVs were SDS-PAGE and western blotted with anti-HA antibody. Of note, all glycoprotein constructs display HA tags in their C-terminus for easy detection.

### Example 12

### Cargo co-expression

As previously described in Example 9.

Background/Context: Cargo can be included/co-expressed whether or not there is a payload in the construct.

Application: Can be for any application where it might be suitable to target molecules to the target cell, particularly if they cannot maintain activity/function in the PEV construct. In other cases, could promote specific delivery of an EV cargo molecule to the target cell.

Special Features: Cargo RNA molecules can either have an RNA-binding motif recognition site to bind to RNA-binding motif payload or have an EV-directing motif. Proteins may be preferentially directed to EVs by way of specific sequences that are known in the art to target them.

Targeting moieties: Various, depending on the application.

Payload: RNA-binding motif, for instances where the cargo has an RNA-binding motif recognition sequence.

To produce EVs that are tailored and concomitantly carry specific nucleic acid molecules of interest (cargos, e.g., microRNAs and mRNAs) a PEV can be designed to carry a payload that contains 1 or more RNA-binding domains (RBDs). The RNA cargo can display the binding motif recognized by the RBD (also referred to as the RNA ligand domain) and thus specifically interact and be carried by the PEV containing RBDs ("RNA nucleic acid ligand system" system). For example, well characterized RNA-binding domains found in cellular RNA binding proteins such as the RRM domain, K homology (KH) domain, cold shock domain (CSD), and the zinc finger CCHC domains could be used. Similarly, RNA-binding domains found in viral coat or capsid proteins (e.g., the MS2 bacteriophage coat protein) or bacterial RNA-binding Cas proteins (e.g., Cas13) can be designed as part payloads in PEV constructs that function as RNA-carrying or nucleic acid ligand systems. The cognate RNA binding ligands will be included in the RNA cargo molecules.

Transmembrane domain: the PEV can contain any transmembrane domain according to the other categories outlined in this document- cargos are not part of the presently described chimeric construct.

### Results:

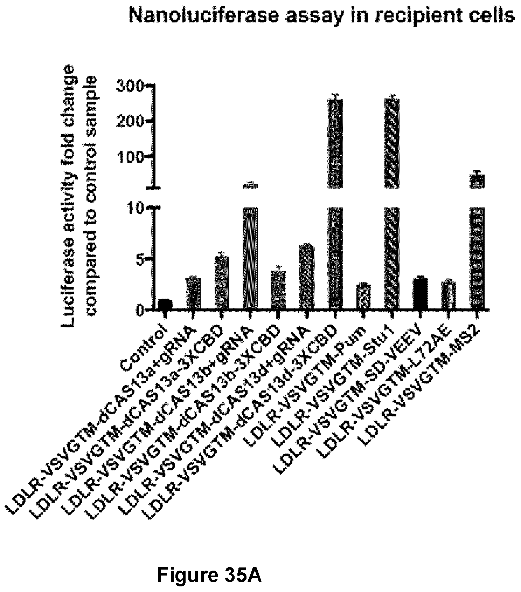

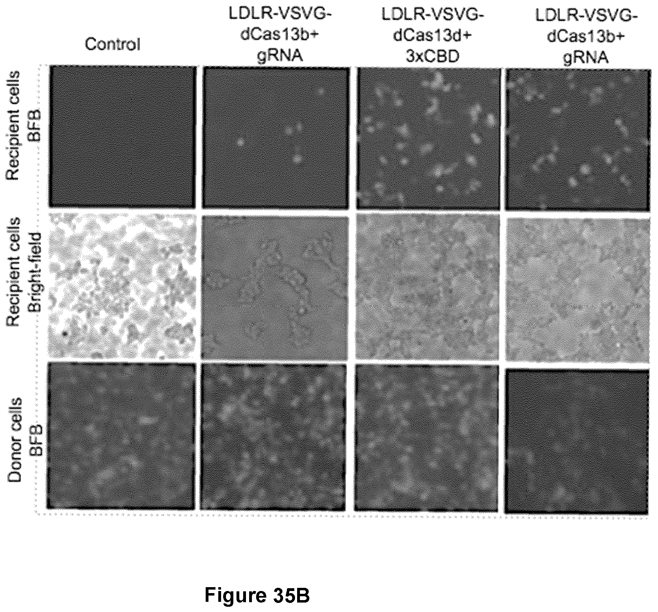

Table 12 provides amino acid motifs (RNA binding motif) that were experimentally shown in **Figure 35** to specifically bind to specific target RNA sequences. RNA sequences are selectively recognized by their respective RNA binding motif and do not exist in the human genome, as determined by bioinformatic analysis. These RNA binding motifs can be used as payloads as described in the present invention, and as shown in the Figures below.

**Table 12: RNA Binding Domain-containing Constructs and Target RNA Sequences**

| **Name** | **Construct Sequence (RNA Binding Motif Underlined)** | **Target RNA Sequences** |
|---|---|---|
| **VSVG-dCas13a** | MKCLLYLAFLFIGVNCKFTIVFPHNQKG NWKNVPSNYHYCPSSSDLNWHNDLIGTA LQVKMPKSHKAIQADGWMCHASKWVTTC DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSMKVTKV DGISHKKYIEEGKLVKSTSEENRTSERL SELLSIRLDIYIKNPDNASEEENRIRRE NLKKFFSNKVLHLKDSVLYLKNRKEKNA VQDKNYSEEDISEYDLKNKNSFSVLKKI LLNEDVNSEELEIFRKDVEAKLNKINSL KYSFEENKANYQKINENNVEKVGGKSKR NIIYDYYRESAKRNDYINNVQEAFDKLY KKEDIEKLFFLIENSKKHEKYKIREYYH KIIGRKNDKENFAKIIYEEIQNVNNIKE LIEKIPDMSELKKSQVFYKYYLDKEELN DKNIKYAFCHFVEIEMSQLLKNYVYKRL SNISNDKIKRIFEYQNLKKLIENKLLNK LDTYVRNCGKYNYYLQVGEIATSDFIAR NRQNEAFLRNIIGVSSVAYFSLRNILET | GATTTAGACTACCCCAAAAACGAAG GGGACTAAAACGGAATTCGAGCTCG GTACCTTCCCGGGTTCATTAGAGAT TTAGACTACCCCAAAAACGAAGGGG ACTAAAACGTCTGCAGGTCGACTCT AGAAAGATTTAGACTACCCCAAAAA CGAAGGGGACTAAAAC |
| | ENENGITGRMRGKTVKNNKGEEKYVSGE VDKIYNENKQNEVKENLKMFYSYDFNMD NKNEIEDFFANIDEAISSIAHGIVHFNL ELEGKDIFAFKNIAPSEISKKMFQNEIN EKKLKLKIFKQLNSANVFNYYEKDVIIK YLKNTKFNFVNKNIPFVPSFTKLYNKIE DLRNTLKFFWSVPKDKEEKDAQIYLLKN IYYGEFLNKFVKNSKVFFKITNEVIKIN KQRNQKTGHYKYQKFENIEKTVPVEYLA IIQSREMINNQDKEEKNTYIDFIQQIFL KGFIDYLNKNNLKYIESNNNNDNNDIFS KIKIKKDNKEKYDKILKNYEKHNRNKEI PHEINEFVREIKLGKILKYTENLNMFYL ILKLLNHKELTNLKGSLEKYQSANKEET FSDELELINLLNLDNNRVTEDFELEANE IGKFLDFNENKIKDRKELKKFDTNKIYF DGENIIKHRAFYNIKKYGMLNLLEKIAD KAKYKISLKELKEYSNKKNEIEKNYTMQ QNLHRKYARPKKDEKFNDEDYKEYEKAI GNIQKYTHLKNKVEFNELNLLQGLLLKI LHRLVGYTSIWERDLRFRLKGEFPENHY IEEIFNFDNSKNVKYKSGQIVEKYINFY KELYKDNVEKRSIYSDKKVKKLKQEKKD LYIANYIAHFNYIPHAEISLLEVLENLR KLLSYDRKLKNAIMKSIVDILKEYGFVA TFKIGADKKIEIQTLESEKIVHLKNLKK KKLMTDRNSEELCELVKVMFEYKALEAA ARV | |
| **VSVG-dCas13b** | MKCLLYLAFLFIGVNCKFTIVFPHNQKG NWKNVPSNYHYCPSSSDLNWHNDLIGTA LQVKMPKSHKAIQADGWMCHASKWVTTC DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN | GTTGTGGAAGGTCCAGTTTTGAGGG GCTATTACAACGGAATTCGAGCTCG GTACCTTCCCGGGTTCATTAGAGTT GTGGAAGGTCCAGTTTTGAGGGGCT ATTACAACGTCTGCAGGTCGACTCT AGAAAGTTGTGGAAGGTCCAGTTTT GAGGGGCTATTACAAC |
| | YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSMNIPAL VENQKKYFGTYSVMAMLNAQTVLDHIQK VADIEGEQNENNENLWFHPVMSHLYNAK NGYDKQPEKTMFIIERLQSYFPFLKIMA ENQREYSNGKYKQNRVEVNSNDIFEVLK RAFGVLKMYRDLTNAYKTYEEKLNDGCE FLTSTEQPLSGMINNYYTVALRNMNERY GYKTEDLAFIQDKRFKFVKDAYGKKKSQ VNTGFFLSLQDYNGDTQKKLHLSGVGIA LLICLFLDKQYINIFLSRLPIFSSYNAQ SEERRIIIRSFGINSIKLPKDRIHSEKS NKSVAMDMLNEVKRCPDELFTTLSAEKQ SRFRIISDDHNEVLMKRSSDRFVPLLLQ YIDYGKLFDHIRFHVNMGKLRYLLKADK TCIDGQTRVRVIEQPLNGFGRLEEAETM RKQENGTFGNSGIRIRDFENMKRDDANP ANYPYIVDTYTHYILENNKVEMFINDKE DSAPLLPVIEDDRYVVKTIPSCRMSTLE IPAMAFHMFLFGSKKTEKLIVDVHNRYK RLFQAMQKEEVTAENIASFGIAESDLPQ KILDLISGNAHGKDVDAFIRLTVDDMLT DTERRIKRFKDDRKSIRSADNKMGKRGF KQISTGKLADFLAKDIVLFQPSVNDGEN KITGLNYRIMQSAIAVYDSGDDYEAKQQ FKLMFEKARLIGKGTTEPHPFLYKVFAR SIPANAVEFYERYLIERKFYLTGLSNEI KKGNRVDVPFIRRDQNKWKTPAMKTLGR | |
| | IYSEDLPVELPRQMFDNEIKSHLKSLPQ MEGIDFNNANVTYLIAEYMKRVLDDDFQ TFYQWNRNYRYMDMLKGEYDRKGSLQHC FTSVEEREGLWKERASRTERYRKQASNK IRSNRQMRNASSEEIETILDKRLSNSRN EYQKSEKVIRRYRVQDALLFLLAKKTLT ELADFDGERFKLKEIMPDAEKGILSEIM PMSFTFEKGGKKYTITSEGMKLKNYGDF FVLASDKRIGNLLELVGSDIVSKEDIME EFNKYDQCRPEISSIVFNLEKWAFDTYP ELSARVDREEKVDFKSILKILLNNKNIN KEQSDILRKIRNAFDANNYPDKGVVEIK ALPEIAMSIKKAFGEYAIMKGSLQLPPL ERLTLGSSYPYDVPDYAYPYDVPDYAYP YDVPDYA | |
| **VSVG-dCas13d** | MKCLLYLAFLFIGVNCKFTIVFPHNQKG NWKNVPSNYHYCPSSSDLNWHNDLIGTA LQVKMPKSHKAIQADGWMCHASKWVTTC DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSEASIEK KKSFAKGMGVKSTLVSGSKVYMTTFAEG SDARLEKIVEGDSIRSVNEGEAFSAEMA | GAACCCCTACCAACTGGTCGGGGTT TGAAACGGAATTCGAGCTCGGTACC TTCCCGGGTTCATTAGAGAACCCCT ACCAACTGGTCGGGGTTTGAAACGT CTGCAGGTCGACTCTAGAAAGAACC CCTACCAACTGGTCGGGGTTTGAAA C |
| | DKNAGYKIGNAKFSHPKGYAVVANNPLY TGPVQQDMLGLKETLEKRYFGESADGND NICIQVIHNILDIEKILAEYITNAAYAV NNISGLDKDIIGFGKFSTVYTYDEFKDP EHHRAAFNNNDKLINAIKAQYDEFDNFL DNPRLGYFGQAFFSKEGRNYIINYGNEC YDILALLSGLAHWVVANNEEESRISRTW LYNLDKNLDNEYISTLNYLYDRITNELT NSFSKNSAANVNYIAETLGINPAEFAEQ YFRFSIMKEQKNLGFNITKLREVMLDRK DMSEIRKNHKVFDSIRTKVYTMMDFVIY RYYIEEDAKVAAANKSLPDNEKSLSEKD IFVINLRGSFNDDQKDALYYDEANRIWR KLENIMHNIKEFRGNKTREYKKKDAPRL PRILPAGRDVSAFSKLMYALTMFLDGKE INDLLTTLINKFDNIQSFLKVMPLIGVN AKFVEEYAFFKDSAKIADELRLIKSFAR MGEPIADARRAMYIDAIRILGTNLSYDE LKALADTFSLDENGNKLKKGKHGMRNFI INNVISNKRFHYLIRYGDPAHLHEIAKN EAVVKFVLGRIADIQKKQGQNGKNQIDR YYETCIGKDKGKSVSEKVDALTKIITGM NYDQFDKKRSVIEDTGRENAEREKFKKI ISLYLTVIYHILKNIVNINARYVIGFHC VERDAQLYKEKGYDINLKKLEEKGFSSV TKLCAGIDETAPDKRKDVEKEMAERAKE SIDSLESANPKLYANYIKYSDEKKAEEF TRQINREKAKTALNAYLRNTKWNVIIRE DLLRIDNKTCTLFANKAVALEVARYVHA YINDIAEVNSYFQLYHYIMQRIIMNERY EKSSGKVSEYFDAVNDEKKYNDRLLKLL CVPFGYCIPRFKNLSIEALFDRNEAAKF DKEKKKVSGNSGSGAAARV | |
| **VSVG-Pum** | MKCLLYLAFLFIGVNCKFTIVFPHNQKG NWKNVPSNYHYCPSSSDLNWHNDLIGTA LQVKMPKSHKAIQADGWMCHASKWVTTC | TGGAATTCGAGCTCGGTACCTTCCC GGGTTCATTAGATCCTAAGGTTCAT ATAATCGTTGTCCAGAATTGTATAT |
| | DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSMGRSRL LEDFRNNRYPNLQLREIAGHIMEFSQDQ HGSRFIQLKLERATPAERQLVFNEILQA AYQLMVDVFGNYVIQKFFEFGSLEQKLA LAERIRGHVLSLALQMYGCRVIQKALEF IPSDQQNEMVRELDGHVLKCVKDQNGNH VVQKCIECVQPQSLQFIIDAFKGQVFAL STHPYGCRVIQRILEHCLPDQTLPILEE LHQHTEQLVQDQYGNYVIQHVLEHGRPE DKSKIVAEIRGNVLVLSQHKFASNVVEK CVTHASRTERAVLIDEVCTMNDGPHSAL YTMMKDQYANYVVQKMIDVAEPGQRKIV MHKIRPHIATLRKYTYGKHILAKLEKYY MKNGVDLGGSGYPYDVPDYA | ATTCGTGCAGGTCGACTCTAGATCA TATAATCGTTGTCCAGAATTGTATA TATTCGTTGGCACTGGCGTCGTTCA TATAATCGTTGTCCAGAATTGTATA TATTCG |
| **VSVG-Stu1** | MKCLLYLAFLFIGVNCKFTIVFPHNQKG NWKNVPSNYHYCPSSSDLNWHNDLIGTA LQVKMPKSHKAIQADGWMCHASKWVTTC DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN | CATTAGATCCTAAGGTGAGTGCCAG AAGCTGCCTCGATTCAACGAGGCAG TTTCTGGTACTCTGCAGGTCGACTC TAGAGAGTGCCAGAAGCTGCCTCGA TTCAACGAGGCAGTTTCTGGTACTC TTGGCACTGGCGTCGTGAGTGCCAG |
| | LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSNLNKSE ISQVFEIALKRNLPVNFEVARESGPPHM KNFVTKVSVGEFVGEGEGKSKKISKKNA AIAVLEELKKLPPLPAVERVKPRIKKKT KPIVKPQTSPEYGQGINPISRLAQIQQA KKEKEPEYTLLTERGLPRRREFVMQVKV GNHTAEGTGTNKKVAKRNAAENMLEILG FKVPQRQGSGYPYDVPDYA | AAGCTGCCTCGATTCAACGAGGCAG TTTCTGGTACTC |
| **VSVG-VEEV** | MKCLLYLAFLFIGVNCKFTIVFPHNQKG NWKNVPSNYHYCPSSSDLNWHNDLIGTA LQVKMPKSHKAIQADGWMCHASKWVTTC DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII | AGTGGCGGCTTAATTAAATTACACA TGTCGGTGTGAGACTATAGTTAGTT GCGACGGGTACGTCGTTAAAAGAAT AGCTATCAGTCCAGGCCTGTATGGG AAGCCTTCAGGCTATGCTGCTACGA TGCACCGCGAGGGATTCTTGTGCTG CAAAGTGACAGACACATTGAACGGG GAGAGGGTCTCTTTTCCCGTGTGCA CGTATGTGCCAGCTACATTGTGTGA CCAAATGACTGGCATACTGGCAACA GATGTCAGTGCGGACGACGCGCAAA AACTGCTGGTTGGGCTCAACCAGCG CATAGTCGTCAAAGCG |
| | GLFLVLRVGIHLCIKLKHTKKRQIYTDI EMNRLGKAIAALTATRSSGSGSMFPFQP MYPMQPMPYRNPFAAPRRPWFPRTDPFL AMQVQELTRSMANLTFKQRRDAPPEGPP AKKPKREAPQKQKGGGQGKKKKNQGKKK AKTGPPNPKAQSGNKKKPNKKPGKRQRM VMKLESDKGSGGSGYPYDVPDYAYPYDV PDYAYPYDVPDYA | |
| **VSVG-L72AE** | MKCLLYLAFLFIGVNCKFTIVFPHNQKG NWKNVPSNYHYCPSSSDLNWHNDLIGTA LQVKMPKSHKAIQADGWMCHASKWVTTC DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSMYVRFE VPEDMQNEALSLLEKVRESGKVKKGTNE TTKAVERGLAKLVYIAEDVDPPEIVAHL PLLCEEKNVPYIYVKSKNDLGRAVGIEV PCASAAIINEGELRKELGSLVEKIKGLQ KGSGGSGYPYDVPDYAYPYDVPDYAYPY DVPDYA | CCCGGGTTCATTAGATCCTAAGGTG CTCTGACCGAAAGGCGTGATGAGCT GCAGGTCGACTCTAGAGCTCTGACC GAAAGGCGTGATGAGCTTGGCACTG GCGTCGTGCTCTGACCGAAAGGCGT GATGAGCTGCGGTACCTTTAAGACC AATGACTTACA |
| **VSVG-MS2** | MKCLLYLAFLFIGVNCKFTIVFPHNQKG NWKNVPSNYHYCPSSSDLNWHNDLIGTA LQVKMPKSHKAIQADGWMCHASKWVTTC | AAGGTAAACATGAGGATCACCCATG TCTGCAGGTCGACTCTAGAAAACAT GAGGATCACCCATGTCTTGGCACTG |
| | DFRWYGPKYITHSIRSFTPSVEQCKESI EQTKQGTWLNPGFPPQSCGYATVTDAEA VIVQVTPHHVLVDEYTGEWVDSQFINGK CSNYICPTVHNSTTWHSDYKVKGLCDSN LISMDITFFSEDGELSSLGKEGTGFRSN YFAYETGGKACKMQYCKHWGVRLPSGVW FEMADKDLFAAARFPECPEGSSISAPSQ TSVDVSLIQDVERILDYSLCQETWSKIR AGLPISPVDLSYLAPKNPGTGPAFTIIN GTLKYFETRYIRVDIAAPILSRMVGMIS GTTTERELWDDWAPYEDVEIGPNGVLRT SSGYKFPLYMIGHGMLDSDLHLSSKAQV FEHPHIQDAASQLPDDESLFFGDTGLSK NPIELVEGWFSSWKSSIASFFFIIGLII GLFLVLRVGIHLCIKLKHTKKRQIYTDI EMNRLGKAIAALTATRSSGSGSMASNFT QFVLVDNGGTGDVTVAPSNFANGIAEWI SSNSRSQAYKVTCSVRQSSAQNRKYTIK VEVPKGAWRSYLNMELTIPIFATNSDCE LIVKAMQGLLKDGNPIPSAIAANSGIYA MASNFTQFVLVDNGGTGDVTVAPSNFAN GIAEWISSNSRSQAYKVTCSVRQSSAQN RKYTIKVEVPKGAWRSYLNMELTIPIFA TNSDCELIVKAMQGLLKDGNPIPSAIAA NSGIYGSGYPYDVPDYA | GCGTCGTAAACATGAGGATCACCCA TGTCTGCGGTACCTTTAAGA |

**Figure 35A****.** Quantitative read-out of Nanoluc^{™} activity in recipient cells educated with EVs loaded with different PEV constructs. Nanoluc^{™} activity was then quantified to demonstrate that plasmids containing LDLRT(LDLR-targeting)-VSVG transmembrane domain (VSVGTM)- fused to an RNA binding motif can package mRNA coding for blue fluorescent protein (BFP) fused to Nanoluc^{™} (Nluc) and deliver it to recipient cells positive for the LDLR on the cell surface. In brief, a Transwell^{™} system was used in these experiments, in which HEK293 T cells seeded in the Transwell^{™} inserts (depicted as donor cells in the Figure) were co-transfected with plasmids expressing LDLR-VSVGTM-dCas13a + gRNA motif-BFP_NLuc or LDLR-VSVGTM-dCas13a + BFP _NLuc-3X CBD or LDLR-VSVGTM-dCas13d + gRNA motif-BFP_NLuc or LDLR-VSVGTM-dCas13d + BFP_NLuc- 3X CBD or LDLR-VSVGTM-Pum+ BFP_NLuc- 3XPBD or LDLR-VSVGTM-Stuf+ BFP_NLuc- 3XSBD or LDLR-VSVGTM-SD-VEEV+ BFP_NLuc- 3XPS or LDLR-VSVGTM-L72AE+ BFP_NLuc- 3X C/D Box or LDLR-VSVGTM-MS2+ BFP_NLuc- 3X HAM. After transfection, Transwell^{™}s were incubated with recipient cells (plated in the bottom of the Transwell^{™} system) for 24 hours and then cells in the bottom compartment of the Transwell^{™} system (recipient cells) were collected and Nanoluc^{™} activity was measured in the recipient cells as per the manufacturer's suggested protocol (Promega).

**Figure 35B****.** Representative fluorescence microscopy images of the same experiment depicted in Figure 35A. After transfections as indicated in Figure 35A, Transwell^{™}s were incubated with recipient cells (plated in the bottom of the Transwell^{™} system) for 24 hours and then cells in the bottom and upper compartment of the Transwell^{™} system were imaged using an EVOS fluorescent microscope to detect expression of BFP (shown in figure as light grey signal instead of the actual Blue fluorescence) in the transfected cells (donor) but also in the recipient cells. Of note, Figure 35B depicts bright-field images of the recipient cells to demonstrate that there were cells in the mock control condition but there was not expression of BFP expression.

### Example 13

### Mono-targeting with cytotoxic payload

As previously described in Example 7.

Application: Can be for any application where it might be suitable to target cytotoxic molecules [e.g., Granzyme B (GZMB)] to the target cell, in this example the targeted cells are cancer-associated fibroblasts or activated fibroblasts, cancer cells and pancreatic cancer patient tumour samples that express the fibroblast activating protein (FAP).

### Results

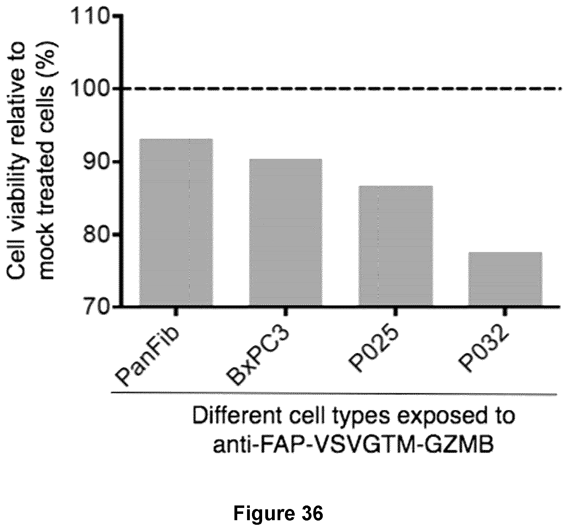

**Figure 36****.** Cellular viability of fibroblast-activating protein (FAP) positive pancreatic fibroblasts (PanFib), pancreatic cancer cells (BxPC3), and pancreatic cancer patient-derided samples (P025, P032) treated with anti-FAP-VSVG-mGZMB loaded EVs is shown in this figure. In brief, HEK293T cells were transfected with a plasmid expressing anti-FAP-VSVG-mGZMB or left untrasfected as a mock negative control. After 24 h, supernatant from transfected cells were collected and cell debris was pre-cleared by centrifugation at 1000xg for 10 minutes. Then, supernatants were transferred to various cell types known to express FAP and cell viability was measured using Alamar Blue as per the manufacturer's instructions. Viability of cells exposed to supernatant derived from donor cells transfected with anti-FAP-VSVG-mGZMB was calculated as change in viability (%) compared to cells exposed to negative control supernatant.

### Example 14

### Mono-targeting- Immunologic adjuvant payloads

### As previously described in Example 3.

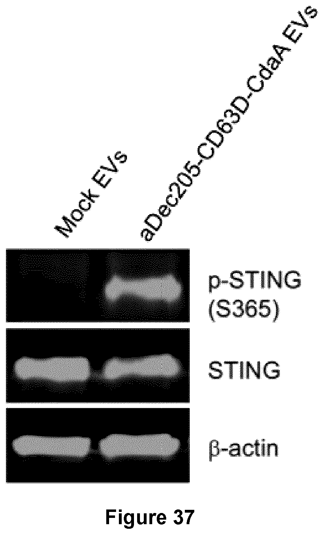

**Figure 37** depicts that EVs loaded with an anti-DEC205 targeting moiety and a CdaA payloads [inserted in the first loop of CD63], the expression of which is shown in Figure 8, are efficient at activating the STING pathway in primary isolated dendritic cells. HEK293 T cells were transfected with the PEV construct aDEC205-CD63D-CdaA-Flag in a pcDNA3.1 vector (labelled in the Figure as CD63 EVs) or left untransfected, then 48 h later EVs were purified by serial ultracentrifugation and saved at -80C until further use. Bone marrow progenitor cells from the femurs and tibiae of C57BL/6 mice were cultured in RPMI with murine GM-CSF (PeproTech #315-03, at a final concentration of 40 ng/ml) for 6 days. On day 7, the differentiated DCs were plated and treated with either untransfected exosomes or CD63 EVs as indicated for 24 h. Thereafter, STING phosphorylation (S365) in the treated DCs was evaluated by Western blot with a STING phosphorylation status-specific antibody (Cell Signaling Technology #72971). The total STING protein levels were assessed by STING antibody (Cell Signaling Technology #13647). β-actin was used as a loading control.

### Example 15

### Mono-targeting - Immune Reprogramming Payloads

### As previously described in Examples 3, 4, and Example 6a.

Application: Can be for any application where it might be suitable to target "immune reprogramming" molecules to the target immune cell. In this example the targeted cells are primary macrophages. In this particular example, macrophages treated with EVs loaded with PEV constructs that targeted these EVs specifically to macrophages (via the anti-Marco targeting moiety) and simultaneously deliver the STING pathway activator bacterial enzyme, CdaA. Of note, macrophages, especially tumour-associated macrophages or TAMs which are characterized by expressing high levels of MARCO on their surface, are known in the literature to be reprogrammed (or polarized) into a pro-inflammatory phenotype upon STING activation.

### Results

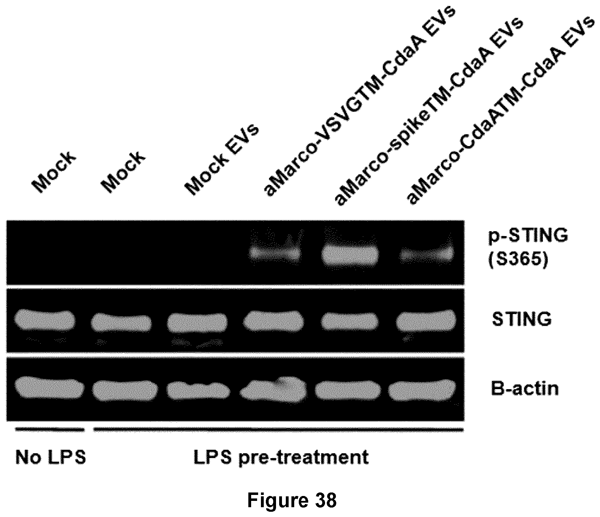

**Figure 38****.** Depicted in this figure are western blots showing activation of STING in activated primary macrophages treated with EVs loaded with three different PEV constructs as explained below. In brief, anti-MARCO-VSVGTM-CdaA or anti-MARCO-SARS2TM-CdaA or anti-MARCO-CdaATM-CdaA vectors were expressed in HEK293 T cells. After 48 hours, supernatants were collected and EVs isolated by serial ultracentrifugation. Pelleted EVs were resuspended in PBS and saved at -80C until further use. Bone marrow progenitor cells from the femurs and tibiae of C57BL/6 mice were cultured in DMEM with murine M-CSF (PeproTech #315-02-final concentration of 25 ng/ml) for 6 days. On day 7, the differentiated macrophages were plated and left untreated or pre-treated with lipopolysaccharides (LPS) at 5 EU units/ml for 24 h to induce MARCO expression on the surface of these cells. Thereafter, the pre-treated macrophages were either left mock treated or treated with various EV preparations for 24 h. Subsequently, STING phosphorylation (S365) in the treated macrophages was evaluated by Western blot with a STING phosphorylation status-specific antibody (Cell Signaling Technology #72971). As a control, the total STING protein levels were assessed by STING antibody (Cell Signaling Technology #13647). β-actin was used as a loading control.

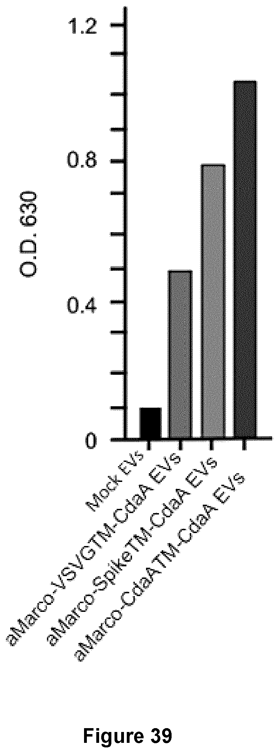

**Figure 39****.** Depicted is an experiment demonstrating the potency of various anti-Marco linked CdaA PEV construct (i.e. anti-MARCO-VSVGTM-CdaA or anti-MARCO-SARS2TM-CdaA or anti-MARCO-CdaATM-CdaA vectors) in stimulating the Interferon (IFN) signaling pathway. The functional potency of the bacterial cyclase CdaA in various MARCO-CdaA fusion proteins was assessed for generating c-di-AMP-STING signaling responses. To this end, HEK293T cells were either mock or transfected for 24 hours with various MARCO plasmids as indicated. Subsequently, c-di-AMP-containing lysates were prepared and applied to THP1-Blue-ISG IRF (IFN regulatory factor) reporter cells (InvivoGen) to stimulate STING-IRF signaling axis for 24 hours to allow for Quanti-Blue assay as per Manufacture's protocol.

### Example 16

### Multi-targeting two-part constructs (EV-BiTEs)

As previously described in Example 2.

Background: The Major Histocompatibility Complex (MHC) is required for T cells to recognize and kill tumor cells. However, most tumors downregulate the expression of the (MHC) to escape immune attack. One existing strategy in the art to circumvent the tumor's escape mechanism is by way of engineered bi-specific antibodies which draw T-cells and tumor cells to close proximity. These bi-specific antibodies are also referred to as Bi-specific T cell Engagers or BiTEs.

BiTEs are able to mediate the T cell's capacity to recognize and kill tumor cells in an MHC independent fashion. BiTEs consist of linked variable chain antibody fragments directed against the T cell antigen CD3 and a specific tumor-associated antigen (TAA). Similarly, Bi-specific NK cell engagers or BiKEs can mediate simultaneous binding to an activating receptor on NK cells and a surface tumor antigen to thus promote NK cell-dependent killing of tumor cells. Although existing BiKE and BiTE technologies are promising, many that are currently in clinical development have issues with associated toxicity during systemic administration, drug stability issues (short half-life), and challenges to reaching high enough local concentrations to be effective in most solid cancers

Application: PEV constructs with two targeting moieties: one that recognizes T cell targets, and the other targeting tumor cells (cancer cell or CAFs).

How these platforms work: These PEVs promote the synapsis between T cells and tumor cells, thus promoting the directed killing of tumor cells by these immune cell types.

Advantages: Displaying BiTEs and BiKEs in a PEV format is more stable than the bi-specific antibody constructs.

Special Features: Generally, payload-less - the PEV construct itself is a stable bi-specific cell engager bringing T or NK cells closer to cancer cells. These PEVs can be produced *in vivo* or *ex vivo.*

Delivery modalities: Using tumor-selective viruses as delivery vehicles in patients to secrete BiTEs and BiKEs in the infected cancer cell. As such, the PEV is delivered to the exact site where needed, and therefore likely to be effective at picomolar concentrations. i.e., lower dose treatment than the current bi-specific antibody approaches. Viral-based platforms such as: Vaccinia virus, lentivirus, adeno-associated virus [AAV], VSV, HSV-1, etc. could be used.

Plasmids (e.g. pcDNA 3.1) for preparing the virus and infecting cells, as well as for manufacturing isolated PEVs are also contemplated.

Targeting moieties: Single chain variable fragments or nanobodies as described above. These bind to: tumor cells through surface tumor antigen targets (e.g. anti-CEA, anti-CA9, anti-FAP, etc.) and T cells through molecules that bind to T cells (e.g. CD3 target, via an anti-CD3 scFV targeting moieties.

Payloads: None

Transmembrane Domain: All the examples listed in Table 1 could be used. Examples included here are with tetraspanin proteins, however single pass TM proteins may be used "multimerization technology" (see special features for details).

### Results

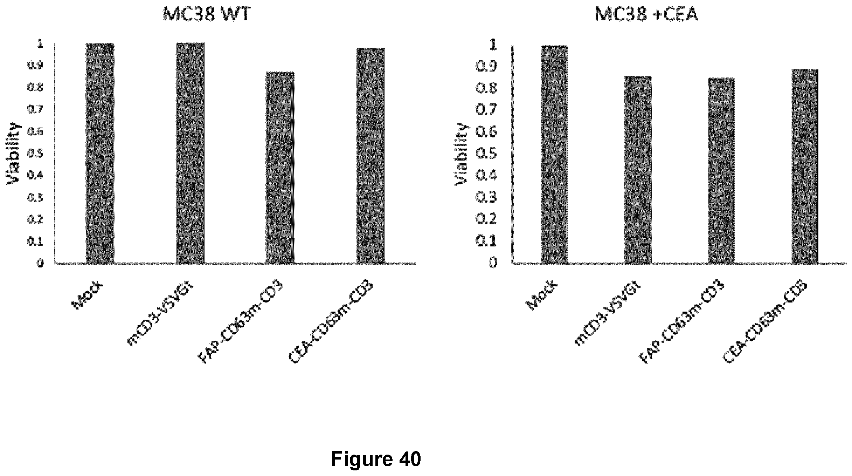

**Figure 40** shows cell viability in MC38 (WT and CEA-expressing) cells co-cultured with murine splenocytes (10:1 ratio splenocyte:MC38) in the presence of naïve EVs or EVs decorated with αCD3-VSVG, αCD3-αFAP-CD63, or αCD3-αCEA-CD63. Exo-BiTE constructs on EVs increase cell killing by splenocytes compared to naïve EVs. In brief, vectors encoding αCD3-VSVG, αCD3-αFAP-CD63, or αCD3-αCEA-CD63 were expressed in HEK293 T cells. After 48 h, supernatants were collected and EVs isolated by serial ultracentrifugation. Pelleted EVs were resuspended in PBS and saved at -80C until further use. Splenocytes were collected from homogenized mouse spleens and used fresh.

### Example 17

### Mono-targeting - Cancer Vaccine Payloads

As previously described in Examples 3 and 4.

Background/Context: Tumor-associated antigens and/or immune reprograming moieties (e.g. STING or ERAdP pathway activators) can be specifically delivered to surface molecules on APCs, such as dendritic cells via PEVs. This construct would express a targeting moiety to target PEVs to DCs (dendritic cells) and it could concomitantly carry one or multiple payloads.

Application: These platforms will represent effective means to elicit robust tumor antigen-specific immunity.

How these platforms work: DCs exhibit a largely immature or tolerizing phenotype. Tumor antigen delivery via PEVs (as payloads or cargo), in conjunction with co-administration of an adjuvant (DC maturation stimuli such as agonistic anti-CD40 mAbs, poly(I:C), cytosine-phosphate-guanine (CpG), lipo-polysaccharide (LPS), or toll-like receptor 7/8 (TLR7/8) agonists) or targeted co-delivery of PEVs containing STING or ERAdP pathway activators as described above (e.g. Bacterial dinucleotide cyclases such as CdaA and MtbDisa which are c-di-AMP cyclases, and VCA0848, which is a c-di-GMP cyclase) results in enhanced tumor-associated antigen presentation capacity and increased expression of T cell costimulatory molecules

Tumor-associated antigens alone or in combination with adjuvants or in combination with immune reprograming moieties (e.g. STING or ERAdP pathway activators) can be specifically delivered to surface molecules on dendritic cells via PEVs.

Targeting moieties: Targets: Antigen presenting cell-surface molecules, including CD40, a TNF-α family receptor, DEC205, a C-type lectin receptor (CLEC9) and CD11c, an integrin receptor, are targeted by targeting moieties including specific monoclonal antibodies, scFvs, single domain antibodies, nanobodies (i.e. anti-DEC205, anti-Clec9A, anti-CD11c), ligands or targeted peptides (e.g. CD40 ligand or CD40-targeted peptide).

Payloads: Specific tumor-associated antigens (For proof-of-concept in mouse tumor models: DCT and OVA are being explored). Human tumor-associated antigens relevant for clinical testing can be used (e.g. HPV-E6 and E7, NY-ESO-1, etc.). Cancer-specific neoantigens can also be used.

Concomitant expression of specific disease cell antigens is contemplated, such as tumor-associated/specific antigens (e.g. OVA, DCT, mERKm9 etc.). Also, adjuvant molecules such as a STING or ERAdP activator could be concomitantly delivered with disease-specific antigens or tumor-associated/specific antigens

Transmembrane domain: All the examples listed in Table 1 could be used.

Delivery/manufacturing modalities: Viral-based platforms such as, Vaccinia virus, lentivirus, adeno-associated virus [AAV], VSV, HSV-1 etc. could be used. Plasmids (e.g. pcDNA 3.1) for transfecting cells, as well as manufactured isolated PEVs are also contemplated.

### Results

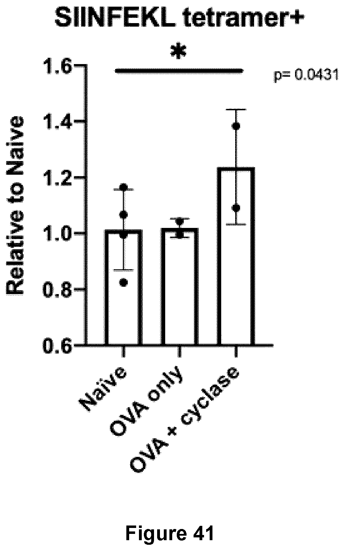

**Figure 41****.** Vaccination experiment with naïve EVs, or EVs decorated with aDEC205-VSVGTM-OVA (named in the figure as "OVA") or both aDEC205-CD63D-CdaA-Flag and aDEC205-VSVGTM-OVA (refereed in the Figure as OVA+cyclase) (also note that these constructs previously shown in Figures 8, 14, and 38) showed that a combination of both dendritic cell-targeted antigen [e.g. Ovalbumin (OVA)] and immune adjuvant (e.g. CdaA enzyme) induces immune responses in vivo. In brief, vectors encoding aDEC205-CD63D-CdaA-Flag and/or anti-DEC205-VSVGTM-OVA were expressed in HEK293 T cells. Empty vector transfection was included as a negative mock EV control. After 48 h, supernatants were collected and EVs isolated by serial ultracentrifugation, resuspended in 750ul of PBS and saved at -80C until further use. Mice were then vaccinated with 200ul of each EV preparation per mouse IV (Naïve n=5, OVA only n=3 and OVA+cyclase n=3). Fourteen days later splenocytes were harvested and stained with a SIINFEKL tetramer. Data are displayed as tetramer+ CD8+ T cells by percentage of total CD8+ T cells relative to background staining seen in naïve mice.

### Example 18

### Mono-targeting- Immunologic adjuvant payloads

### As previously described in Example 3.

The "response receiver-modulated diguanylate cyclase" of Geobacter sulfurreducens [GsPCA] produces cyclic AMP-GMP (3',3'-cGAMP) in this common soil bacteria. The REC (signal receiving/dimerizing) regulatory domain was deleted and the diguanylate-cyclase (DGC) or GGDEF domain were expressed yielding a unique, constitutively active form. When this active form was expressed in a PEV construct targeted to dendritic cells by the anti-DEC205 scFV and loaded into EVs by the VSVG transmembrane domain, functional activation of the interferon response was observed.

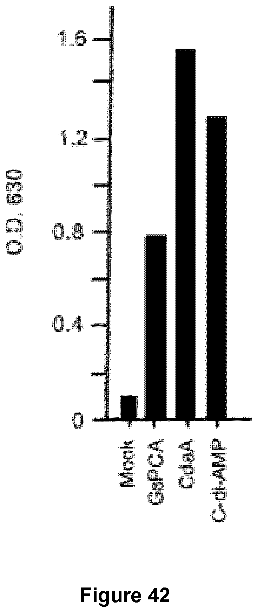

**Figure 42****.** This figure shows Dendritic cell-directed PEV constructs that can act as immune adjuvants by stimulating the interferon response in a reported cell line. Functional characterization of wild-type and various mutated c-di-GMP and c-di-AMP bacteria cyclase enzymes in the mammalian cell THP1-Blue-ISG system using a Quanti-Blue colorimetry assay. Various codon optimized transgene constructs as detailed below were transfected in HEK 293 T cells for 48 hours. Cell lysates were collected and transferred to IFN-β promoter-SEAP reporter cells [THP1-Blue-ISG IRF (IFN regulatory factor) reporter cells (InvivoGen)] to stimulate STING-IRF signaling axis for 24 hours to allow for Quanti-Blue assay as per the Manufacturer's protocol. In Figure 42, GsPCA: anti-DEC205-VSVGTM-GsPCA-FLAG-pcDNA3.1(+) plasmid. Positive controls include (1) CdaA: CD63_anti-DEC205_CD63_CdaA_FLAG- pcDNA3.1(+) plasmid and (2) c-di-AMP: 10 µg/ml. Negative controls include mock transfected cells.

Where features are named herein, it will be understood that corresponding example sequences for the features (or sequences that comprise) may found in **Table 13.**

**Table 13: Master Table of Sequences**

| **EXAMPLE EV-DIRECTED TRANSMEMBRANE DOMAINS & GLYCOPROTEINS** | | |
|---|---|---|
| **Name** | **Sequence or GenBank Accession** | **SEQ ID NO.** |
| **Murine CD63** | NP_001269895.1 | n/a |
| **Human CD63** | NP_001244319.1 | n/a |
| **CD9** | NP_001760.1 | n/a |
| **LAMP2B** | AAB67314.1 | n/a |
| **LAMP2B TM Domain** | LVPIAVGAALAGVLILVLLAYFIG | 1 |
| **VSV-G** | NP_041715.1 | n/a |
| **VSV-G TM Domain** | FFFIIGLIIGLFLVLRVGIHL | 2 |
| **VSV-G TM Domain-containing Truncation** | GDTGLSKNPIELVEGWFSSWKSSIASFFFIIGLII GLFLVLRVGIHLCIKLKHTKKRQIYTDIEMNRLGK | 3 |
| **CD81** | NP_004347.1 | n/a |
| **CD82** | NP_002222.1 | n/a |
| **LAMP1** | NP_005552.3 | n/a |
| **LAMP1 TM Domain** | LIPIAVGGALAGLVLIVLIAYLV | 4 |
| **Junin virus glycoprotein** | NP_899218.1 | n/a |
| **Junin virus glycoprotein TM Domain** | ICFWSTVFFTASLFLHLVGIP | 5 |
| **Lassa fever virus glycoprotein** | AIT17400.1 | n/a |
| **Lassa fever virus glycoprotein TM Domain** | LFVFSTSFYLISIFLHLVKIP | 6 |
| **LCMV glycoprotein** | AAX49341.1 | n/a |
| **LCMV glycoprotein TM Domain** | LLMFSTSAYLVSIFLHLVKIP | 7 |
| **SARS-CoV-2 glycoprotein** | | 8 |
| | | |
| **SARS-CoV-2 glycoprotein TM Domain** | WYIWLGFIAGLIAIVMVTIML | 9 |
| **SARS-CoV-2 glycoprotein TM Domain + Intravesicular Tail** | WPWYIWLGFIAGLIAIVMVTIMLCCMTSCCSCLKG CCSCGSCCKFDEDDSEPVLKGVKLHYTYPYDVPDY A | 10 |
| **Tamiami virus glycoprotein** | AAN32955.1 | n/a |
| **Tamiami virus glycoprotein TM Domain** | VCFWSTLFYTASIFLHLIRIP | 11 |
| **Guanarito virus glycoprotein** | NP_899210.1 | n/a |
| **Guanarito virus glycoprotein TM Domain** | KTPLTLVDLCFWSAIFFTTSLFLHLVGFPTH | 12 |
| **Machupo virus glycoprotein** | NP_899212.1 | n/a |
| **Machupo virus glycoprotein TM Domain** | ICFWSTIFFTASLFLHLVGIP | 13 |
| **Sabia virus glycoprotein** | YP_089665 | n/a |
| **Sabia virus glycoprotein TM Domain** | ICFWSTLFFTTTLFLHLVGFP | 14 |
| **Parana virus glycoprotein** | AAN32957.1 | n/a |
| **Parana virus glycoprotein TM Domain** | ICFWSLVYFTVSVFLQLVGIP | 15 |
| **CdAa TM Domain** | MDFSNMSILHYLANIVDILVVWFVIYKVIMLIRGT KAVQLLKGI | 16 |

| **EXAMPLE TARGETING MOIETIES** | | |
|---|---|---|
| **Name** | **Sequence or GenBank Accession** | **SEQ ID NO.** |
| **Anti-mDEC205 scFv** | EVKLQQSGTEVVKPGASVKLSCCKASGYIFTSYDI DWVRQTPEQGLEWIGWIFPGEGSTEYNEKFKGRAT LSVDKSSSTAYMELTRLTSEDSAVYFCARGDYYRR YFDLWGQGTTVTVSSGGGGSGGGGSGGGGSGGGGS DIQMTQSPSFLSTSLGNSITITCHASQNIKGWLAW YQQKSGNAPQLLIYKASSLQSGVPSRFSGSGSGTD YIFTISNLQPEDIATYYCQHYQSFPWTFGGGTKLE IKRAA | 17 |
| **Anti-mCLEC9A scFv** | DIVMTQTPSSQAVSAGEKVTMNCKSSQSVLYDENK KNYLAWYQQKSGQSPKLLIYWASTGESGVPDRFIG SGSGTDFTLTISSVQAEDLAVYYCQQYYDFPPTFG GGTKGGSSRSSSSGGGGSGGGGQIVESGGGLVQPK ESLKISCTASGFTFSNAAIYWVRQTPGKGLEWVGR IRTRPSKYATDYADSVRGRFTISRDDSKSMVYLQM DNLRTEDTAMYYCTPRATEDVPFYWGQGVMVTVSS | 18 |
| **Anti-CEACAM5 scFv** | QVKLQQSGAELVRSGTSVKLSCTASGFNIKDSYMH WLRQGPEQCLEWIGWIDPENGDTEYAPKFQGKATF TTDTSSNTAYLQLSSLTSEDTAVYYCNEGTPTGPY YFDYWGQGTTVTVSSGGGGSGGGGSGGGGSENVLT QSPAIMSASPGEKVTITCSASSSVSYMHWFQQKPG TSPKLWIYSTSNLASGVPARFSGSGSGTSYSLTIS RMEAEDAATYYCQQRSSYPLTFGCGTKLELKR | 19 |
| **Anti-CTLA4 scFv (9D9 Clone)** | METDTLLLWVLLLWVPGSTGIRRADIVMTQTTLSL PVSLGDQASISCRSSQSIVHSNGNTYLEWYLQKPG | 20 |
| | QSPKLLIYKVSNRFSGVPDRFSGSGSGTDFTLKIS RVEAEDLGVYYCFQGSHVPYTFGGGTKLEIKRGGG GSGGGGSGGGGSEAKLQESGPVLVKPGASVKMSCK ASGYTFTDYYMNWVKQSHGKSLEWIGVINPYNGDT SYNQKFKGKATLTVDKSSSTAYMELNSLTSEDSAV YYCARYYGSWFAYWGQGTLITVSTAKTTPPSVYPL APRS | |
| **Anti-CD19 scFv** | DIQMTQTTSSLSASLGDRVTISCRASQDISKYLNW YQQKPDGTVKLLIYHTSRLHSGVPSRFSGSGSGTD YSLTISNLEQEDIATYFCQQGNTLPYTFGGGTKLE ITGSTSGSGKPGSGEGSTKGEVKLQESGPGLVAPS QSLSVTCTVSGVSLPDYGVSWIRQPPRKGLEWLGV IWGSETTYYNSALKSRLTIIKDNSKSQVFLKMNSL QTDDTAIYYCAKHYYYGGSYAMDYWGQGTSVTVSS T | 21 |
| **Anti-CD20 scFv** | DIVMTQTPLSLPVTPGEPASISCRSSKSLLHSNGI TYLYWYLQKPGQSPQLLIYQMSNLVSGVPDRFSGS GSGTDFTLKISRVEAEDVGVYYCAQNLELPYTFGG GTKVEIKRGGGGSGGGGSGGGGSQVQLVQSGAEVK KPGSSVKVSCKASGYAFSYSWINWVRQAPGQGLEW MGRIFPGDGDTDYNGKFKGRVTITADKSTSTAYME LSSLRSEDTAVYYCARNVFDGYWLVYWGQGTLVTV SSA | 22 |
| **Anti-FAP scFv** | QVQLQESDPGLVKPSETLSLTCTVSGGSISSNNYY WGWIRQTPGKGLEWIGSIYYSGSTNYNPSLKSRVT ISVDTSKNQFSLKLSSVTAADTAVYYCARGARWQA RPATRIDGVAFDIWGQGTMVTVSSGGSSRSSSSGG GGSGGGGETTLTQSPGTLSLSPGERATLSCRASQS VTRNYLAWYQQKPGQAPRLLMYGASNRAAGVPDRF SGSGSGTDFTLTISRLEPEDFAVYYCQQFGSPYTF GQGTKVEIK | 23 |
| **Anti-CTLA4 scFv** | IRRADIVLTQSPGTLSLSPGERATLSCRASQSVGS SYLAWYQQKPGQAPRLLIYGAFSRATGIPDRFSGS GSGTDFTLTISRLEPEDFAVYYCQQYGSSPWTFGQ GTKVEIKR | 24 |
| **Chlorotoxin** | MCMPCFTTDHQMARKCDDCCGGKGRGKCYGPQCLC R | 25 |
| **PD1 ectodomain** | MWVRQVPWSFTWAVLQLSWQSGWLLEVPNGPWRSL TFYPAWLTVSEGANATFTCSLSNWSEDLMLNWNRL SPSNQTEKQAAFCNGLSQPVQDARFQIIQLPNRHD FHMNILDTRRNDSGIYLCGAISLHPKAKIEESPGA ELVVTERILETSTRYPSPSPKPEGRFQGM | 26 |
| **VAR2 domain of Plasmodium falciparum protein, VAR2CSA,** | RGSDLKSSNSRVTTPKVDVGNYKCLEYNDNKYGRK MTRASGCSWQNMYEKRDDCTEENTPIKCQCAYKYG HPTNGLPSPVNVVVATNCQQLKSKDTEKNCKETTT YTTYTTWPAKDAGCINDDLVISLYSSPTTLGIDIL HKFFSDIDSQVCKNEAANTTSSPGCNKTGAKRNRK ADEIYKSYRKYIQDWRKVWSSGATGDGGKCDEIFK KYVECKNKCETKCEGNCTGGSEKCSKCNEIVPKVK EQRQKCFHEVWEQLFRLYQPILDITPIDDCSSGSG TVSGDGCCTTSNMGAGHKMALWIYKKNTNWWSERL EDLSSYSTDQEATNNKKIYKRFLKGFIKQLNLELD KTYENDWISTGKILDGYDAFSYELAKCLKKGNDDN KKEHSPKLNKGEHFAAIIWEKLLESNTRINKLEQT KKGKEDLCVVLCLSQTRPPLGITNAYEKQLGGEKG SSKKWIWNKNNKKSQESKCKDCKYCNTLSALVKEL NKECAEQNKNNCSGNSSSGSKNDSCNEQLIRLFDQ CCCNEVGSLSTHEIVCVRLDKDNERVGLKVHKCVK KKNAKISSHTICTKNSSPDSIQEQEVSAIGSENCN CVEDNNKQIKESPNNADSSNLIFSLKTAYEAFYPD GKIYN | 27 |
| **Anti-CD3** | QVQLQQSGAELARPGASVKMSCKASGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSGGGGSGGGGSGGGGSQIVLTQ SPAIMSASPGEKVTMTCSASSSVSYMNWYQQKSGT SPKRWIYDTSKLASGVPAHFRGSGSGTSYSLTISG MEAEDAATYYCQQWNSNPFTFGSGTKLEIN | 28 |
| **Anti-MARCO** | IRSLSSLQSLSTKDLTMGWICIIFLVATATGVLPQ VKLLQSGAALVKPGASVKMSCKASGYTFTDYWVSW VKQSHGKSLECIGEISPNSGTTNFNEKFKGKATLT | 29 |
| | VDKSTSTAYMELSRLTSEDSAIYYCTRCRYTTGVH YFDYWGQGVMVTVSSAETTAPSVYPLAPGTALKSN SMVTLGCLVKGYFPEPVTVTWNSGALSSGVHTFPA VLQSGLYTLTSSVTVPSSTWPSQTVTCNVAHPASS TKVDKKIEKGEFATYMAAGGGGSGGGGSGGGGSGG GGVIPLLISSPQNDESCQSLFLLLLWILGTKGDVV LTQTPSILSVTIGQSVSISCRSSQSLLDSDGNSYL YWFLQRPGQSPQRLIYLVSNLGSGVPNRFSGSGSG TDFTLKISGVEAEDLGVYYCMQATHAPWTFGGGTK LELKRADAAPTVSIFPPSTEQLATGGASVVCLMNN FYPRDISVKWKIDGTERRDGVLDSVTDQDSKDSTY SMSSTLSLTKADYESHNLYTCEVVHKHHPHPGRIP ATGAY | |
| **Human Anti-DEC205** | QAVVTQESALTTSPGETVTLTCRSSTGAVTISNYA NWVQEKPDHLFTGLIGGTNNRAPGVPARFSGSLIG DKAALTITGAQTEDEAIYFCALWYNNQFIFGSGTK VTVLGGGGGSGGGGSGGGGSGGGGSEVQLQQSGPV LVKPGASVKMSCKASGNTFTDSFMHWMKQSHGKSL EWIGIINPYNGGTSYNQKFKGKATLTVDKSSSTAY MELNSLTSEDSAVYYCARNGVRYYFDYWGQGTTLT VSSASGAGGGGSAAA | 30 |
| **Human/mouse anti-langerin (aka anti-CD207)** | AELVRPGASVTLSCKASGYTFIDHDMHWVQQTPVY GLEWIGAIDPETGDTGYNQKFKGKAILTADKSSRT AYMELRSLTSEDSAVYYCTIPFYYSNYSPFAYWGQ GTLVTVSGGGGSGGGGSGGGGSIVLSQSPAILSAS PGEKVTMTCRASSSVSYMHWYQRKPGSSPKPWIYA TSNLASGVPARFSGSGSGTSYSLTISRVEAEDAAT YYCQQWSSNPLTFGAGTKLEL | 31 |
| **N-Terminal V-set Ig domain of SIRPa (residues 1-118)** | EEEVQVIQPDKSVSVAAGESAILHCTITSLIPVGP IQWFRGAGPARELIYNQREGHFPRVTTVSETTRRE NMDFSISISNITPADAGTYYCVKFRKGSPDTEVKS GAGTELSVRAKPS | 32 |
| **GE11 Peptide** | YHWYGYTPQNVI | 33 |
| **iRGD Peptide** | MIETYNQTSPRSAATGLPISMKIFMYLLTVFLITQ MIGSALFAVYLHRRLDKIEDERNLH | 34 |
| **CD40 Ligand** | | 35 |
| **CD40 Binding Peptide** | VLQWAEKGYYTMSNN | 36 |

| **EXAMPLE EV PAYLOADS** | | |
|---|---|---|
| **Name** | **Sequence or GenBank Accession + Coordinates** | **SEQ ID NO.** |
| **Nanoluc^{™}** | VFTLEDFVGDWRQTAGYNLDQVLEQGGVSSLFQNL GVSVTPIQRIVLSGENGLKIDIHVIIPYEGLSGDQ MGQIEKIFKVVYPVDDHHFKVILHYGTLVIDGVTP NMIDYFGRPYEGIAVFDGKKITVTGTLWNGNKIID ERLINPDGSLLFRVTINGVTGWRLCERILA | 37 |
| **mCherry** | LVQLVHAAGGVAALGAFVLFHDGVVLVVGGDVQLD VDVVGAGQLHGLLGLVGGLDLSVVVAAVLQLQPLL DLALQGAVLGVHPLGGGLPAHGLLLHYGAVGGEVG AAQLHLVDELAVLQGGVLGHGHHAAVLEVHHALPL EALGEGQLQVVGDVGGVLHVGLGAVHELRGQDVPG EGQGATLGHLQLGGLGALVGAALALALDLELVAVH GALHVHLEAHELLDDGHVILLALAHH | 38 |
| **PE38** | SUC59349.1 POS: 17-235 I30V | n/a |
| **Diphtheria Toxin** | WP_181997938.1 POS26-414 | n/a |
| **Human GZMB R201K** | NP_001332940.1 POS: 7-235 R201K | n/a |
| **Human TRAIL** | AMMEVQGGPSLGQTCVLIVIFTVLLQSLCVAVTYV YFTNELKQMQDKYSKSGIACFLKEDDSYWDPNDEE SMNSPCWQVKWQLRQLVRKMILRTSEETISTVQEK QQNISPLVRERGPQRVAAHITGTRGRSNTLSSPNS KNEKALGRKINSWESSRSGHSFLSNLHLRNGELVI HEKGFYYIYSQTYFRFQEEIKENTKNDKQMVQYIY KYTSYPDPILLMKSARNSCWSKDAEYGLYSIYQGG IFELKENDRIFVSVTNEHLIDMDHEASFFGAFLVG | 39 |
| **Ovalbumin** | MGSIGAASMEFCFDVFKELKVHHANENIFYCPIAI MSALAMVYLGAKDSTRTQINKVVRFDKLPGFGDSI EAQCGTSVNVHSSLRDILNQITKPNDVYSFSLASR LYAEERYPILPEYLQCVKELYRGGLEPINFQTAAD QARELINSWVESQTNGIIRNVLQPSSVDSQTAMVL VNAIVFKGLWEKAFKDEDTQAMPFRVTEQESKPVQ MMYQIGLFRVASMASEKMKILELPFASGTMSMLVL LPDEVSGLEQLESIINFEKLTEWTSSNVMEERKIK VYLPRMKMEEKYNLTSVLMAMGITDVFSSSANLSG ISSAESLKTSQAVHAAHAEINEAGREVVGSAEAGV DAASVSEEFRADHPFLFCIKHIATNAVLFFGRCVS P | 40 |
| **Murine GZMB** | NP_038570.1 POS: 19-247 | n/a |
| **CdaA** | MDFSNMSILHYLANIVDILVVWFVIYKVIMLIRGT KAVQLLKGIFIIIAVKLLSGFFGLQTVEWITDQML TWGFLAIIIIFQPELRRALETLGRGNIFTRYGSRI EREQHHLIESIEKSTQYMAKRRIGALISVARDTGM DDYIETGIPLNAKISSQLLINIFIPNTPLHDGAVI IKGNEIASAASYLPLSDSPFLSKELGTRHRAALGI SEVTDSITIVVSEETGGISLTKGGELFRDVSEEEL HKILLKELVTVTAKKPSIFSKWKGGKSE | 41 |
| **mtbDISA** | HAVTRPTLREAVARLAPGTGLRDGLERILRGRTGA LIVLGHDENVEAICDGGFSLDVRYAATRLRELCKM DGAVVLSTDGSRIVRANVQLVPDPSIPTDESGTRH RSAERAAIQTGYPVISVSHSMNIVTVYVRGERHVL TDSATILSRANQAIATLERYKTRLDEVSRQLSRAE IEDFVTLRDVMTVVQRLELVRRIGLVIDYDVVELG TDGRQLRLQLDELLGGNDTARELIVRDYHANPEPP STGQINATLDELDALSDGDLLDFTALAKVFGYPTT TEAQDSTLSPRGYRAMAGIPRLQFAHADLLVRAFG TLQGLLAASAGDLQSVDGIGAMWARHVREGLSQLA ESTISDQ | 42 |
| **Rotavirus VP6 Protein** | Q32WR3 | n/a |
| **dCas13a RBM** | AIAALTATRSSGSGSMKVTKVDGISHKKYIEEGKL VKSTSEENRTSERLSELLSIRLDIYIKNPDNASEE ENRIRRENLKKFFSNKVLHLKDSVLYLKNRKEKNA | 43 |
| | VQDKNYSEEDISEYDLKNKNSFSVLKKILLNEDVN SEELEIFRKDVEAKLNKINSLKYSFEENKANYQKI NENNVEKVGGKSKRNIIYDYYRESAKRNDYINNVQ EAFDKLYKKEDIEKLFFLIENSKKHEKYKIREYYH KIIGRKNDKENFAKIIYEEIQNVNNIKELIEKIPD MSELKKSQVFYKYYLDKEELNDKNIKYAFCHFVEI EMSQLLKNYVYKRLSNISNDKIKRIFEYQNLKKLI ENKLLNKLDTYVRNCGKYNYYLQVGEIATSDFIAR NRQNEAFLRNIIGVSSVAYFSLRNILETENENGIT GRMRGKTVKNNKGEEKYVSGEVDKIYNENKQNEVK ENLKMFYSYDFNMDNKNEIEDFFANIDEAISSIAH GIVHFNLELEGKDIFAFKNIAPSEISKKMFQNEIN EKKLKLKIFKQLNSANVFNYYEKDVIIKYLKNTKF NFVNKNIPFVPSFTKLYNKIEDLRNTLKFFWSVPK DKEEKDAQIYLLKNIYYGEFLNKFVKNSKVFFKIT NEVIKINKQRNQKTGHYKYQKFENIEKTVPVEYLA IIQSREMINNQDKEEKNTYIDFIQQIFLKGFIDYL NKNNLKYIESNNNNDNNDIFSKIKIKKDNKEKYDK ILKNYEKHNRNKEIPHEINEFVREIKLGKILKYTE NLNMFYLILKLLNHKELTNLKGSLEKYQSANKEET FSDELELINLLNLDNNRVTEDFELEANEIGKFLDF NENKIKDRKELKKFDTNKIYFDGENIIKHRAFYNI KKYGMLNLLEKIADKAKYKISLKELKEYSNKKNEI EKNYTMQQNLHRKYARPKKDEKFNDEDYKEYEKAI GNIQKYTHLKNKVEFNELNLLQGLLLKILHRLVGY TSIWERDLRFRLKGEFPENHYIEEIFNFDNSKNVK YKSGQIVEKYINFYKELYKDNVEKRSIYSDKKVKK LKQEKKDLYIANYIAHFNYIPHAEISLLEVLENLR KLLSYDRKLKNAIMKSIVDILKEYGFVATFKIGAD KKIEIQTLESEKIVHLKNLKKKLMTDRNSEELCE LVKVMFEYKALEAAARV | |
| **dCas13b RBM** | AIAALTATRSSGSGSMNIPALVENQKKYFGTYSVM AMLNAQTVLDHIQKVADIEGEQNENNENLWFHPVM SHLYNAKNGYDKQPEKTMFIIERLQSYFPFLKIMA ENQREYSNGKYKQNRVEVNSNDIFEVLKRAFGVLK MYRDLTNAYKTYEEKLNDGCEFLTSTEQPLSGMIN | 44 |
| | NYYTVALRNMNERYGYKTEDLAFIQDKRFKFVKDA YGKKKSQVNTGFFLSLQDYNGDTQKKLHLSGVGIA LLICLFLDKQYINIFLSRLPIFSSYNAQSEERRII IRSFGINSIKLPKDRIHSEKSNKSVAMDMLNEVKR CPDELFTTLSAEKQSRFRIISDDHNEVLMKRSSDR FVPLLLQYIDYGKLFDHIRFHVNMGKLRYLLKADK TCIDGQTRVRVIEQPLNGFGRLEEAETMRKQENGT FGNSGIRIRDFENMKRDDANPANYPYIVDTYTHYI LENNKVEMFINDKEDSAPLLPVIEDDRYVVKTIPS CRMSTLEIPAMAFHMFLFGSKKTEKLIVDVHNRYK RLFQAMQKEEVTAENIASFGIAESDLPQKILDLIS GNAHGKDVDAFIRLTVDDMLTDTERRIKRFKDDRK SIRSADNKMGKRGFKQISTGKLADFLAKDIVLFQP SVNDGENKITGLNYRIMQSAIAVYDSGDDYEAKQQ FKLMFEKARLIGKGTTEPHPFLYKVFARSIPANAV EFYERYLIERKFYLTGLSNEIKKGNRVDVPFIRRD QNKWKTPAMKTLGRIYSEDLPVELPRQMFDNEIKS HLKSLPQMEGIDFNNANVTYLIAEYMKRVLDDDFQ TFYQWNRNYRYMDMLKGEYDRKGSLQHCFTSVEER EGLWKERASRTERYRKQASNKIRSNRQMRNASSEE IETILDKRLSNSRNEYQKSEKVIRRYRVQDALLFL LAKKTLTELADFDGERFKLKEIMPDAEKGILSEIM PMSFTFEKGGKKYTITSEGMKLKNYGDFFVLASDK RIGNLLELVGSDIVSKEDIMEEFNKYDQCRPEISS IVFNLEKWAFDTYPELSARVDREEKVDFKSILKIL LNNKNINKEQSDILRKIRNAFDANNYPDKGVVEIK ALPEIAMSIKKAFGEYAIMKGSLQLPPLERLTLGS SYPYDVPDYAYPYDVPDYAYPYDVPDYA | |
| **dCas13d RBM** | AIAALTATRSSGSGSEASIEKKKSFAKGMGVKSTL VSGSKVYMTTFAEGSDARLEKIVEGDSIRSVNEGE AFSAEMADKNAGYKIGNAKFSHPKGYAVVANNPLY TGPVQQDMLGLKETLEKRYFGESADGNDNICIQVI HNILDIEKILAEYITNAAYAVNNISGLDKDIIGFG KFSTVYTYDEFKDPEHHRAAFNNNDKLINAIKAQY DEFDNFLDNPRLGYFGQAFFSKEGRNYIINYGNEC YDILALLSGLAHWVVANNEEESRISRTWLYNLDKN | 45 |
| | LDNEYISTLNYLYDRITNELTNSFSKNSAANVNYI AETLGINPAEFAEQYFRFSIMKEQKNLGFNITKLR EVMLDRKDMSEIRKNHKVFDSIRTKVYTMMDFVIY RYYIEEDAKVAAANKSLPDNEKSLSEKDIFVINLR GSFNDDQKDALYYDEANRIWRKLENIMHNIKEFRG NKTREYKKKDAPRLPRILPAGRDVSAFSKLMYALT MFLDGKEINDLLTTLINKFDNIQSFLKVMPLIGVN AKFVEEYAFFKDSAKIADELRLIKSFARMGEPIAD ARRAMYIDAIRILGTNLSYDELKALADTFSLDENG NKLKKGKHGMRNFIINNVISNKRFHYLIRYGDPAH LHEIAKNEAVVKFVLGRIADIQKKQGQNGKNQIDR YYETCIGKDKGKSVSEKVDALTKIITGMNYDQFDK KRSVIEDTGRENAEREKFKKIISLYLTVIYHILKN IVNINARYVIGFHCVERDAQLYKEKGYDINLKKLE EKGFSSVTKLCAGIDETAPDKRKDVEKEMAERAKE SIDSLESANPKLYANYIKYSDEKKAEEFTRQINRE KAKTALNAYLRNTKWNVIIREDLLRIDNKTCTLFA NKAVALEVARYVHAYINDIAEVNSYFQLYHYIMQR IIMNERYEKSSGKVSEYFDAVNDEKKYNDRLLKLL CVPFGYCIPRFKNLSIEALFDRNEAAKFDKEKKKV SGNSGSGAAARV | |
| **Pum RBM** | AIAALTATRSSGSGSMGRSRLLEDFRNNRYPNLQL REIAGHIMEFSQDQHGSRFIQLKLERATPAERQLV FNEILQAAYQLMVDVFGNYVIQKFFEFGSLEQKLA LAERIRGHVLSLALQMYGCRVIQKALEFIPSDQQN EMVRELDGHVLKCVKDQNGNHVVQKCIECVQPQSL QFIIDAFKGQVFALSTHPYGCRVIQRILEHCLPDQ TLPILEELHQHTEQLVQDQYGNYVIQHVLEHGRPE DKSKIVAEIRGNVLVLSQHKFASNVVEKCVTHASR TERAVLIDEVCTMNDGPHSALYTMMKDQYANYVVQ KMIDVAEPGQRKIVMHKIRPHIATLRKYTYGKHIL AKLEKYYMKNGVDLGGSGYPYDVPDYA | 46 |
| **Stu1 RBM** | AIAALTATRSSGSGSNLNKSEISQVFEIALKRNLP VNFEVARESGPPHMKNFVTKVSVGEFVGEGEGKSK KISKKNAAIAVLEELKKLPPLPAVERVKPRIKKKT KPIVKPQTSPEYGQGINPISRLAQIQQAKKEKEPE | 47 |
| | YTLLTERGLPRRREFVMQVKVGNHTAEGTGTNKKV AKRNAAENMLEILGFKVPQRQGSGYPYDVPDYA | |
| **VEEV RBM** | AIAALTATRSSGSGSMFPFQPMYPMQPMYRNPFA APRRPWFPRTDPFLAMQVQELTRSMANLTFKQRRD APPEGPPAKKPKREAPQKQKGGGQGKKKKNQGKKK AKTGPPNPKAQSGNKKKPNKKPGKRQRMVMKLESD KGSGGSGYPYDVPDYAYPYDVPDYAYPYDVPDYA | 48 |
| **L72AE RBM** | AIAALTATRSSGSGSMYVRFEVPEDMQNEALSLLE KVRESGKVKKGTNETTKAVERGLAKLVYIAEDVDP PEIVAHLPLLCEEKNVPYIYVKSKNDLGRAVGIEV PCASAAIINEGELRKELGSLVEKIKGLQKGSGGSG YPYDVPDYAYPYDVPDYAYPYDVPDYA | 49 |
| **MS2 RBM** | AIAALTATRSSGSGSMASNFTQFVLVDNGGTGDVT VAPSNFANGIAEWISSNSRSQAYKVTCSVRQSSAQ NRKYTIKVEVPKGAWRSYLNMELTIPIFATNSDCE LIVKAMQGLLKDGNPIPSAIAANSGIYAMASNFTQ FVLVDNGGTGDVTVAPSNFANGIAEWISSNSRSQA YKVTCSVRQSSAQNRKYTIKVEVPKGAWRSYLNME LTIPIFATNSDCELIVKAMQGLLKDGNPIPSAIAA NSGIYGSGYPYDVPDYA | 50 |

| **EXAMPLE EV CARGO** | | |
|---|---|---|
| **dCas13a RBM RNA Target Sequence** | GATTTAGACTACCCCAAAAACGAAGGGGACTAAAA CGGAATTCGAGCTCGGTACCTTCCCGGGTTCATTA GAGATTTAGACTACCCCAAAAACGAAGGGGACTAA AACGTCTGCAGGTCGACTCTAGAAAGATTTAGACT ACCCCAAAAACGAAGGGGACTAAAAC | 51 |
| **dCas13b RBM RNA Target Sequence** | GTTGTGGAAGGTCCAGTTTTGAGGGGCTATTACAA CGGAATTCGAGCTCGGTACCTTCCCGGGTTCATTA GAGTTGTGGAAGGTCCAGTTTTGAGGGGCTATTAC AACGTCTGCAGGTCGACTCTAGAAAGTTGTGGAAG GTCCAGTTTTGAGGGGCTATTACAAC | 52 |
| **dCas13d RBM RNA Target Sequence** | GAACCCCTACCAACTGGTCGGGGTTTGAAACGGAA TTCGAGCTCGGTACCTTCCCGGGTTCATTAGAGAA CCCCTACCAACTGGTCGGGGTTTGAAACGTCTGCA GGTCGACTCTAGAAAGAACCCCTACCAACTGGTCG GGGTTTGAAAC | 53 |
| **Pum RBM RNA Target Sequence** | TGGAATTCGAGCTCGGTACCTTCCCGGGTTCATTA GATCCTAAGGTTCATATAATCGTTGTCCAGAATTG TATATATTCGTGCAGGTCGACTCTAGATCATATAA TCGTTGTCCAGAATTGTATATATTCGTTGGCACTG GCGTCGTTCATATAATCGTTGTCCAGAATTGTATA TATTCG | 54 |
| **Stu1 RBM RNA Target Sequence** | CATTAGATCCTAAGGTGAGTGCCAGAAGCTGCCTC GATTCAACGAGGCAGTTTCTGGTACTCTGCAGGTC GACTCTAGAGAGTGCCAGAAGCTGCCTCGATTCAA CGAGGCAGTTTCTGGTACTCTTGGCACTGGCGTCG TGAGTGCCAGAAGCTGCCTCGATTCAACGAGGCAG TTTCTGGTACTC | 55 |
| **VEEV RBM RNA Target Sequence** | AGTGGCGGCTTAATTAAATTACACATGTCGGTGTG AGACTATAGTTAGTTGCGACGGGTACGTCGTTAAA AGAATAGCTATCAGTCCAGGCCTGTATGGGAAGCC TTCAGGCTATGCTGCTACGATGCACCGCGAGGGAT TCTTGTGCTGCAAAGTGACAGACACATTGAACGGG GAGAGGGTCTCTTTTCCCGTGTGCACGTATGTGCC AGCTACATTGTGTGACCAAATGACTGGCATACTGG CAACAGATGTCAGTGCGGACGACGCGCAAAAACTG CTGGTTGGGCTCAACCAGCGCATAGTCGTCAAAGC G | 56 |
| **L72AE RBM RNA Target Sequence** | CCCGGGTTCATTAGATCCTAAGGTGCTCTGACCGA AAGGCGTGATGAGCTGCAGGTCGACTCTAGAGCTC TGACCGAAAGGCGTGATGAGCTTGGCACTGGCGTC GTGCTCTGACCGAAAGGCGTGATGAGCTGCGGTAC CTTTAAGACCAATGACTTACA | 57 |
| **MS2 RBM RNA Target Sequence** | AAGGTAAACATGAGGATCACCCATGTCTGCAGGTC GACTCTAGAAAACATGAGGATCACCCATGTCTTGG CACTGGCGTCGTAAACATGAGGATCACCCATGTCT GCGGTACCTTTAAGA | 58 |

| **EXAMPLE CONSTRUCTS** | | |
|---|---|---|
| **PD-L1-targeted, PD1-ectodomain+linker VSVG-Nanoluc^{™}** | TMSVFACFPCLGLGSCWCAWCSVLCGLMLGEFMWV RQVPWSFTWAVLQLSWQSGWLLEVPNGPWRSLTFY PAWLTVSEGANATFTCSLSNWSEDLMLNWNRLSPS NQTEKQAAFCNGLSQPVQDARFQIIQLPNRHDFHM | 59 |
| | NILDTRRNDSGIYLCGAISLHPKAKIEESPGAELV VTERILETSTRYPSPSPKPEGRFQGMHHHHHHGGG GSGGGGSGGGGSGGTTTPAPRPPTPAPTIASQPLS LRPEACRPAAGGAVHTRGLDFACDLGGGGSDIGDT GLSKNPIELVEGWFSSWKSSIASFFFIIGLIIGLF LVLRVGIHLCIKLKHTKKRQIYTDIEMNRLGKVFT LEDFVGDWRQTAGYNLDQVLEQGGVSSLFQNLGVS VTPIQRIVLSGENGLKIDIHVIIPYEGLSGDQMGQ IEKIFKVVYPVDDHHFKVILHYGTLVIDGVTPNMI DYFGRPYEGIAVFDGKKITVTGTLWNGNKIIDERL INPDGSLLFRVTINGVTGWRLCERILADYKDDDDK | |
| **PD-L1-targeted, PD1-ectodomain VSVG-Nanoluc^{™}** | TMFMPSSLSYSSWATCWLLCCLIIILAKNSSTDPSR NSDTMKCLLYLAFLFIGVNCEFMWVRQVPWSFTWA VLQLSWQSGWLLEVPNGPWRSLTFYPAWLTVSEGA NATFTCSLSNWSEDLMLNWNRLSPSNQTEKQAAFC NGLSQPVQDARFQIIQLPNRHDFHMNILDTRRNDS GIYLCGAISLHPKAKIEESPGAELVVTERILETST RYPSPSPKPEGRFQGMHHHHHHDIGDTGLSKNPIE LVEGWFSSWKSSIASFFFIIGLIIGLFLVLRVGIH LCIKLKHTKKRQIYTDIEMNRLGKVFTLEDFVGDW RQTAGYNLDQVLEQGGVSSLFQNLGVSVTPIQRIV LSGENGLKIDIHVIIPYEGLSGDQMGQIEKIFKVV YPVDDHHFKVILHYGTLVIDGVTPNMIDYFGRPYE GIAVFDGKKITVTGTLWNGNKIIDERLINPDGSLL FRVTINGVTGWRLCERILADYKDDDDK | 60 |
| **PD-L1-targeted, PD1-ectodomain +large linker VSVG-Nanoluc^{™}** | TMEFGLSWVFLVALFRGVQCTGEFMWVRQVPWSFT WAVLQLSWQSGWLLEVPNGPWRSLTFYPAWLTVSE GANATFTCSLSNWSEDLMLNWNRLSPSNQTEKQAA FCNGLSQPVQDARFQIIQLPNRHDFHMNILDTRRN DSGIYLCGAISLHPKAKIEESPGAELVVTERILET STRYPSPSPKPEGRFQGMHHHHHHKFTIVFPHNQK GNWKNVPSNYHYCPSSSDLNWHNDLIGTALQVKMP KSHKAIQADGWMCHASKWVTTCDFRWYGPKYITHS IRSFTPSVEQCKESIEQTKQGTWLNPGFPPQSCGY ATVTDAEAVIVQVTPHHVLVDEYTGEWVDSQFING KCSNYICPTVHNSTTWHSDYKVKGLCDSNLISMDI | 61 |
| | TFFSEDGELSSLGKEGTGFRSNYFAYETGGKACKM QYCKHWGVRLPSGVWFEMADKDLFAAARFPECPEG SSISAPSQTSVDVSLIQDVERILDYSLCQETWSKI RAGLPISPVDLSYLAPKNPGTGPAFTIINGTLKYF ETRYIRVDIAAPILSRMVGMISGTTTERELWDDWA PYEDVEIGPNGVLRTSSGYKFPLYMIGHGMLDSDL HLSSKAQVFEHPHIQDAASQLPDDESLFFDIGDTG LSKNPIELVEGWFSSWKSSIASFFFIIGLIIGLFL VLRVGIHLCIKLKHTKKRQIYTDIEMNRLGKVFTL EDFVGDWRQTAGYNLDQVLEQGGVSSLFQNLGVSV TPIQRIVLSGENGLKIDIHVIIPYEGLSGDQMGQI EKIFKVVYPVDDHHFKVILHYGTLVIDGVTPNMID YFGRPYEGIAVFDGKKITVTGTLWNGNKIIDERLI NPDGSLLFRVTINGVTGWRLCERILADYKDDDDK | |
| **anti-CD3** | TMSVFACFPCLGLGSCWCAWCSVLCGLMLGDIEVQ LVESGGGLVQPGKSLKLSCEASGFTFSGYGMHWVR QAPGRGLESVAYITSSSINIKYADAVKGRFTVSRD NAKNLLFLQMNILKSEDTAMYYCARFDWDKNYWGQ GTMVTVSSGGGGSGGGGSGGGGSDIQMTQSPSSLP ASLGDRVTINCQASQDISNYLNWYQQKPGKAPKLL IYYTNKLADGVPSRFSGSGSGRDSSFTISSLESED IGSYYCQQYYNYPWTFGPGTKLEIKHHHHHHDIGD TGLSKNPIELVEGWFSSWKSSIASFFFIIGLIIGL FLVLRVGIHLCIKLKHTKKRQIYTDIEMNRLGKEF DYKDDDDK | 62 |
| **Bi-specific EV targeting human CEA and engaging human T cells (through CD3)** | TMAVEGGMKCVKFLLYVLLLAFCACAVGLIAIGVA VQVVLKQAITHETTAGSLLPVVIIAVGAFLFLVAF VGCCGACKENYCLMITFAIFLSLIMLVEVAVAIAG YVFRDQLKYPYDVPDYAEVQLVESGGGLVQPGKSL KLSCEASGFTFSGYGMHWVRQAPGRGLESVAYITS SSINIKYADAVKGRFTVSRDNAKNLLFLQMNILKS EDTAMYYCARFDWDKNYWGQGTMVTVSSGGGGSGG GGSGGGGSDIQMTQSPSSLPASLGDRVTINCQASQ DISNYLNWYQQKPGKAPKLLIYYTNKLADGVPSRF SGSGSGRDSSFTISSLESEDIGSYYCQQYYNYPWT FGPGTKLEIKGSVKSEFNKSFQQQMQNYLKDNKTA | 63 |
| | EFQVKLQQSGAELVRSGTSVKLSCTASGFNIKDSY MHWLRQGPEQCLEWIGWIDPENGDTEYAPKFQGKA TFTTDTSSNTAYLQLSSLTSEDTAVYYCNEGTPTG PYYFDYWGQGTTVTVSSGGGGSGGGGSGGGGSENV LTQSPAIMSASPGEKVTITCSASSSVSYMHWFQQK PGTSPKLWIYSTSNLASGVPARFSGSGSGTSYSLT ISRMEAEDAATYYCQQRSSYPLTFGCGTKLELKRH HHHHHDITILDKLQKENNCCGASNYTDWENIPGMA KDRVPDSCCINITVGCGNDFKESTIHTQGCVETIA IWLRKNILLVAAAALGIAFVEVLGIIFSCCLVKSI RSGYEVMSRDYKDDDDK | |
| **Bi-specific EV targeting murine and human FAP and engaging murine T cells (through CD3)** | TMAVEGGMKCVKFLLYVLLLAFCACAVGLIAIGVA VQVVLKQAITHETTAGSLLPVVIIAVGAFLFLVAF VGCCGACKENYCLMITFAIFLSLIMLVEVAVAIAG YVFRDQLKYPYDVPDYAEVQLVESGGGLVQPGKSL KLSCEASGFTFSGYGMHWVRQAPGRGLESVAYITS SSINIKYADAVKGRFTVSRDNAKNLLFLQMNILKS EDTAMYYCARFDWDKNYWGQGTMVTVSSGGGGSGG GGSGGGGSDIQMTQSPSSLPASLGDRVTINCQASQ DISNYLNWYQQKPGKAPKLLIYYTNKLADGVPSRF SGSGSGRDSSFTISSLESEDIGSYYCQQYYNYPWT FGPGTKLEIKGSVKSEFNKSFQQQMQNYLKDNKTA EFQVQLQESDPGLVKPSETLSLTCTVSGGSISSNN YYWGWIRQTPGKGLEWIGSIYYSGSTNYNPSLKSR VTISVDTSKNQFSLKLSSVTAADTAVYYCARGARW QARPATRIDGVAFDIWGQGTMVTVSSGGSSRSSSS GGGGSGGGGETTLTQSPGTLSLSPGERATLSCRAS QSVTRNYLAWYQQKPGQAPRLLMYGASNRAAGVPD RFSGSGSGTDFTLTISRLEPEDFAVYYCQQFGSPY TFGQGTKVEIKHHHHHHDITILDKLQKENNCCGAS NYTDWENIPGMAKDRVPDSCCINITVGCGNDFKES TIHTQGCVETIAIWLRKNILLVAAAALGIAFVEVL GIIFSCCLVKSIRSGYEVMSRDYKDDDDK | 64 |
| **Murine CLEC9a-targeted, VSVG-CdaA** | TMSVFACFPCLGLGSCWCAWCSVLCGLMLGDIEVK LQQSGTEVVKPGASVKLSCCKASGYIFTSYDIDWV RQTPEQGLEWIGWIFPGEGSTEYNEKFKGRATLSV | 65 |
| | DKSSSTAYMELTRLTSEDSAVYFCARGDYYRRYFD LWGQGTTVTVSSGGGGSGGGGSGGGGSGGGGSDIQ MTQSPSFLSTSLGNSITITCHASQNIKGWLAWYQQ KSGNAPQLLIYKASSLQSGVPSRFSGSGSGTDYIF TISNLQPEDIATYYCQHYQSFPWTFGGGTKLEIKR AAHHHHHHDIGDTGLSKNPIELVEGWFSSWKSSIA SFFFIIGLIIGLFLVLRVGIHLCIKLKHTKKRQIY TDIEMNRLGKEFDFSNMSILHYLANIVDILVVWFV IYKVIMLIRGTKAVQLLKGIFIIIAVKLLSGFFGL QTVEWITDQMLTWGFLAIIIIFQPELRRALETLGR GNIFTRYGSRIEREQHHLIESIEKSTQYMAKRRIG ALISVARDTGMDDYIETGIPLNAKISSQLLINIFI PNTPLHDGAVIIKGNEIASAASYLPLSDSPFLSKE LGTRHRAALGISEVTDSITIVVSEETGGISLTKGG ELFRDVSEEELHKILLKELVTVTAKKPSIFSKWKG GKSEEFDYKDDDDK | |
| **Murine CLEC9a-targeted, VSVG-mCherry** | TMSVFACFPCLGLGSCWCAWCSVLCGLMLGDIDIV MTQTPSSQAVSAGEKVTMNCKSSQSVLYDENKKNY LAWYQQKSGQSPKLLIYWASTGESGVPDRFIGSGS GTDFTLTISSVQAEDLAVYYCQQYYDFPPTFGGGT KGGSSRSSSSGGGGSGGGGQIVESGGGLVQPKESL KISCTASGFTFSNAAIYWVRQTPGKGLEWVGRIRT RPSKYATDYADSVRGRFTISRDDSKSMVYLQMDNL RTEDTAMYYCTPRATEDVPFYWGQGVMVTVSSHHH HHHDIGDTGLSKNPIELVEGWFSSWKSSIASFFFI IGLIIGLFLVLRVGIHLCIKLKHTKKRQIYTDIEM NRLGKEFMVSKGEEDNMAIIKEFMRFKVHMEGSVN GHEFEIEGEGEGRPYEGTQTAKLKVTKGGPLPFAW DILSPQFMYGSKAYVKHPADIPDYLKLSFPEGFKW ERVMNFEDGGVVTVTQDSSLQDGEFIYKVKLRGTN FPSDGPVMQKKTMGWEASSERMYPEDGALKGEIKQ RLKLKDGGHYDAEVKTTYKAKKPVQLPGAYNVNIK LDITSHNEDYTIVEQYERAEGRHSTGGMDELYKEF DYKDDDDK | 66 |
| **Murine CLEC9a-targeted, VSVG-Nanoluc^{™}** | TMSVFACFPCLGLGSCWCAWCSVLCGLMLGDIDIV MTQTPSSQAVSAGEKVTMNCKSSQSVLYDENKKNY | 67 |
| | LAWYQQKSGQSPKLLIYWASTGESGVPDRFIGSGS GTDFTLTISSVQAEDLAVYYCQQYYDFPPTFGGGT KGGSSRSSSSGGGGSGGGGQIVESGGGLVQPKESL KISCTASGFTFSNAAIYWVRQTPGKGLEWVGRIRT RPSKYATDYADSVRGRFTISRDDSKSMVYLQMDNL RTEDTAMYYCTPRATEDVPFYWGQGVMVTVSSHHH HHHDIGDTGLSKNPIELVEGWFSSWKSSIASFFFI IGLIIGLFLVLRVGIHLCIKLKHTKKRQIYTDIEM NRLGKEFVFTLEDFVGDWRQTAGYNLDQVLEQGGV SSLFQNLGVSVTPIQRIVLSGENGLKIDIHVIIPY EGLSGDQMGQIEKIFKVVYPVDDHHFKVILHYGTL VIDGVTPNMIDYFGRPYEGIAVFDGKKITVTGTLW NGNKIIDERLINPDGSLLFRVTINGVTGWRLCERI LAEFDYKDDDDK | |
| **Murine DEC205-targeted, VSVG-CdaA** | MRRMQLLLLIALSLALVTNSEVKLQQSGTEVVKPG ASVKLSCCKASGYIFTSYDIDWVRQTPEQGLEWIG WIFPGEGSTEYNEKFKGRATLSVDKSSSTAYMELT RLTSEDSAVYFCARGDYYRRYFDLWGQGTTVTVSS GGGGSGGGGSGGGGSGGGGSDIQMTQSPSFLSTSL GNSITITCHASQNIKGWLAWYQQKSGNAPQLLIYK ASSLQSGVPSRFSGSGSGTDYIFTISNLQPEDIAT YYCQHYQSFPWTFGGGTKLEIKRAAGGGGGDTGLS KNPIELVEGWFSSWKSSIASFFFIIGLIIGLFLVL RVGIHLCIKLKHTKKRQIYTDIEMNRLGKDFSNMS ILHYLANIVDILVVWFVIYKVIMLIRGTKAVQLLK GIFIIIAVKLLSGFFGLQTVEWITDQMLTWGFLAI IIIFQPELRRALETLGRGNIFTRYGSRIEREQHHL IESIEKSTQYMAKRRIGALISVARDTGMDDYIETG IPLNAKISSQLLINIFIPNTPLHDGAVIIKGNEIA SAASYLPLSDSPFLSKELGTRHRAALGISEVTDSI TIVVSEETGGISLTKGGELFRDVSEEELHKILLKE LVTVTAKKPSIFSKWKGGKSEDYKDDDDK | 68 |
| **Murine DEC205-targeted, VSVG-CdaA (2)** | TMSVFACFPCLGLGSCWCAWCSVLCGLMLGDIEVK LQQSGTEVVKPGASVKLSCCKASGYIFTSYDIDWV RQTPEQGLEWIGWIFPGEGSTEYNEKFKGRATLSV DKSSSTAYMELTRLTSEDSAVYFCARGDYYRRYFD | 69 |
| | LWGQGTTVTVSSGGGGSGGGGSGGGGSGGGGSDIQ MTQSPSFLSTSLGNSITITCHASQNIKGWLAWYQQ KSGNAPQLLIYKASSLQSGVPSRFSGSGSGTDYIF TISNLQPEDIATYYCQHYQSFPWTFGGGTKLEIKR AAHHHHHHDIGDTGLSKNPIELVEGWFSSWKSSIA SFFFIIGLIIGLFLVLRVGIHLCIKLKHTKKRQIY TDIEMNRLGKEFDFSNMSILHYLANIVDILVVWFV IYKVIMLIRGTKAVQLLKGIFIIIAVKLLSGFFGL QTVEWITDQMLTWGFLAIIIIFQPELRRALETLGR GNIFTRYGSRIEREQHHLIESIEKSTQYMAKRRIG ALISVARDTGMDDYIETGIPLNAKISSQLLINIFI PNTPLHDGAVIIKGNEIASAASYLPLSDSPFLSKE LGTRHRAALGISEVTDSITIVVSEETGGISLTKGG ELFRDVSEEELHKILLKELVTVTAKKPSIFSKWKG GKSEEFDYKDDDDK | |
| **Murine DEC205-targeted, VSVG-mCherry** | TMSVFACFPCLGLGSCWCAWCSVLCGLMLGDIEVK LQQSGTEVVKPGASVKLSCCKASGYIFTSYDIDWV RQTPEQGLEWIGWIFPGEGSTEYNEKFKGRATLSV DKSSSTAYMELTRLTSEDSAVYFCARGDYYRRYFD LWGQGTTVTVSSGGGGSGGGGSGGGGSGGGGSDIQ MTQSPSFLSTSLGNSITITCHASQNIKGWLAWYQQ KSGNAPQLLIYKASSLQSGVPSRFSGSGSGTDYIF TISNLQPEDIATYYCQHYQSFPWTFGGGTKLEIKR AAHHHHHHDIGDTGLSKNPIELVEGWFSSWKSSIA SFFFIIGLIIGLFLVLRVGIHLCIKLKHTKKRQIY TDIEMNRLGKEFMVSKGEEDNMAIIKEFMRFKVHM EGSVNGHEFEIEGEGEGRPYEGTQTAKLKVTKGGP LPFAWDILSPQFMYGSKAYVKHPADIPDYLKLSFP EGFKWERVMNFEDGGVVTVTQDSSLQDGEFIYKVK LRGTNFPSDGPVMQKKTMGWEASSERMYPEDGALK GEIKQRLKLKDGGHYDAEVKTTYKAKKPVQLPGAY NVNIKLDITSHNEDYTIVEQYERAEGRHSTGGMDE LYKEFDYKDDDDK | 70 |
| **Murine DEC205-targeted, VSVG-Nanoluc^{™}** | TMSVFACFPCLGLGSCWCAWCSVLCGLMLGDIEVK LQQSGTEVVKPGASVKLSCCKASGYIFTSYDIDWV RQTPEQGLEWIGWIFPGEGSTEYNEKFKGRATLSV | 71 |
| | DKSSSTAYMELTRLTSEDSAVYFCARGDYYRRYFD LWGQGTTVTVSSGGGGSGGGGSGGGGSGGGGSDIQ MTQSPSFLSTSLGNSITITCHASQNIKGWLAWYQQ KSGNAPQLLIYKASSLQSGVPSRFSGSGSGTDYIF TISNLQPEDIATYYCQHYQSFPWTFGGGTKLEIKR AAHHHHHHDIGDTGLSKNPIELVEGWFSSWKSSIA SFFFIIGLIIGLFLVLRVGIHLCIKLKHTKKRQIY TDIEMNRLGKEFVFTLEDFVGDWRQTAGYNLDQVL EQGGVSSLFQNLGVSVTPIQRIVLSGENGLKIDIH VIIPYEGLSGDQMGQIEKIFKVVYPVDDHHFKVIL HYGTLVIDGVTPNMIDYFGRPYEGIAVFDGKKITV TGTLWNGNKIIDERLINPDGSLLFRVTINGVTGWR LCERILAEFDYKDDDDK | |
| **Murine DEC205-targeted, mCD63-CdaA** | MAVEGGMKCVKFLLYVLLLAFCACAVGLIAIGVAV QVVLKQAITHETTAGSLLPVVIIAVGAFLFLVAFV GCCGACKENYCLMITFAIFLSLIMLVEVAVAIAGY VFRDQEVKLQQSGTEVVKPGASVKLSCCKASGYIF TSYDIDWVRQTPEQGLEWIGWIFPGEGSTEYNEKF KGRATLSVDKSSSTAYMELTRLTSEDSAVYFCARG DYYRRYFDLWGQGTTVTVSSGGGGSGGGGSGGGGS GGGGSDIQMTQSPSFLSTSLGNSITITCHASQNIK GWLAWYQQKSGNAPQLLIYKASSLQSGVPSRFSGS GSGTDYIFTISNLQPEDIATYYCQHYQSFPWTFGG GTKLEIKRAARKNILLVAAAALGIAFVEVLGIIFS CCLVKSIRSGYEVMGGGGDFSNMSILHYLANIVDI LVVWFVIYKVIMLIRGTKAVQLLKGIFIIIAVKLL SGFFGLQTVEWITDQMLTWGFLAIIIIFQPELRRA LETLGRGNIFTRYGSRIEREQHHLIESIEKSTQYM AKRRIGALISVARDTGMDDYIETGIPLNAKISSQL LINIFIPNTPLHDGAVIIKGNEIASAASYLPLSDS PFLSKELGTRHRAALGISEVTDSITIVVSEETGGI SLTKGGELFRDVSEEELHKILLKELVTVTAKKPSI FSKWKGGKSEDYKDDDDK | 72 |
| **Murine DEC205-targeted, VSVG-OVA** | TMSVFACFPCLGLGSCWCAWCSVLCGLMLGDIEVK LQQSGTEVVKPGASVKLSCCKASGYIFTSYDIDWV RQTPEQGLEWIGWIFPGEGSTEYNEKFKGRATLSV | 73 |
| | DKSSSTAYMELTRLTSEDSAVYFCARGDYYRRYFD LWGQGTTVTVSSGGGGSGGGGSGGGGSGGGGSDIQ MTQSPSFLSTSLGNSITITCHASQNIKGWLAWYQQ KSGNAPQLLIYKASSLQSGVPSRFSGSGSGTDYIF TISNLQPEDIATYYCQHYQSFPWTFGGGTKLEIKR AAHHHHHHDIGDTGLSKNPIELVEGWFSSWKSSIA SFFFIIGLIIGLFLVLRVGIHLCIKLKHTKKRQIY TDIEMNRLGKEFMGSIGAASMEFCFDVFKELKVHH ANENIFYCPIAIMSALAMVYLGAKDSTRTQINKVV RFDKLPGFGDSIEAQCGTSVNVHSSLRDILNQITK PNDVYSFSLASRLYAEERYPILPEYLQCVKELYRG GLEPINFQTAADQARELINSWVESQTNGIIRNVLQ PSSVDSQTAMVLVNAIVFKGLWEKAFKDEDTQAMP FRVTEQESKPVQMMYQIGLFRVASMASEKMKILEL PFASGTMSMLVLLPDEVSGLEQLESIINFEKLTEW TSSNVMEERKIKVYLPRMKMEEKYNLTSVLMAMGI TDVFSSSANLSGISSAESLKTSQAVHAAHAEINEA GREVVGSAEAGVDAASVSEEFRADHPFLFCIKHIA TNAVLFFGRCVSPEFDYKDDDDK | |
| **Anti-CD19+anti-CD20-CD63-mGZMB** | MAVEGGMKCVKFLLYVLLLAFCACAVGLIAIGVAV QVVLKQAITHETTAGSLLPVVIIAVGAFLFLVAFV GCCGACKENYCLMITFAIFLSLIMLVEVAVAIAGY VFRDQGPDIQMTQTTSSLSASLGDRVTISCRASQD ISKYLNWYQQKPDGTVKLLIYHTSRLHSGVPSRFS GSGSGTDYSLTISNLEQEDIATYFCQQGNTLPYTF GGGTKLEITGSTSGSGKPGSGEGSTKGEVKLQESG PGLVAPSQSLSVTCTVSGVSLPDYGVSWIRQPPRK GLEWLGVIWGSETTYYNSALKSRLTIIKDNSKSQV FLKMNSLQTDDTAIYYCAKHYYYGGSYAMDYWGQG TSVTVSSTYPYDVPDYAGPVKSEFNKSFQQQMQNY LKDNKTADIDIVMTQTPLSLPVTPGEPASISCRSS KSLLHSNGITYLYWYLQKPGQSPQLLIYQMSNLVS GVPDRFSGSGSGTDFTLKISRVEAEDVGVYYCAQN LELPYTFGGGTKVEIKRGGGGSGGGGSGGGGSQVQ LVQSGAEVKKPGSSVKVSCKASGYAFSYSWINWVR QAPGQGLEWMGRIFPGDGDTDYNGKFKGRVTITAD | 74 |
| | KSTSTAYMELSSLRSEDTAVYYCARNVFDGYWLVY WGQGTLVTVSSAHHHHHHDITILDKLQKENNCCGA SNYTDWENIPGMAKDRVPDSCCINITVGCGNDFKE STIHTQGCVETIAIWLRKNILLVAAAALGIAFVEV LGIIFSCCLVKSIRSGYEVMEFGEIIGGHEVKPHS RPYMALLSIKDQQPEAICGGFLIREDFVLTAAHCE GSIINVTLGAHNIKEQEKTQQVIPMVKCIPHPDYN PKTFSNDIMLLKLKSKAKRTRAVRPLNLPRRNVNV KPGDVCYVAGWGRMAPMGKYSNTLQEVELTVQKDR ECESYFKNRYNKTNQICAGDPKTKRASFRGDSGGP LVCKKVAAGIVSYGYKDGSPPRAFTKVNSFLSWIK KTMKSSEFDYKDDDDK | |
| **hGZMBR201K-linker-CD63-Anti-CD19+anti-CD20** | TMRARYKRIIGGHEAKPHSRPYMAYLMIWDQKSLK RCGGFLIRDDFVLTAAHCWGSSINVTLGAHNIKEQ EPTQQFIPVKRPIPHPAYNPKNFSNDIMLLQLERK AKRTRAVQPLRLPSNKAQVKPGQTCSVAGWGQTAP LGKHSHTLQEVKMTVQEDRKCESDLRHYYDSTIEL CVGDPEIKKTSFKGDSGGPLVCNKVAQGIVSYGKN NGMPPRACTKVSSFVHWIKKTMKRYGSSGSSGTRA RYKRGSSGSSGTAVEGGMKCVKFLLYVLLLAFCAC AVGLIAIGVAVQVVLKQAITHETTAGSLLPVVIIA VGAFLFLVAFVGCCGACKENYCLMITFAIFLSLIM LVEVAVAIAGYVFRDQGPDIQMTQTTSSLSASLGD RVTISCRASQDISKYLNWYQQKPDGTVKLLIYHTS RLHSGVPSRFSGSGSGTDYSLTISNLEQEDIATYF CQQGNTLPYTFGGGTKLEITGSTSGSGKPGSGEGS TKGEVKLQESGPGLVAPSQSLSVTCTVSGVSLPDY GVSWIRQPPRKGLEWLGVIWGSETTYYNSALKSRL TIIKDNSKSQVFLKMNSLQTDDTAIYYCAKHYYYG GSYAMDYWGQGTSVTVSSTYPYDVPDYAGPVKSEF NKSFQQQMQNYLKDNKTADIAQVQLVQSGAELVKP GASVKMSCKASGYTFTSYNMHWVKQTPGQGLEWIG AIYPGNGDTSYNQKFKGKATLTADKSSSTAYMQLS SLTSEDSAVYYCARAQLRPNYWYFDVWGAGTTVTV SKISGGGGSGGGGSGGGGSGGSSDIVLSQSPAILS ASPGEKVTMTCRASSSVSYMHWYQQKPGSSPKPWI | 75 |
| | YATSNLASGVPARFSGSGSGTSYSLTISRVEAEDA ATYYCQQWISNPPTFGAGTKLELKAHHHHHHDITI LDKLQKENNCCGASNYTDWENIPGMAKDRVPDSCC INITVGCGNDFKESTIHTQGCVETIAIWLRKNILL VAAAALGIAFVEVLGIIFSCCLVKSIRSGYEVMEF VFTLEDFVGDWRQTAGYNLDQVLEQGGVSSLFQNL GVSVTPIQRIVLSGENGLKIDIHVIIPYEGLSGDQ MGQIEKIFKVVYPVDDHHFKVILHYGTLVIDGVTP NMIDYFGRPYEGIAVFDGKKITVTGTLWNGNKIID ERLINPDGSLLFRVTINGVTGWRLCERILAEFDYK DDDDK | |
| **anti-CD20-CD63-mGZMB** | MAVEGGMKCVKFLLYVLLLAFCACAVGLIAIGVAV QVVLKQAITHETTAGSLLPVVIIAVGAFLFLVAFV GCCGACKENYCLMITFAIFLSLIMLVEVAVAIAGY VFRDQGPVKSEFNKSFQQQMQNYLKDNKTADIDIV MTQTPLSLPVTPGEPASISCRSSKSLLHSNGITYL YWYLQKPGQSPQLLIYQMSNLVSGVPDRFSGSGSG TDFTLKISRVEAEDVGVYYCAQNLELPYTFGGGTK VEIKRGGGGSGGGGSGGGGSQVQLVQSGAEVKKPG SSVKVSCKASGYAFSYSWINWVRQAPGQGLEWMGR IFPGDGDTDYNGKFKGRVTITADKSTSTAYMELSS LRSEDTAVYYCARNVFDGYWLVYWGQGTLVTVSSA HHHHHHDITILDKLQKENNCCGASNYTDWENIPGM AKDRVPDSCCINITVGCGNDFKESTIHTQGCVETI AIWLRKNILLVAAAALGIAFVEVLGIIFSCCLVKS IRSGYEVMEFGEIIGGHEVKPHSRPYMALLSIKDQ QPEAICGGFLIREDFVLTAAHCEGSIINVTLGAHN IKEQEKTQQVIPMVKCIPHPDYNPKTFSNDIMLLK LKSKAKRTRAVRPLNLPRRNVNVKPGDVCYVAGWG RMAPMGKYSNTLQEVELTVQKDRECESYFKNRYNK TNQICAGDPKTKRASFRGDSGGPLVCKKVAAGIVS YGYKDGSPPRAFTKVNSFLSWIKKTMKSSEFDYKD DDDK | 76 |
| **Anti-CD19-CD63-mGZMB** | MAVEGGMKCVKFLLYVLLLAFCACAVGLIAIGVAV QVVLKQAITHETTAGSLLPVVIIAVGAFLFLVAFV GCCGACKENYCLMITFAIFLSLIMLVEVAVAIAGY | 77 |
| | VFRDQGPDIQMTQTTSSLSASLGDRVTISCRASQD ISKYLNWYQQKPDGTVKLLIYHTSRLHSGVPSRFS GSGSGTDYSLTISNLEQEDIATYFCQQGNTLPYTF GGGTKLEITGSTSGSGKPGSGEGSTKGEVKLQESG PGLVAPSQSLSVTCTVSGVSLPDYGVSWIRQPPRK GLEWLGVIWGSETTYYNSALKSRLTIIKDNSKSQV FLKMNSLQTDDTAIYYCAKHYYYGGSYAMDYWGQG TSVTVSSTYPYDVPDYAGPVKSEFNKSFQQQMQNY LKDNKTADITILDKLQKENNCCGASNYTDWENIPG MAKDRVPDSCCINITVGCGNDFKESTIHTQGCVET IAIWLRKNILLVAAAALGIAFVEVLGIIFSCCLVK SIRSGYEVMEFGEIIGGHEVKPHSRPYMALLSIKD QQPEAICGGFLIREDFVLTAAHCEGSIINVTLGAH NIKEQEKTQQVIPMVKCIPHPDYNPKTFSNDIMLL KLKSKAKRTRAVRPLNLPRRNVNVKPGDVCYVAGW GRMAPMGKYSNTLQEVELTVQKDRECESYFKNRYN KTNQICAGDPKTKRASFRGDSGGPLVCKKVAAGIV SYGYKDGSPPRAFTKVNSFLSWIKKTMKSSEFDYK DDDDK | |
| **Anti-CD20-VSVG-PE38** | TMSVFACFPCLGLGSCWCAWCSVLCGLMLGDIAQV QLVQSGAELVKPGASVKMSCKASGYTFTSYNMHWV KQTPGQGLEWIGAIYPGNGDTSYNQKFKGKATLTA DKSSSTAYMQLSSLTSEDSAVYYCARAQLRPNYWY FDVWGAGTTVTVSKISGGGGSGGGGSGGGGSGGSS DIVLSQSPAILSASPGEKVTMTCRASSSVSYMHWY QQKPGSSPKPWIYATSNLASGVPARFSGSGSGTSY SLTISRVEAEDAATYYCQQWISNPPTFGAGTKLEL KAHHHHHHDIGDTGLSKNPIELVEGWFSSWKSSIA SFFFIIGLIIGLFLVLRVGIHLCIKLKHTKKRQIY TDIEMNRLGKEFRARYKRPTGAEFLGDGGDVSFST RGTQNWTVERLLQAHRQLEERGYVFVGYHGTFLEA AQSIVFGGVRARSQDLDAIWRGFYIAGDPALAYGY AQDQEPDARGRIRNGALLRVYVPRSSLPGFYRTGL TLAAPEAAGEVERLIGHPLPLRLDAITGPEEEGGR LETILGWPLAERTVVIPSAIPTDPRNVGGDLDPSS | 78 |
| | IPDKEQAISALPDYASQPGKPSREDLKEFDYKDDD DK | |
| **CTX-VSVG-mGZMB** | TMSVFACFPCLGLGSCWCAWCSVLCGLMLGDIMCM PCFTTDHQMARKCDDCCGGKGRGKCYGPQCLCRHH HHHHDIGDTGLSKNPIELVEGWFSSWKSSIASFFF IIGLIIGLFLVLRVGIHLCIKLKHTKKRQIYTDIE MNRLGKEFGEIIGGHEVKPHSRPYMALLSIKDQQP EAICGGFLIREDFVLTAAHCEGSIINVTLGAHNIK EQEKTQQVIPMVKCIPHPDYNPKTFSNDIMLLKLK SKAKRTRAVRPLNLPRRNVNVKPGDVCYVAGWGRM APMGKYSNTLQEVELTVQKDRECESYFKNRYNKTN QICAGDPKTKRASFRGDSGGPLVCKKVAAGIVSYG YKDGSPPRAFTKVNSFLSWIKKTMKSSEFDYKDDD DK | 79 |
| **CTX-VSVG-hGZMB R201K** | TMSVFACFPCLGLGSCWCAWCSVLCGLMLGDIMCM PCFTTDHQMARKCDDCCGGKGRGKCYGPQCLCRHH HHHHDIGDTGLSKNPIELVEGWFSSWKSSIASFFF IIGLIIGLFLVLRVGIHLCIKLKHTKKRQIYTDIE MNRLGKEFIIGGHEAKPHSRPYMAYLMIWDQKSLK RCGGFLIRDDFVLTAAHCWGSSINVTLGAHNIKEQ EPTQQFIPVKRPIPHPAYNPKNFSNDIMLLQLERK AKRTRAVQPLRLPSNKAQVKPGQTCSVAGWGQTAP LGKHSHTLQEVKMTVQEDRKCESDLRHYYDSTIEL CVGDPEIKKTSFKGDSGGPLVCNKVAQGIVSYGKN NGMPPRACTKVSSFVHWIKKTMKRYEFDYKDDDDK | 80 |
| **CTX-VSVG-Diphtheria Toxin** | TMSVFACFPCLGLGSCWCAWCSVLCGLMLGDIMCM PCFTTDHQMARKCDDCCGGKGRGKCYGPQCLCRHH HHHHDIGDTGLSKNPIELVEGWFSSWKSSIASFFF IIGLIIGLFLVLRVGIHLCIKLKHTKKRQIYTDIE MNRLGKEFMGADDVVDSSKSFVMENFSSYHGTKPG YVDSIQKGIQKPKSGTQGNYDDDWKGFYSTDNKYD AAGYSVDNENPLSGKAGGVVKVTYPGLTKVLALKV DNAETIKKELGLSLTEPLMEQVGTEEFIKRFGDGA SRVVLSLPFAEGSSSVEYINNWEQAKALSVELEIN FETRGKRGQDAMYEYMAQACAGNRVRRSVGSSLSC INLDWDVIRDKTKTKIESLKEHGPIKNKMSESPNK | 81 |
| | TVSEEKAKQYLEEFHQTALEHPELSELKTVTGTNP VFAGANYAAWAVNVAQVIDSETADNLEKTTAALSI LPGIGSVMGIADGAVHHNTEEIVAQSIALSSLMVA QAIPLVGELVDIGFAAYNFVESIINLFQVVHNSYN RPAYSPGHKTQPMHEFEFDYKDDDDK | |
| **Anti-FAP-VSVG-mGZMB** | MSVFACFPCLGLGSCWCAWCSVLCGLMLGDIQVQL QESDPGLVKPSETLSLTCTVSGGSISSNNYYWGWI RQTPGKGLEWIGSIYYSGSTNYNPSLKSRVTISVD TSKNQFSLKLSSVTAADTAVYYCARGARWQARPAT RIDGVAFDIWGQGTMVTVSSGGSSRSSSSGGGGSG GGGETTLTQSPGTLSLSPGERATLSCRASQSVTRN YLAWYQQKPGQAPRLLMYGASNRAAGVPDRFSGSG SGTDFTLTISRLEPEDFAVYYCQQFGSPYTFGQGT KVEIKHHHHHHDIGDTGLSKNPIELVEGWFSSWKS SIASFFFIIGLIIGLFLVLRVGIHLCIKLKHTKKR QIYTDIEMNRLGKEFGEIIGGHEVKPHSRPYMALL SIKDQQPEAICGGFLIREDFVLTAAHCEGSIINVT LGAHNIKEQEKTQQVIPMVKCIPHPDYNPKTFSND IMLLKLKSKAKRTRAVRPLNLPRRNVNVKPGDVCY VAGWGRMAPMGKYSNTLQEVELTVQKDRECESYFK NRYNKTNQICAGDPKTKRASFRGDSGGPLVCKKVA AGIVSYGYKDGSPPRAFTKVNSFLSWIKKTMKSSE FDYKDDDDK | 82 |
| **Anti-FAP-VSVG-hGZMB R201K** | MSVFACFPCLGLGSCWCAWCSVLCGLMLGDIQVQL QESDPGLVKPSETLSLTCTVSGGSISSNNYYWGWI RQTPGKGLEWIGSIYYSGSTNYNPSLKSRVTISVD TSKNQFSLKLSSVTAADTAVYYCARGARWQARPAT RIDGVAFDIWGQGTMVTVSSGGSSRSSSSGGGGSG GGGETTLTQSPGTLSLSPGERATLSCRASQSVTRN YLAWYQQKPGQAPRLLMYGASNRAAGVPDRFSGSG SGTDFTLTISRLEPEDFAVYYCQQFGSPYTFGQGT KVEIKHHHHHHDIGDTGLSKNPIELVEGWFSSWKS SIASFFFIIGLIIGLFLVLRVGIHLCIKLKHTKKR QIYTDIEMNRLGKEFIIGGHEAKPHSRPYMAYLMI WDQKSLKRCGGFLIRDDFVLTAAHCWGSSINVTLG AHNIKEQEPTQQFIPVKRPIPHPAYNPKNFSNDIM | 83 |
| | LLQLERKAKRTRAVQPLRLPSNKAQVKPGQTCSVA GWGQTAPLGKHSHTLQEVKMTVQEDRKCESDLRHY YDSTIELCVGDPEIKKTSFKGDSGGPLVCNKVAQG IVSYGKNNGMPPRACTKVSSFVHWIKKTMKRYEFD YKDDDDK | |
| **Anti-FAP-VSVG-Diphtheria Toxin** | MSVFACFPCLGLGSCWCAWCSVLCGLMLGDIQVQL QESDPGLVKPSETLSLTCTVSGGSISSNNYYWGWI RQTPGKGLEWIGSIYYSGSTNYNPSLKSRVTISVD TSKNQFSLKLSSVTAADTAVYYCARGARWQARPAT RIDGVAFDIWGQGTMVTVSSGGSSRSSSSGGGGSG GGGETTLTQSPGTLSLSPGERATLSCRASQSVTRN YLAWYQQKPGQAPRLLMYGASNRAAGVPDRFSGSG SGTDFTLTISRLEPEDFAVYYCQQFGSPYTFGQGT KVEIKHHHHHHDIGDTGLSKNPIELVEGWFSSWKS SIASFFFIIGLIIGLFLVLRVGIHLCIKLKHTKKR QIYTDIEMNRLGKEFMGADDVVDSSKSFVMENFSS YHGTKPGYVDSIQKGIQKPKSGTQGNYDDDWKGFY STDNKYDAAGYSVDNENPLSGKAGGVVKVTYPGLT KVLALKVDNAETIKKELGLSLTEPLMEQVGTEEFI KRFGDGASRVVLSLPFAEGSSSVEYINNWEQAKAL SVELEINFETRGKRGQDAMYEYMAQACAGNRVRRS VGSSLSCINLDWDVIRDKTKTKIESLKEHGPIKNK MSESPNKTVSEEKAKQYLEEFHQTALEHPELSELK TVTGTNPVFAGANYAAWAVNVAQVIDSETADNLEK TTAALSILPGIGSVMGIADGAVHHNTEEIVAQSIA LSSLMVAQAIPLVGELVDIGFAAYNFVESIINLFQ VVHNSYNRPAYSPGHKTQPMHEFEFDYKDDDDK | 84 |
| **TRAIL-VSVG-Diphtheria Toxin** | TMSVFACFPCLGLGSCWCAWCSVLCGLMLGDIAMM EVQGGPSLGQTCVLIVIFTVLLQSLCVAVTYVYFT NELKQMQDKYSKSGIACFLKEDDSYWDPNDEESMN SPCWQVKWQLRQLVRKMILRTSEETISTVQEKQQN ISPLVRERGPQRVAAHITGTRGRSNTLSSPNSKNE KALGRKINSWESSRSGHSFLSNLHLRNGELVIHEK GFYYIYSQTYFRFQEEIKENTKNDKQMVQYIYKYT SYPDPILLMKSARNSCWSKDAEYGLYSIYQGGIFE LKENDRIFVSVTNEHLIDMDHEASFFGAFLVGHHH | 85 |
| | HHHDIGDTGLSKNPIELVEGWFSSWKSSIASFFFI IGLIIGLFLVLRVGIHLCIKLKHTKKRQIYTDIEM NRLGKEFGEIIGGHEVKPHSRPYMALLSIKDQQPE AICGGFLIREDFVLTAAHCEGSIINVTLGAHNIKE QEKTQQVIPMVKCIPHPDYNPKTFSNDIMLLKLKS KAKRTRAVRPLNLPRRNVNVKPGDVCYVAGWGRMA PMGKYSNTLQEVELTVQKDRECESYFKNRYNKTNQ ICAGDPKTKRASFRGDSGGPLVCKKVAAGIVSYGY KDGSPPRAFTKVNSFLSWIKKTMKSSEFDYKDDDD K | |
| **TRAIL-VSVG-Diphtheria Toxin (2)** | TMSVFACFPCLGLGSCWCAWCSVLCGLMLGDIAMM EVQGGPSLGQTCVLIVIFTVLLQSLCVAVTYVYFT NELKQMQDKYSKSGIACFLKEDDSYWDPNDEESMN SPCWQVKWQLRQLVRKMILRTSEETISTVQEKQQN ISPLVRERGPQRVAAHITGTRGRSNTLSSPNSKNE KALGRKINSWESSRSGHSFLSNLHLRNGELVIHEK GFYYIYSQTYFRFQEEIKENTKNDKQMVQYIYKYT SYPDPILLMKSARNSCWSKDAEYGLYSIYQGGIFE LKENDRIFVSVTNEHLIDMDHEASFFGAFLVGHHH HHHDIGDTGLSKNPIELVEGWFSSWKSSIASFFFI IGLIIGLFLVLRVGIHLCIKLKHTKKRQIYTDIEM NRLGKEFIIGGHEAKPHSRPYMAYLMIWDQKSLKR CGGFLIRDDFVLTAAHCWGSSINVTLGAHNIKEQE PTQQFIPVKRPIPHPAYNPKNFSNDIMLLQLERKA KRTRAVQPLRLPSNKAQVKPGQTCSVAGWGQTAPL GKHSHTLQEVKMTVQEDRKCESDLRHYYDSTIELC VGDPEIKKTSFKGDSGGPLVCNKVAQGIVSYGKNN GMPPRACTKVSSFVHWIKKTMKRYEFDYKDDDDK | 86 |
| **TRAIL-VSVG-Diphtheria Toxin (3)** | TMSVFACFPCLGLGSCWCAWCSVLCGLMLGDIAMM EVQGGPSLGQTCVLIVIFTVLLQSLCVAVTYVYFT NELKQMQDKYSKSGIACFLKEDDSYWDPNDEESMN SPCWQVKWQLRQLVRKMILRTSEETISTVQEKQQN ISPLVRERGPQRVAAHITGTRGRSNTLSSPNSKNE KALGRKINSWESSRSGHSFLSNLHLRNGELVIHEK GFYYIYSQTYFRFQEEIKENTKNDKQMVQYIYKYT SYPDPILLMKSARNSCWSKDAEYGLYSIYQGGIFE | 87 |
| | LKENDRIFVSVTNEHLIDMDHEASFFGAFLVGHHH HHHDIGDTGLSKNPIELVEGWFSSWKSSIASFFFI IGLIIGLFLVLRVGIHLCIKLKHTKKRQIYTDIEM NRLGKEFMGADDVVDSSKSFVMENFSSYHGTKPGY VDSIQKGIQKPKSGTQGNYDDDWKGFYSTDNKYDA AGYSVDNENPLSGKAGGVVKVTYPGLTKVLALKVD NAETIKKELGLSLTEPLMEQVGTEEFIKRFGDGAS RVVLSLPFAEGSSSVEYINNWEQAKALSVELEINF ETRGKRGQDAMYEYMAQACAGNRVRRSVGSSLSCI NLDWDVIRDKTKTKIESLKEHGPIKNKMSESPNKT VSEEKAKQYLEEFHQTALEHPELSELKTVTGTNPV FAGANYAAWAVNVAQVIDSETADNLEKTTAALSIL PGIGSVMGIADGAVHHNTEEIVAQSIALSSLMVAQ AIPLVGELVDIGFAAYNFVESIINLFQVVHNSYNR PAYSPGHKTQPMHEFEFDYKDDDDK | |
| **Human CTLA4-targeted, VSVG-Diphtheria Toxin** | TMSVFACFPCLGLGSCWCAWCSVLCGLMLGDIIRR ADIVLTQSPGTLSLSPGERATLSCRASQSVGSSYL AWYQQKPGQAPRLLIYGAFSRATGIPDRFSGSGSG TDFTLTISRLEPEDFAVYYCQQYGSSPWTFGQGTK VEIKRGGGGSGGGGSGGGGSEAKLVESGGGVVQPG RSLRLSCAASGFTFSSYTMHWVRQAPGKGLEWVTF ISYDGNNKYYADSVKGRFTISRDNSKNTLYLQMNS LRAEDTAIYYCARTGWLGPFDYWGQGTLVTVSTAK TTPPSVYPLAPRSHHHHHHDIGDTGLSKNPIELVE GWFSSWKSSIASFFFIIGLIIGLFLVLRVGIHLCI KLKHTKKRQIYTDIEMNRLGKEFRARYKRMGADDV VDSSKSFVMENFSSYHGTKPGYVDSIQKGIQKPKS GTQGNYDDDWKGFYSTDNKYDAAGYSVDNENPLSG KAGGVVKVTYPGLTKVLALKVDNAETIKKELGLSL TEPLMEQVGTEEFIKRFGDGASRVVLSLPFAEGSS SVEYINNWEQAKALSVELEINFETRGKRGQDAMYE YMAQACAGNRVRRSVGSSLSCINLDWDVIRDKTKT KIESLKEHGPIKNKMSESPNKTVSEEKAKQYLEEF HQTALEHPELSELKTVTGTNPVFAGANYAAWAVNV AQVIDSETADNLEKTTAALSILPGIGSVMGIADGA VHHNTEEIVAQSIALSSLMVAQAIPLVGELVDIGF | 88 |
| | AAYNFVESIINLFQVVHNSYNRPAYSPGHKTEFDY KDDDDK | |
| **Murine CTLA4-targeted, VSVG-Diphtheria Toxin** | TMSVFACFPCLGLGSCWCAWCSVLCGLMLGDIMET DTLLLWVLLLWVPGSTGIRRADIVMTQTTLSLPVS LGDQASISCRSSQSIVHSNGNTYLEWYLQKPGQSP KLLIYKVSNRFSGVPDRFSGSGSGTDFTLKISRVE AEDLGVYYCFQGSHVPYTFGGGTKLEIKRGGGGSG GGGSGGGGSEAKLQESGPVLVKPGASVKMSCKASG YTFTDYYMNWVKQSHGKSLEWIGVINPYNGDTSYN QKFKGKATLTVDKSSSTAYMELNSLTSEDSAVYYC ARYYGSWFAYWGQGTLITVSTAKTTPPSVYPLAPR SHHHHHHDIGDTGLSKNPIELVEGWFSSWKSSIAS FFFIIGLIIGLFLVLRVGIHLCIKLKHTKKRQIYT DIEMNRLGKEFMGADDVVDSSKSFVMENFSSYHGT KPGYVDSIQKGIQKPKSGTQGNYDDDWKGFYSTDN KYDAAGYSVDNENPLSGKAGGVVKVTYPGLTKVLA LKVDNAETIKKELGLSLTEPLMEQVGTEEFIKRFG DGASRVVLSLPFAEGSSSVEYINNWEQAKALSVEL EINFETRGKRGQDAMYEYMAQACAGNRVRRSVGSS LSCINLDWDVIRDKTKTKIESLKEHGPIKNKMSES PNKTVSEEKAKQYLEEFHQTALEHPELSELKTVTG TNPVFAGANYAAWAVNVAQVIDSETADNLEKTTAA LSILPGIGSVMGIADGAVHHNTEEIVAQSIALSSL MVAQAIPLVGELVDIGFAAYNFVESIINLFQVVHN SYNRPAYSPGHKTQPMHEFEFDYKDDDDK | 89 |
| **CTX-VSVG-mCherry** | MSVFACFPCLGLGSCWCAWCSVLCGLMLGDIMCMP CFTTDHQMARKCDDCCGGKGRGKCYGPQCLCRHHH HHHDIGDTGLSKNPIELVEGWFSSWKSSIASFFFI IGLIIGLFLVLRVGIHLCIKLKHTKKRQIYTDIEM NRLGKEFMVSKGEEDNMAIIKEFMRFKVHMEGSVN GHEFEIEGEGEGRPYEGTQTAKLKVTKGGPLPFAW DILSPQFMYGSKAYVKHPADIPDYLKLSFPEGFKW ERVMNFEDGGVVTVTQDSSLQDGEFIYKVKLRGTN FPSDGPVMQKKTMGWEASSERMYPEDGALKGEIKQ RLKLKDGGHYDAEVKTTYKAKKPVQLPGAYNVNIK | 90 |
| | LDITSHNEDYTIVEQYERAEGRHSTGGMDELYKEF DYKDDDDK | |
| **CTX-CD63-Nanoluc^{™}** | TMAVEGGMKCVKFLLYVLLLAFCACAVGLIAIGVA VQVVLKQAITHETTAGSLLPVVIIAVGAFLFLVAF VGCCGACKENYCLMITFAIFLSLIMLVEVAVAIAG YVFRDQVKSEFNKSFQQQMQNYLKDNKTADIMCMP CFTTDHQMARKCDDCCGGKGRGKCYGPQCLCRHHH HHHDITILDKLQKENNCCGASNYTDWENIPGMAKD RVPDSCCINITVGCGNDFKESTIHTQGCVETIAIW LRKNILLVAAAALGIAFVEVLGIIFSCCLVKSIRS GYEVMEFVFTLEDFVGDWRQTAGYNLDQVLEQGGV SSLFQNLGVSVTPIQRIVLSGENGLKIDIHVIIPY EGLSGDQMGQIEKIFKVVYPVDDHHFKVILHYGTL VIDGVTPNMIDYFGRPYEGIAVFDGKKITVTGTLW NGNKIIDERLINPDGSLLFRVTINGVTGWRLCERI LAEFDYKDDDDK | 91 |
| **CTX-VSVG-Nanoluc^{™}** | TMSVFACFPCLGLGSCWCAWCSVLCGLMLGDIMCM PCFTTDHQMARKCDDCCGGKGRGKCYGPQCLCRHH HHHHDIGDTGLSKNPIELVEGWFSSWKSSIASFFF IIGLIIGLFLVLRVGIHLCIKLKHTKKRQIYTDIE MNRLGKEFVFTLEDFVGDWRQTAGYNLDQVLEQGG VSSLFQNLGVSVTPIQRIVLSGENGLKIDIHVIIP YEGLSGDQMGQIEKIFKVVYPVDDHHFKVILHYGT LVIDGVTPNMIDYFGRPYEGIAVFDGKKITVTGTL WNGNKIIDERLINPDGSLLFRVTINGVTGWRLCER ILAEFDYKDDDDK | 92 |
| **Anti-CD19+anti-CD20-CD63-mCherry** | MAVEGGMKCVKFLLYVLLLAFCACAVGLIAIGVAV QVVLKQAITHETTAGSLLPVVIIAVGAFLFLVAFV GCCGACKENYCLMITFAIFLSLIMLVEVAVAIAGY VFRDQGPDIQMTQTTSSLSASLGDRVTISCRASQD ISKYLNWYQQKPDGTVKLLIYHTSRLHSGVPSRFS GSGSGTDYSLTISNLEQEDIATYFCQQGNTLPYTF GGGTKLEITGSTSGSGKPGSGEGSTKGEVKLQESG PGLVAPSQSLSVTCTVSGVSLPDYGVSWIRQPPRK GLEWLGVIWGSETTYYNSALKSRLTIIKDNSKSQV FLKMNSLQTDDTAIYYCAKHYYYGGSYAMDYWGQG | 93 |
| | TSVTVSSTYPYDVPDYAGPVKSEFNKSFQQQMQNY LKDNKTADIAQVQLVQSGAELVKPGASVKMSCKAS GYTFTSYNMHWVKQTPGQGLEWIGAIYPGNGDTSY NQKFKGKATLTADKSSSTAYMQLSSLTSEDSAVYY CARAQLRPNYWYFDVWGAGTTVTVSKISGGGGSGG GGSGGGGSGGSSDIVLSQSPAILSASPGEKVTMTC RASSSVSYMHWYQQKPGSSPKPWIYATSNLASGVP ARFSGSGSGTSYSLTISRVEAEDAATYYCQQWISN PPTFGAGTKLELKAHHHHHHDITILDKLQKENNCC GASNYTDWENIPGMAKDRVPDSCCINITVGCGNDF KESTIHTQGCVETIAIWLRKNILLVAAAALGIAFV EVLGIIFSCCLVKSIRSGYEVMEFMVSKGEEDNMA IIKEFMRFKVHMEGSVNGHEFEIEGEGEGRPYEGT QTAKLKVTKGGPLPFAWDILSPQFMYGSKAYVKHP ADIPDYLKLSFPEGFKWERVMNFEDGGVVTVTQDS SLQDGEFIYKVKLRGTNFPSDGPVMQKKTMGWEAS SERMYPEDGALKGEIKQRLKLKDGGHYDAEVKTTY KAKKPVQLPGAYNVNIKLDITSHNEDYTIVEQYER AEGRHSTGGMDELYKEFDYKDDDDK | |
| **Anti-CD19+anti-CD20-CD63-Nanoluc^{™}** | MAVEGGMKCVKFLLYVLLLAFCACAVGLIAIGVAV QVVLKQAITHETTAGSLLPVVIIAVGAFLFLVAFV GCCGACKENYCLMITFAIFLSLIMLVEVAVAIAGY VFRDQGPDIQMTQTTSSLSASLGDRVTISCRASQD ISKYLNWYQQKPDGTVKLLIYHTSRLHSGVPSRFS GSGSGTDYSLTISNLEQEDIATYFCQQGNTLPYTF GGGTKLEITGSTSGSGKPGSGEGSTKGEVKLQESG PGLVAPSQSLSVTCTVSGVSLPDYGVSWIRQPPRK GLEWLGVIWGSETTYYNSALKSRLTIIKDNSKSQV FLKMNSLQTDDTAIYYCAKHYYYGGSYAMDYWGQG TSVTVSSTYPYDVPDYAGPVKSEFNKSFQQQMQNY LKDNKTADIDIVMTQTPLSLPVTPGEPASISCRSS KSLLHSNGITYLYWYLQKPGQSPQLLIYQMSNLVS GVPDRFSGSGSGTDFTLKISRVEAEDVGVYYCAQN LELPYTFGGGTKVEIKRGGGGSGGGGSGGGGSQVQ LVQSGAEVKKPGSSVKVSCKASGYAFSYSWINWVR QAPGQGLEWMGRIFPGDGDTDYNGKFKGRVTITAD | 94 |
| | KSTSTAYMELSSLRSEDTAVYYCARNVFDGYWLVY WGQGTLVTVSSAHHHHHHDITILDKLQKENNCCGA SNYTDWENIPGMAKDRVPDSCCINITVGCGNDFKE STIHTQGCVETIAIWLRKNILLVAAAALGIAFVEV LGIIFSCCLVKSIRSGYEVMEFVFTLEDFVGDWRQ TAGYNLDQVLEQGGVSSLFQNLGVSVTPIQRIVLS GENGLKIDIHVIIPYEGLSGDQMGQIEKIFKVVYP VDDHHFKVILHYGTLVIDGVTPNMIDYFGRPYEGI AVFDGKKITVTGTLWNGNKIIDERLINPDGSLLFR VTINGVTGWRLCERILAEFDYKDDDDK | |
| **Anti-mCTLA4- VSVG-mCherry** | MSVFACFPCLGLGSCWCAWCSVLCGLMLGDIMETD TLLLWVLLLWVPGSTGIRRADIVMTQTTLSLPVSL GDQASISCRSSQSIVHSNGNTYLEWYLQKPGQSPK LLIYKVSNRFSGVPDRFSGSGSGTDFTLKISRVEA EDLGVYYCFQGSHVPYTFGGGTKLEIKRGGGGSGG GGSGGGGSEAKLQESGPVLVKPGASVKMSCKASGY TFTDYYMNWVKQSHGKSLEWIGVINPYNGDTSYNQ KFKGKATLTVDKSSSTAYMELNSLTSEDSAVYYCA RYYGSWFAYWGQGTLITVSTAKTTPPSVYPLAPRS HHHHHHDIGDTGLSKNPIELVEGWFSSWKSSIASF FFIIGLIIGLFLVLRVGIHLCIKLKHTKKRQIYTD IEMNRLGKEFMVSKGEEDNMAIIKEFMRFKVHMEG SVNGHEFEIEGEGEGRPYEGTQTAKLKVTKGGPLP FAWDILSPQFMYGSKAYVKHPADIPDYLKLSFPEG FKWERVMNFEDGGVVTVTQDSSLQDGEFIYKVKLR GTNFPSDGPVMQKKTMGWEASSERMYPEDGALKGE IKQRLKLKDGGHYDAEVKTTYKAKKPVQLPGAYNV NIKLDITSHNEDYTIVEQYERAEGRHSTGGMDELY KEFDYKDDDDK | 95 |
| **Anti-mCTLA4- VSVG-Nanoluc^{™}** | MSVFACFPCLGLGSCWCAWCSVLCGLMLGDIMETD TLLLWVLLLWVPGSTGIRRADIVMTQTTLSLPVSL GDQASISCRSSQSIVHSNGNTYLEWYLQKPGQSPK LLIYKVSNRFSGVPDRFSGSGSGTDFTLKISRVEA EDLGVYYCFQGSHVPYTFGGGTKLEIKRGGGGSGG GGSGGGGSEAKLQESGPVLVKPGASVKMSCKASGY TFTDYYMNWVKQSHGKSLEWIGVINPYNGDTSYNQ | 96 |
| | KFKGKATLTVDKSSSTAYMELNSLTSEDSAVYYCA RYYGSWFAYWGQGTLITVSTAKTTPPSVYPLAPRS HHHHHHDIGDTGLSKNPIELVEGWFSSWKSSIASF FFIIGLIIGLFLVLRVGIHLCIKLKHTKKRQIYTD IEMNRLGKEFVFTLEDFVGDWRQTAGYNLDQVLEQ GGVSSLFQNLGVSVTPIQRIVLSGENGLKIDIHVI IPYEGLSGDQMGQIEKIFKVVYPVDDHHFKVILHY GTLVIDGVTPNMIDYFGRPYEGIAVFDGKKITVTG TLWNGNKIIDERLINPDGSLLFRVTINGVTGWRLC ERILAEFDYKDDDDK | |
| **anti-CEA-VSVG-mCherry** | TMSVFACFPCLGLGSCWCAWCSVLCGLMLGDIQVK LQQSGAELVRSGTSVKLSCTASGFNIKDSYMHWLR QGPEQCLEWIGWIDPENGDTEYAPKFQGKATFTTD TSSNTAYLQLSSLTSEDTAVYYCNEGTPTGPYYFD YWGQGTTVTVSSGGGGSGGGGSGGGGSENVLTQSP AIMSASPGEKVTITCSASSSVSYMHWFQQKPGTSP KLWIYSTSNLASGVPARFSGSGSGTSYSLTISRME AEDAATYYCQQRSSYPLTFGCGTKLELKRHHHHHH DIGDTGLSKNPIELVEGWFSSWKSSIASFFFIIGL IIGLFLVLRVGIHLCIKLKHTKKRQIYTDIEMNRL GKEFMVSKGEEDNMAIIKEFMRFKVHMEGSVNGHE FEIEGEGEGRPYEGTQTAKLKVTKGGPLPFAWDIL SPQFMYGSKAYVKHPADIPDYLKLSFPEGFKWERV MNFEDGGVVTVTQDSSLQDGEFIYKVKLRGTNFPS DGPVMQKKTMGWEASSERMYPEDGALKGEIKQRLK LKDGGHYDAEVKTTYKAKKPVQLPGAYNVNIKLDI TSHNEDYTIVEQYERAEGRHSTGGMDELYKEFDYK DDDDK | 97 |
| **anti-CEA- VSVG-Nanoluc^{™}** | TMSVFACFPCLGLGSCWCAWCSVLCGLMLGDIQVK LQQSGAELVRSGTSVKLSCTASGFNIKDSYMHWLR QGPEQCLEWIGWIDPENGDTEYAPKFQGKATFTTD TSSNTAYLQLSSLTSEDTAVYYCNEGTPTGPYYFD YWGQGTTVTVSSGGGGSGGGGSGGGGSENVLTQSP AIMSASPGEKVTITCSASSSVSYMHWFQQKPGTSP KLWIYSTSNLASGVPARFSGSGSGTSYSLTISRME AEDAATYYCQQRSSYPLTFGCGTKLELKRHHHHHH | 98 |
| | DIGDTGLSKNPIELVEGWFSSWKSSIASFFFIIGL IIGLFLVLRVGIHLCIKLKHTKKRQIYTDIEMNRL GKEFVFTLEDFVGDWRQTAGYNLDQVLEQGGVSSL FQNLGVSVTPIQRIVLSGENGLKIDIHVIIPYEGL SGDQMGQIEKIFKVVYPVDDHHFKVILHYGTLVID GVTPNMIDYFGRPYEGIAVFDGKKITVTGTLWNGN KIIDERLINPDGSLLFRVTINGVTGWRLCERILAE FDYKDDDDK | |
| **VAR2Δ-VSVG-mCherry** | MSVFACFPCLGLGSCWCAWCSVLCGLMLGDIRGSD LKSSNSRVTTPKVDVGNYKCLEYNDNKYGRKMTRA SGCSWQNMYEKRDDCTEENTPIKCQCAYKYGHPTN GLPSPVNVVVATNCQQLKSKDTEKNCKETTTYTTY TTWPAKDAGCINDDLVISLYSSPTTLGIDILHKFF SDIDSQVCKNEAANTTSSPGCNKTGAKRNRKADEI YKSYRKYIQDWRKVWSSGATGDGGKCDEIFKKYVE CKNKCETKCEGNCTGGSEKCSKCNEIVPKVKEQRQ KCFHEVWEQLFRLYQPILDITPIDDCSSGSGTVSG DGCCTTSNMGAGHKMALWIYKKNTNWWSERLEDLS SYSTDQEATNNKKIYKRFLKGFIKQLNLELDKTYE NDWISTGKILDGYDAFSYELAKCLKKGNDDNKKEH SPKLNKGEHFAAIIWEKLLESNTRINKLEQTKKGK EDLCVVLCLSQTRPPLGITNAYEKQLGGEKGSSKK WIWNKNNKKSQESKCKDCKYCNTLSALVKELNKEC AEQNKNNCSGNSSSGSKNDSCNEQLIRLFDQCCCN EVGSLSTHEIVCVRLDKDNERVGLKVHKCVKKKNA KISSHTICTKNSSPDSIQEQEVSAIGSENCNCVED NNKQIKESPNNADSSNLIFSLKTAYEAFYPDGKIY NHHHHHDIGDTGLSKNPIELVEGWFSSWKSSIAS FFFIIGLIIGLFLVLRVGIHLCIKLKHTKKRQIYT DIEMNRLGKEFMVSKGEEDNMAIIKEFMRFKVHME GSVNGHEFEIEGEGEGRPYEGTQTAKLKVTKGGPL PFAWDILSPQFMYGSKAYVKHPADIPDYLKLSFPE GFKWERVMNFEDGGVVTVTQDSSLQDGEFIYKVKL RGTNFPSDGPVMQKKTMGWEASSERMYPEDGALKG EIKQRLKLKDGGHYDAEVKTTYKAKKPVQLPGAYN | 99 |
| | VNIKLDITSHNEDYTIVEQYERAEGRHSTGGMDEL YKEFDYKDDDDK | |
| **VAR2Δ-VSVG-Nanoluc^{™}** | MSVFACFPCLGLGSCWCAWCSVLCGLMLGDIRGSD LKSSNSRVTTPKVDVGNYKCLEYNDNKYGRKMTRA SGCSWQNMYEKRDDCTEENTPIKCQCAYKYGHPTN GLPSPVNVVVATNCQQLKSKDTEKNCKETTTYTTY TTWPAKDAGCINDDLVISLYSSPTTLGIDILHKFF SDIDSQVCKNEAANTTSSPGCNKTGAKRNRKADEI YKSYRKYIQDWRKVWSSGATGDGGKCDEIFKKYVE CKNKCETKCEGNCTGGSEKCSKCNEIVPKVKEQRQ KCFHEVWEQLFRLYQPILDITPIDDCSSGSGTVSG DGCCTTSNMGAGHKMALWIYKKNTNWWSERLEDLS SYSTDQEATNNKKIYKRFLKGFIKQLNLELDKTYE NDWISTGKILDGYDAFSYELAKCLKKGNDDNKKEH SPKLNKGEHFAAIIWEKLLESNTRINKLEQTKKGK EDLCVVLCLSQTRPPLGITNAYEKQLGGEKGSSKK WIWNKNNKKSQESKCKDCKYCNTLSALVKELNKEC AEQNKNNCSGNSSSGSKNDSCNEQLIRLFDQCCCN EVGSLSTHEIVCVRLDKDNERVGLKVHKCVKKKNA KISSHTICTKNSSPDSIQEQEVSAIGSENCNCVED NNKQIKESPNNADSSNLIFSLKTAYEAFYPDGKIY NHHHHHDIGDTGLSKNPIELVEGWFSSWKSSIAS FFFIIGLIIGLFLVLRVGIHLCIKLKHTKKRQIYT DIEMNRLGKEFVFTLEDFVGDWRQTAGYNLDQVLE QGGVSSLFQNLGVSVTPIQRIVLSGENGLKIDIHV IIPYEGLSGDQMGQIEKIFKVVYPVDDHHFKVILH YGTLVIDGVTPNMIDYFGRPYEGIAVFDGKKITVT GTLWNGNKIIDERLINPDGSLLFRVTINGVTGWRL CERILAEFDYKDDDDK | 100 |
| **TRAIL-VSVG-mCherry** | TMSVFACFPCLGLGSCWCAWCSVLCGLMLGDIAMM EVQGGPSLGQTCVLIVIFTVLLQSLCVAVTYVYFT NELKQMQDKYSKSGIACFLKEDDSYWDPNDEESMN SPCWQVKWQLRQLVRKMILRTSEETISTVQEKQQN ISPLVRERGPQRVAAHITGTRGRSNTLSSPNSKNE KALGRKINSWESSRSGHSFLSNLHLRNGELVIHEK GFYYIYSQTYFRFQEEIKENTKNDKQMVQYIYKYT | 101 |
| | SYPDPILLMKSARNSCWSKDAEYGLYSIYQGGIFE LKENDRIFVSVTNEHLIDMDHEASFFGAFLVGHHH HHHDIGDTGLSKNPIELVEGWFSSWKSSIASFFFI IGLIIGLFLVLRVGIHLCIKLKHTKKRQIYTDIEM NRLGKEFMVSKGEEDNMAIIKEFMRFKVHMEGSVN GHEFEIEGEGEGRPYEGTQTAKLKVTKGGPLPFAW DILSPQFMYGSKAYVKHPADIPDYLKLSFPEGFKW ERVMNFEDGGVVTVTQDSSLQDGEFIYKVKLRGTN FPSDGPVMQKKTMGWEASSERMYPEDGALKGEIKQ RLKLKDGGHYDAEVKTTYKAKKPVQLPGAYNVNIK LDITSHNEDYTIVEQYERAEGRHSTGGMDELYKEF DYKDDDDK | |
| **TRAIL-VSVG-Nanoluc^{™}** | TMSVFACFPCLGLGSCWCAWCSVLCGLMLGDIAMM EVQGGPSLGQTCVLIVIFTVLLQSLCVAVTYVYFT NELKQMQDKYSKSGIACFLKEDDSYWDPNDEESMN SPCWQVKWQLRQLVRKMILRTSEETISTVQEKQQN ISPLVRERGPQRVAAHITGTRGRSNTLSSPNSKNE KALGRKINSWESSRSGHSFLSNLHLRNGELVIHEK GFYYIYSQTYFRFQEEIKENTKNDKQMVQYIYKYT SYPDPILLMKSARNSCWSKDAEYGLYSIYQGGIFE LKENDRIFVSVTNEHLIDMDHEASFFGAFLVGHHH HHHDIGDTGLSKNPIELVEGWFSSWKSSIASFFFI IGLIIGLFLVLRVGIHLCIKLKHTKKRQIYTDIEM NRLGKEFVFTLEDFVGDWRQTAGYNLDQVLEQGGV SSLFQNLGVSVTPIQRIVLSGENGLKIDIHVIIPY EGLSGDQMGQIEKIFKVVYPVDDHHFKVILHYGTL VIDGVTPNMIDYFGRPYEGIAVFDGKKITVTGTLW NGNKIIDERLINPDGSLLFRVTINGVTGWRLCERI LAEFDYKDDDDK | 102 |
| **anti- FAP-VSVG-mCherry** | MSVFACFPCLGLGSCWCAWCSVLCGLMLGDIQVQL QESDPGLVKPSETLSLTCTVSGGSISSNNYYWGWI RQTPGKGLEWIGSIYYSGSTNYNPSLKSRVTISVD TSKNQFSLKLSSVTAADTAVYYCARGARWQARPAT RIDGVAFDIWGQGTMVTVSSGGSSRSSSSGGGGSG GGGETTLTQSPGTLSLSPGERATLSCRASQSVTRN YLAWYQQKPGQAPRLLMYGASNRAAGVPDRFSGSG | 103 |
| | SGTDFTLTISRLEPEDFAVYYCQQFGSPYTFGQGT KVEIKHHHHHHDIGDTGLSKNPIELVEGWFSSWKS SIASFFFIIGLIIGLFLVLRVGIHLCIKLKHTKKR QIYTDIEMNRLGKEFMVSKGEEDNMAIIKEFMRFK VHMEGSVNGHEFEIEGEGEGRPYEGTQTAKLKVTK GGPLPFAWDILSPQFMYGSKAYVKHPADIPDYLKL SFPEGFKWERVMNFEDGGVVTVTQDSSLQDGEFIY KVKLRGTNFPSDGPVMQKKTMGWEASSERMYPEDG ALKGEIKQRLKLKDGGHYDAEVKTTYKAKKPVQLP GAYNVNIKLDITSHNEDYTIVEQYERAEGRHSTGG MDELYKEFDYKDDDDK | |
| **anti-FAP-VSVG-Nanoluc^{™}** | MSVFACFPCLGLGSCWCAWCSVLCGLMLGDIQVQL QESDPGLVKPSETLSLTCTVSGGSISSNNYYWGWI RQTPGKGLEWIGSIYYSGSTNYNPSLKSRVTISVD TSKNQFSLKLSSVTAADTAVYYCARGARWQARPAT RIDGVAFDIWGQGTMVTVSSGGSSRSSSSGGGGSG GGGETTLTQSPGTLSLSPGERATLSCRASQSVTRN YLAWYQQKPGQAPRLLMYGASNRAAGVPDRFSGSG SGTDFTLTISRLEPEDFAVYYCQQFGSPYTFGQGT KVEIKHHHHHHDIGDTGLSKNPIELVEGWFSSWKS SIASFFFIIGLIIGLFLVLRVGIHLCIKLKHTKKR QIYTDIEMNRLGKEFVFTLEDFVGDWRQTAGYNLD QVLEQGGVSSLFQNLGVSVTPIQRIVLSGENGLKI DIHVIIPYEGLSGDQMGQIEKIFKVVYPVDDHHFK VILHYGTLVIDGVTPNMIDYFGRPYEGIAVFDGKK ITVTGTLWNGNKIIDERLINPDGSLLFRVTINGVT GWRLCERILAEFDYKDDDDK | 104 |
| **aMARCO-SARS-2 Spike TMD-CdaA** | MRRMQLLLLIALSLALVTNSGGGGIRSLSSLQSLS TKDLTMGWICIIFLVATATGVLPQVKLLQSGAALV KPGASVKMSCKASGYTFTDYWVSWVKQSHGKSLEC IGEISPNSGTTNFNEKFKGKATLTVDKSTSTAYME LSRLTSEDSAIYYCTRCRYTTGVHYFDYWGQGVMV TVSSAETTAPSVYPLAPGTALKSNSMVTLGCLVKG YFPEPVTVTWNSGALSSGVHTFPAVLQSGLYTLTS SVTVPSSTWPSQTVTCNVAHPASSTKVDKKIEKGE FATYMAAGGGGSGGGGSGGGGSGGGGVIPLLISSP | 105 |
| | QNDESCQSLFLLLLWILGTKGDVVLTQTPSILSVT IGQSVSISCRSSQSLLDSDGNSYLYWFLQRPGQSP QRLIYLVSNLGSGVPNRFSGSGSGTDFTLKISGVE AEDLGVYYCMQATHAPWTFGGGTKLELKRADAAPT VSIFPPSTEQLATGGASVVCLMNNFYPRDISVKWK IDGTERRDGVLDSVTDQDSKDSTYSMSSTLSLTKA DYESHNLYTCEVVHKHHPHPGRIPATGAYGGGGGD TGLSKNPIELVEGWFSSWKSSIASFFFIIGLIIGL FLVLRVGIHLCIKLKHTKKRQIYTDIEMNRLGKGG GGMDFSNMSILHYLANIVDILVVWFVIYKVIMLIR GTKAVQLLKGIFIIIAVKLLSGFFGLQTVEWITDQ MLTWGFLAIIIIFQPELRRALETLGRGNIFTRYGS RIEREQHHLIESIEKSTQYMAKRRIGALISVARDT GMDDYIETGIPLNAKISSQLLINIFIPNTPLHDGA VIIKGNEIASAASYLPLSDSPFLSKELGTRHRAAL GISEVTDSITIVVSEETGGISLTKGGELFRDVSEE ELHKILLKELVTVTAKKPSIFSKWKGGKSEDYKDD DDK | |
| **aMARCO-SARS-2 Spike TMD-CdaA** | MRRMQLLLLIALSLALVTNSGGGGIRSLSSLQSLS TKDLTMGWICIIFLVATATGVLPQVKLLQSGAALV KPGASVKMSCKASGYTFTDYWVSWVKQSHGKSLEC IGEISPNSGTTNFNEKFKGKATLTVDKSTSTAYME LSRLTSEDSAIYYCTRCRYTTGVHYFDYWGQGVMV TVSSAETTAPSVYPLAPGTALKSNSMVTLGCLVKG YFPEPVTVTWNSGALSSGVHTFPAVLQSGLYTLTS SVTVPSSTWPSQTVTCNVAHPASSTKVDKKIEKGE FATYMAAGGGGSGGGGSGGGGSGGGGVIPLLISSP QNDESCQSLFLLLLWILGTKGDVVLTQTPSILSVT IGQSVSISCRSSQSLLDSDGNSYLYWFLQRPGQSP QRLIYLVSNLGSGVPNRFSGSGSGTDFTLKISGVE AEDLGVYYCMQATHAPWTFGGGTKLELKRADAAPT VSIFPPSTEQLATGGASVVCLMNNFYPRDISVKWK IDGTERRDGVLDSVTDQDSKDSTYSMSSTLSLTKA DYESHNLYTCEVVHKHHPHPGRIPATGAYGGGGGG WPWYIWLGFIAGLIAIVMVTIMLCCMTSCCSCLKG CCSCGSCCKFDEDDSEPVLKGVKLHYTGGGGMDFS | 106 |
| | NMSILHYLANIVDILVVWFVIYKVIMLIRGTKAVQ LLKGIFIIIAVKLLSGFFGLQTVEWITDQMLTWGF LAIIIIFQPELRRALETLGRGNIFTRYGSRIEREQ HHLIESIEKSTQYMAKRRIGALISVARDTGMDDYI ETGIPLNAKISSQLLINIFIPNTPLHDGAVIIKGN EIASAASYLPLSDSPFLSKELGTRHRAALGISEVT DSITIVVSEETGGISLTKGGELFRDVSEEELHKIL LKELVTVTAKKPSIFSKWKGGKSEDYKDDDDK | |
| **αLangerin-VSVG-CdaA** | MRRMQLLLLIALSLALVTNSAELVRPGASVTLSCK ASGYTFIDHDMHWVQQTPVYGLEWIGAIDPETGDT GYNQKFKGKAILTADKSSRTAYMELRSLTSEDSAV YYCTIPFYYSNYSPFAYWGQGTLVTVSGGGGSGGG GSGGGGSIVLSQSPAILSASPGEKVTMTCRASSSV SYMHWYQRKPGSSPKPWIYATSNLASGVPARFSGS GSGTSYSLTISRVEAEDAATYYCQQWSSNPLTFGA GTKLELGGGGGDTGLSKNPIELVEGWFSSWKSSIA SFFFIIGLIIGLFLVLRVGIHLCIKLKHTKKRQIY TDIEMNRLGKGGGGGMDFSNMSILHYLANIVDILV VWFVIYKVIMLIRGTKAVQLLKGIFIIIAVKLLSG FFGLQTVEWITDQMLTWGFLAIIIIFQPELRRALE TLGRGNIFTRYGSRIEREQHHLIESIEKSTQYMAK RRIGALISVARDTGMDDYIETGIPLNAKISSQLLI NIFIPNTPLHDGAVIIKGNEIASAASYLPLSDSPF LSKELGTRHRAALGISEVTDSITIVVSEETGGISL TKGGELFRDVSEEELHKILLKELVTVTAKKPSIFS KWKGGKSEDYKDDDDK | 107 |
| **amDEC205-VSVG-GsPCA Catalytic domain** | MAQVKLQESGTELAKPGAAVKEVKLQQSGTEVVKP GASVKLSCCKASGYIFTSYDIDWVRQTPEQGLEWI GWIFPGEGSTEYNEKFKGRATLSVDKSSSTAYMEL TRLTSEDSAVYFCARGDYYRRYFDLWGQGTTVTVS SGGGGSGGGGSGGGGSGGGGSDIQMTQSPSFLSTS LGNSITITCHASQNIKGWLAWYQQKSGNAPQLLIY KASSLQSGVPSRFSGSGSGTDYIFTISNLQPEDIA TYYCQHYQSFPWTFGGGTKLEIKRAAGGGGGDTGL SKNPIELVEGWFSSWKSSIASFFFIIGLIIGLFLV LRVGIHLCIKLKHTKKRQIYTDIEMNRLGKGGGGG | 108 |
| | LYVDELTGLFNYRYLDISLDRELKRADRFGSVVSM IFIDMDHFKGVNDTHGHLFGSQVLHEVGQLLKKSV REVDVIIRYGGDEFTIILVETGEKGAATVAERIRR SIEDHHFLASEGLDVRLTASLGYACYPLDTQSKME LLELADKAMYRGKEEGKNRVFRATAIRDYKDDDDK | |

It will be understood that functional variants are encompassed in some embodiments. The functional variant may comprise sequences that are at least 80% identical to the example sequences set forth in Table 13, wherein said variants retain substantially the same functional as the parent molecule from which they are derived. The functional variants may be at least 90% identical to the respective parent molecule. The functional variants may be at least 95% identical to the respective parent molecule. The functional variants may be at least 96% identical to the respective parent molecule. The functional variants may be at least 97% identical to the respective parent molecule. The functional variants may be at least 98% identical to the respective parent molecule. The functional variants may be at least 99% identical to the respective parent molecule. Likewise, certain embodiment encompass functional fragments that retain substantially the same functional as the full-length parent molecule from which they are derived.

In the preceding description, for purposes of explanation, numerous details are set forth in order to provide a thorough understanding of the embodiments. However, it will be apparent to one skilled in the art that these specific details are not required.

The above-described embodiments are intended to be examples only. Alterations, modifications and variations can be effected to the particular embodiments by those of skill in the art. The scope of the claims should not be limited by the particular embodiments set forth herein, but should be construed in a manner consistent with the specification as a whole.
All references referred to herein are incorporated by reference in their entireties.

### References

Stickney, Z., Losacco, J., McDevitt, S., Zhang, Z., and Lu, B. (2016) Development of exosome surface display technology in living human cells. Biochem Biophys Res Commun. 472(1): 53-9.
Meyer, C., Losacco, J., Stickney, Z., Li, L., Marriott, G., and Lu, B. (2017) Pseudotyping exosomes for enhanced protein delivery in mammalian cells. Int J Nanomedicine. 12:3153-3170.
U.S. Patent No. 10,617,768.
U.S. Patent No. 10,758,486.
U.S. Patent Application No. 16/900,383.

### Embodiments

The invention also provides the following embodiments:
1. A recombinant tumor-selective viral particle comprising a nucleic acid encoding a recombinant polypeptide for directing an extracellular vesicle (EV) to at least one target cell, said recombinant polypeptide comprising:
   - at least one targeting moiety for directing said EV to said at least one target molecule expressed by said at least one target cell,
   - at least one EV-anchoring polypeptide, and
   - at least one intravesicular polypeptide.
2. The recombinant tumor-selective viral particle of embodiment 1, which is of an oncolytic virus.
3. A recombinant polypeptide for directing an extracellular vesicle (EV) to at least one target cell comprising:
   - at least one targeting moiety for directing said EV to said at least one target molecule expressed by said at least one target cell,
   - at least one EV-anchoring polypeptide, and
   - at least one intravesicular polypeptide.
4. The recombinant polypeptide of embodiment 3, wherein said at least one EV-anchoring polypeptide comprises an EV-directed transmembrane polypeptide linked to said at least one targeting moiety.
5. The recombinant polypeptide of embodiment 4, wherein said EV-directed transmembrane polypeptide comprises a transmembrane domain from LAMP2b, VSVG, CD81, CD82, or LAMP1.
6. The recombinant polypeptide of embodiment 4, wherein said EV-directed transmembrane polypeptide comprises a transmembrane domain from Junin virus glycoprotein, Lassa fever virus glycoprotein, LCMV (lymphocytic choriomeningitis virus) glycoprotein, SARS-CoV-2 glycoprotein, Tamiami virus glycoprotein, Guanarito virus glycoprotein, Paraná virus glycoprotein, Machupo virus glycoprotein, Sabia virus glycoprotein or CdaA.
7. The recombinant polypeptide of any one of embodiments 3 to 6, wherein said at least one target cell comprises a mammalian cell.
8. The recombinant polypeptide of embodiment 7, wherein said mammalian cell is a human cell.
9. The recombinant polypeptide of any one of embodiments 3 to 8, wherein said at least one target cell is a tumor cell, a tumor stromal cell, or an immune cell.
10. The recombinant polypeptide of embodiment 9, wherein said tumor stromal cell comprises a cancer-associated fibroblast.
11. The recombinant polypeptide of embodiment 9, wherein said immune cell is a T-cell, a B-cell, a natural killer (NK) cell, a dendritic cell, a macrophage, or a neutrophil.
12. The recombinant polypeptide of embodiment 11, wherein said T-cell is a regulatory T-cell or a cytotoxic T cell.
13. The recombinant polypeptide of any one of embodiments 3 to 12, wherein said at least one target molecule is a cell surface marker or a cell surface receptor.
14. The recombinant polypeptide of any one of embodiments 3 to 13, wherein said at least one target molecule is a TNF-α family receptor, an integrin, a C-type lectin receptor, a leptin, a carcinoembryonic antigen, a CD antigens, a carbonic anhydrase, FAP, MMP2, DEC205, DC40, CLEC9, CD3, a glycosaminoglycan, a polysaccharide, or a lipid.
15. The recombinant polypeptide of any one of embodiments 3 to 14, wherein said at least one target molecule comprises a disease-specific cell surface molecule, which is:
   - expressed by a disease cell and not by a healthy control cell, or
   - expressed in a greater quantity by said disease cell compared to said healthy control cell.
16. The recombinant polypeptide of embodiment 15, wherein said disease-specific cell surface molecule comprises a tumor-associated antigen.
17. The recombinant polypeptide of any one of embodiments 3 to 16, wherein said at least one target molecule comprises DEC205, CLEC9A, CEACAM5, CTLA4, CD3, CD7, CD11c, CD19, CD20, CD22, CD40, CD44, CD206, EGFR, EGFRvIII, fibroblast activating protein (FAP), CA9, MMP-2, PD-L1, SIRPa, chondroitin sulfate, αv-Integrin, or folate receptor.
18. The recombinant polypeptide of any one of embodiments 3 to 17, wherein said at least one targeting moiety comprises a receptor ligand, an antibody or a functional fragment thereof, an scFv, a single domain antibody or a DARPin.
19. The recombinant polypeptide of embodiment 18, wherein said antibody is a single domain antibody.
20. The recombinant polypeptide of embodiment 18 or 19, wherein said antibody is a humanized antibody.
21. The recombinant polypeptide of embodiment 18, wherein said functional fragment is an Fab' or an F(ab')₂.
22. The recombinant polypeptide of embodiment 18, wherein said at least one targeting moiety comprises anti-DEC205, anti-Clec9A, anti-FAP, anti-CEA, anti-CA9, anti-CTL4, anti-CD3, anti-CD206, anti-EGFRViii, anti-CD19, anti-CD20, anti-CD22, anti-CD44, anti-CD7, SIRPα ectodomain, GE11 peptide, CTX, VAR2Δ,CD40 ligand, CD40-targeting peptide, iRGD, or PD1.
23. The recombinant polypeptide of any one of embodiments 3 to 22, wherein said intravesicular polypeptide comprises at least one EV payload polypeptide linked to said at least one targeting moiety via said EV-anchoring polypeptide.
24. The recombinant polypeptide of embodiment 23, wherein the at least one EV payload polypeptide comprises at least one EV therapeutic payload polypeptide.
25. The recombinant polypeptide of embodiment 3, wherein said EV-anchoring polypeptide and said intravesicular polypeptide together comprise an EV-directed recombinant tetraspanin comprising said at least one targeting moiety inserted between two transmembrane domains thereof.
26. The recombinant polypeptide of embodiment 25, wherein said recombinant tetraspanin comprises or is derived from human CD63 or CD9.
27. The recombinant polypeptide of embodiment 25 or 26, wherein said at least one target cell comprises a mammalian cell.
28. The recombinant polypeptide of embodiment 27, wherein said mammalian cell is a human cell.
29. The recombinant polypeptide of any one of embodiments 27 to 28, wherein said at least one target cell is a tumor cell, a tumor stromal cell, or an immune cell.
30. The recombinant polypeptide of embodiment 29, wherein said tumor stromal cell comprises a cancer-associated fibroblast.
31. The recombinant polypeptide of embodiment 29 wherein said immune cell is a T-cell, a B-cell, a natural killer (NK) cell, a dendritic cell, a macrophage, or a neutrophil.
32. The recombinant polypeptide of embodiment 31, wherein said T-cell is a regulatory T-cell or a cytotoxic T cell.
33. The recombinant polypeptide of any one of embodiments 25 to 32, wherein said at least one target molecule is a cell surface marker or a cell surface receptor.
34. The recombinant polypeptide of any one of embodiments 25 to 33, wherein said at least one target molecule is a TNF-α family receptor, an integrin, a C-type lectin receptor, a leptin, a carcinoembryonic antigen, a CD antigens, a carbonic anhydrase, FAP, MMP2, DEC205, DC40, CLEC9, CD3, a glycosaminoglycan, a polysaccharide, or a lipid.
35. The recombinant polypeptide of any one of embodiments 25 to 34, wherein said at least one target molecule comprises a disease-specific antigen, which is:
   - expressed by a disease cell and not by a healthy control cell, or
   - expressed in a greater quantity by said disease cell compared to said healthy control cell.
36. The recombinant polypeptide of embodiment 35, wherein said disease-specific antigen comprises a tumor-associated antigen.
37. The recombinant polypeptide of any one of embodiments 25 to 36, wherein said at least one target molecule comprises DEC205, CLEC9A, CEACAM5, CTLA4, CD3, CD7, CD11c, CD19, CD20, CD22, CD40, CD44, CD206, EGFR, EGFRvIII, fibroblast activating protein (FAP), CA9, MMP-2, PD-L1, SIRPa, chondroitin sulfate, αv-Integrin, or folate receptor.
38. The recombinant polypeptide of any one of embodiments 25 to 37, wherein said at least one targeting moiety comprises a receptor ligand, an antibody or a functional fragment thereof, an scFv, a single domain antibody or a DARPin.
39. The recombinant polypeptide of embodiment 38, wherein said antibody is a single domain antibody.
40. The recombinant polypeptide of embodiment 38 or 39, wherein said antibody is a humanized antibody.
41. The recombinant polypeptide of embodiment 38, wherein said functional fragment is an Fab' or an F(ab')₂.
42. The recombinant polypeptide of embodiment 38, wherein said at least one targeting moiety comprises anti-DEC205, anti-Clec9A, anti-FAP, anti-CEA, anti-CA9, anti-CTL4, anti-CD3, anti-CD206, anti-EGFRViii, anti-CD19, anti-CD20, anti-CD22, anti-CD44, anti-CD7, SIRPα ectodomain, GE11 peptide, CTX, VAR2Δ,CD40 ligand, CD40-targeting peptide, iRGD, or PD1.
43. The recombinant polypeptide of any one of embodiments 25 to 42, further comprising at least one EV payload polypeptide linked to an N- and/or C-terminus of said recombinant tetraspanin.
44. The recombinant polypeptide of embodiment 43, wherein the at least one EV payload polypeptide comprises at least one EV therapeutic polypeptide.
45. The recombinant polypeptide of embodiment 3,
   wherein said at least one targeting moiety comprises at least two targeting moieties,
   wherein said EV-anchoring polypeptide and said intravesicular polypeptide together comprise an EV-directed recombinant tetraspanin comprising four transmembrane domains numbered 1, 2, 3, and 4 from N- to C-terminus, wherein a first of said two targeting moieties is inserted between transmembrane domains 1 and 2, and a second of said two targeting moieties is inserted between transmembrane domains 3 and 4.
46. The recombinant polypeptide of embodiment 45, wherein said EV-directed recombinant tetraspanin is derived from human CD63 or CD9.
47. The recombinant polypeptide of embodiment 45 or 46, wherein said at least two targeting moieties specifically bind to at least two different target molecules.
48. The recombinant polypeptide of embodiment 47, wherein said at least two different target molecules are expressed by the same target cell.
49. The recombinant polypeptide of embodiment 48, wherein said target cell comprises a mammalian cell.
50. The recombinant polypeptide of embodiment 49, wherein said mammalian cell comprises a human cell.
51. The recombinant polypeptide of any one of embodiments 48 to 50, wherein said target cell comprises a tumor cell, a tumor stromal cell, or an immune cell.
52. The recombinant polypeptide of embodiment 51, wherein said tumor stromal cell comprises a cancer-associated fibroblast.
53. The recombinant polypeptide of embodiment 51, wherein said immune cell is a T-cell, a B-cell, a natural killer (NK) cell, a dendritic cell, a macrophage, or a neutrophil.
54. The recombinant polypeptide of embodiment 53, wherein said T-cell is a regulatory T cell or a cytotoxic T cell.
55. The recombinant polypeptide of any one of embodiments 48 to 54, wherein each of said at least two target molecules is a cell surface molecule.
56. The recombinant polypeptide of any one of embodiments 48 to 55, wherein each of said at least two target molecules is a TNF-α family receptor, an integrin, a C-type lectin receptor, a leptin, a carcinoembryonic antigen, a CD antigens, a carbonic anhydrase, FAP, MMP2, DEC205, DC40, CLEC9, CD3, a glycosaminoglycan, a polysaccharide, or a lipid.
57. The recombinant polypeptide of any one of embodiments 48 to 56, wherein each of said at least two target molecules comprise a disease-specific cell surface molecule, which is:
   - expressed by a disease cell and not by a healthy control cell, or
   - expressed in a greater quantity by said disease cell compared to said healthy control cell.
58. The recombinant polypeptide of embodiment 57, wherein said disease-specific cell surface molecule comprises a tumor-associated antigen.
59. The recombinant polypeptide of any one of embodiments 48 to 58, wherein each of said at least two target molecules independently comprises DEC205, CLEC9A, CEACAM5, CTLA4, CD3, CD7, CD11c, CD19, CD20, CD22, CD40, CD44, CD206, EGFR, EGFRvIII, fibroblast activating protein (FAP), CA9, MMP-2, PD-L1, SIRPa, chondroitin sulfate, αv-Integrin, or folate receptor.
60. The recombinant polypeptide of any one of embodiments 48 to 59, wherein each of said at least two targeting moieties independently comprises a receptor ligand, an antibody or a functional fragment thereof, an scFv, a single domain antibody, or a DARPin.
61. The recombinant polypeptide of embodiment 60, wherein said antibody is a single domain antibody.
62. The recombinant polypeptide of embodiment 60 or 61, wherein said antibody is a humanized antibody.
63. The recombinant polypeptide of embodiment 60, wherein said functional fragment is an Fab' or an F(ab')₂.
64. The recombinant polypeptide of embodiment 60, wherein said at least one targeting moiety comprises anti-DEC205, anti-Clec9A, anti-FAP, anti-CEA, anti-CA9, anti-CTL4, anti-CD3, anti-CD206, anti-EGFRViii, anti-CD19, anti-CD20, anti-CD22, anti-CD44, anti-CD7, SIRPα ectodomain, GE11 peptide, CTX, VAR2Δ,CD40 ligand, CD40-targeting peptide, iRGD, or PD1.
65. The recombinant polypeptide of embodiment 47, wherein said at least two different target molecules are expressed by different target cells.
66. The recombinant polypeptide of embodiment 65, wherein said different target cells comprise a disease cell and an immune cell, and wherein said at least two targeting moieties are directed, respectively, to a disease cell surface molecule and an immune cell surface molecule.
67. The recombinant polypeptide of embodiment 65, wherein said different target cells comprise a tumor cell and an immune cell, and wherein said at least two targeting moieties are directed, respectively, to a tumor cell surface molecule and an immune cell surface molecule.
68. The recombinant polypeptide of embodiment 67, wherein said immune cell is a T cell, and said immune cell surface molecule is a T cell surface molecule.
69. The recombinant polypeptide of embodiment 68, wherein said T cell is a regulatory T cell or a cytotoxic T cell.
70. The recombinant polypeptide of embodiment 67, wherein said immune cell is a natural killer (NK) cell, and said immune cell surface molecule is an NK cell surface molecule.
71. The recombinant polypeptide of embodiment 67, wherein said immune cell is a B cell, and said immune cell surface molecule is a B cell surface molecule.
72. The recombinant polypeptide of embodiment 67, wherein said immune cell is a macrophage, and said immune cell surface molecule is a macrophage cell surface molecule.
73. The recombinant polypeptide of embodiment 67, wherein said immune cell is a dendritic cell, and said immune cell surface molecule is a dendritic cell surface molecule.
74. The recombinant polypeptide of embodiment 67, wherein said immune cell is a neutrophil, and said immune cell surface molecule is a neutrophil cell surface molecule.
75. The recombinant polypeptide of any one of embodiments 67 to 74, wherein said tumor cell surface molecule comprises a tumor-associated antigen.
76. The recombinant polypeptide of any one of embodiments 67 to 74, wherein said tumor cell surface molecule comprises one of CEACAM5, CD19, CD20, CD22, EGFR, EGFRvIII, fibroblast activating protein (FAP), CA9, MMP-2, PD-L1, SIRPα, chondroitin sulfate, αv-Integrin, or folate receptor.
77. The recombinant polypeptide of any one of embodiments 65 to 76, wherein said at least one targeting moiety comprises an antibody or a functional fragment thereof, an scFv, a single domain antibody, or a DARPin.
78. The recombinant polypeptide of embodiment 77, wherein said antibody is a single domain antibody.
79. The recombinant polypeptide of embodiment 77 or 78, wherein said antibody is a humanized antibody.
80. The recombinant polypeptide of embodiment 77, wherein said functional fragment is an Fab' or an F(ab')₂.
81. The recombinant polypeptide of any one of embodiments 45 to 80, further comprising at least one EV payload polypeptide.
82. The recombinant polypeptide of embodiment 81, wherein the comprising at least one EV payload polypeptide comprises at least one EV therapeutic payload polypeptide.
83. The recombinant polypeptide of embodiment 82, wherein said at least one EV therapeutic payload polypeptide is linked to said N- and/or C-terminus of said recombinant tetraspanin.
84. The recombinant polypeptide of any one of embodiments 23, 43, and 81, wherein said at least one EV payload polypeptide is linked via a cleavage site for releasing said at least one EV therapeutic payload polypeptide.
85. The recombinant polypeptide of any one of embodiments 24, 44, and 82, wherein said at least one EV therapeutic payload polypeptide is linked via a cleavage site for releasing said at least one EV therapeutic payload polypeptide.
86. The recombinant polypeptide of embodiment 84 or 85, wherein said cleavage site comprises a self-cleavage peptide, a pH-dependent cleavage site, or a site for enzymatic cleavage.
87. The recombinant polypeptide of any one of embodiments 24, 44, and 82, wherein said EV therapeutic payload polypeptide comprises an active pharmaceutical ingredient (API).
88. The recombinant polypeptide of any one of embodiments 24, 44, and 82, wherein said EV therapeutic payload polypeptide comprises a cytotoxic molecule.
89. The recombinant polypeptide of embodiment 88, wherein said cytotoxic molecule comprises human GZMB R201K, murine GZMB, diphtheria toxin, a PE38 domain from Pseudomonas exotoxin A, or human TRAIL.
90. The recombinant polypeptide of any one of embodiments 24, 44, and 82, wherein said payload polypeptide comprises an immunomodulatory molecule.
91. The recombinant polypeptide of embodiment 90, wherein said immunomodulatory molecular comprises a STING or ERAdP pathway activator.
92. The recombinant polypeptide of embodiment 91, wherein said STING pathway activator comprises a bacterial dinucleotide cyclase.
93. The recombinant polypeptide of embodiment 92, wherein said bacterial dinucleotide cyclase comprises CdaA.
94. The recombinant polypeptide of any one of embodiments 24, 44, and 82, wherein said payload polypeptide comprises an enzyme.
95. The recombinant polypeptide of any one of embodiments 24, 44, and 82, wherein said payload polypeptide comprises a nucleic acid-binding domain.
96. The recombinant polypeptide of embodiment 95, wherein said nucleic acid binding domain comprises an RNA-binding motif.
97. The recombinant polypeptide of embodiment 96, wherein said RNA binding motif comprises a nucleic acid ligand system.
98. The recombinant polypeptide of any one of embodiments 24, 44, and 82, wherein said payload polypeptide comprises an antigen.
99. The recombinant polypeptide of embodiment 98, wherein said antigen is a tumor-associated antigen.
100. The recombinant polypeptide of embodiment 98, wherein said antigen is from a pathogen.
101. The recombinant polypeptide of any one of embodiments 98 to 100, wherein said EV therapeutic payload polypeptide further comprises an adjuvant.
102. The recombinant polypeptide of embodiment 101, wherein said adjuvant comprises a STING or ERAdP pathway activator.
103. The recombinant polypeptide of embodiment 102, wherein said STING pathway activator comprises a bacterial dinucleotide cyclase.
104. The recombinant polypeptide of embodiment 103, wherein said bacterial dinucleotide cyclase comprises CdaA.
104. The recombinant polypeptide of any one of embodiments 24, 44, and 82, wherein said at least one EV therapeutic payload polypeptide is linked to at least one further EV payload polypeptide.
105. The recombinant polypeptide of embodiment 104, wherein said at least one EV therapeutic payload polypeptide is linked to said at least one further EV payload polypeptide by a cleavage site.
106. The recombinant polypeptide of embodiment 104, wherein said at least one EV therapeutic payload polypeptide is separated from said at least one further EV payload polypeptide by at least two EV transmembrane domains.
107. A nucleic acid molecule encoding the recombinant polypeptide as defined in any one of embodiments 1 to 22, 25 to 42, and 45 to 80.
108. The nucleic acid of embodiment 107, wherein said nucleic acid further encodes a separate EV cargo molecule.
109. The nucleic acid of embodiment 108, wherein said EV cargo molecule comprises a nucleic acid.
110. The nucleic acid of embodiment 108, wherein said nucleic acid comprises an RNA.
111. The nucleic acid of embodiment 110, wherein said RNA comprises an mRNA, an miRNA, or an shRNA.
112. The nucleic acid of embodiment 108, wherein said EV cargo molecule comprises a polypeptide.
113. A nucleic acid molecule encoding the recombinant polypeptide as defined in any one of embodiments 24, 44, and 82 to 107.
114. The nucleic acid of embodiment 113, wherein said nucleic acid further encodes a separate EV cargo molecule.
115. The nucleic acid of embodiment 114, wherein said EV cargo molecule comprises a nucleic acid.
116. The nucleic acid of embodiment 115, wherein said nucleic acid comprises an RNA.
117. The nucleic acid of embodiment 116, wherein said RNA comprises an mRNA, an miRNA, or an shRNA.
118. The nucleic acid of embodiment 113, wherein said EV cargo molecule comprises a polypeptide.
119. A vector comprising the nucleic acid as defined in any one of embodiments 107 to 108.
120. A vector comprising the nucleic acid as defined in any one of embodiments 113 to 118.
121. A recombinant viral genome comprising the nucleic acid as defined in any one of embodiments 107 to 112, or a nucleic acid complementary thereto.
122. The recombinant viral genome of embodiment 121, wherein the viral genome is from a virus that is tumor-selective.
123. The recombinant viral genome of embodiment 121 or 122, wherein said viral genome is from an oncolytic virus.
124. The recombinant viral genome of embodiment 123, wherein said oncolytic virus is vesicular stomatitis virus (VSV), Vaccinia virus, Herpes virus simplex 1 (HSV-1), Herpes virus 2 (HSV-2), adenovirus.
125. A recombinant viral genome comprising the nucleic acid as defined in any one of embodiments 113 to 118, or a nucleic acid complementary thereto.
126. The recombinant viral genome of embodiment 125, wherein said viral genome is from a virus that is tumor-selective.
127. The recombinant viral genome of embodiment 125 or 126, wherein said viral genome is from an oncolytic virus.
128. The recombinant viral genome of embodiment 127, wherein said oncolytic virus is vesicular stomatitis virus (VSV), Vaccinia virus, Herpes virus simplex 1 (HSV-1), Herpes virus 2 (HSV-2), adenovirus].
129. A viral particle comprising the nucleic acid as defined in any one of embodiments 107 to 112, or a nucleic acid complementary thereto.
130. The viral particle of embodiment 129, wherein said viral particle is of a virus that is tumor-selective.
131. The viral particle of embodiment 129 or 130, wherein said viral particle is of an oncolytic virus.
132. The viral particle of embodiment 131, wherein said oncolytic virus is vesicular stomatitis virus (VSV), Vaccinia virus, Herpes virus simplex 1 (HSV-1), Herpes virus 2 (HSV-2), adenovirus].
133. A viral particle comprising the nucleic acid as defined in any one of embodiments 113 to 118, or a nucleic acid complementary thereto.
134. The viral particle of embodiment 133, wherein said viral particle is of a virus that is tumor-selective.
135. The viral particle of embodiment 133 or 134, wherein said viral particle is of an oncolytic virus.
136. The viral particle of embodiment 135, wherein said oncolytic virus is vesicular stomatitis virus (VSV), Vaccinia virus, Herpes virus simplex 1 (HSV-1), Herpes virus 2 (HSV-2), adenovirus.
137. A host cell comprising the nucleic acid as defined in any one of embodiments 107 to 104, the vector as defined in embodiment 119, the recombinant viral genome of any one of embodiments 121 to 124, or the viral particle as defined in any one of embodiments 129 to 132.
138. The host cell of embodiment 137, which is a prokaryotic cell.
139. The host cell of embodiment 137, which is a eukaryotic cell.
140. The host cell of embodiment 139, which is a yeast cell or an insect cell.
141. The host cell of embodiment 139, which is a mammalian cell.
142. The host cell of embodiment 141, which is a human cell.
143. The host cell of embodiment 137, which is an immune cell.
144. The host cell of embodiment 143, wherein said immune cell is a B cell, a T cell, a dendritic cell, a macrophage or a neutrophil.
145. The host cell of embodiment 144, which is a regulatory T cell or a cytotoxic T cell.
146. The host cell of any one of embodiments 137 to 145, wherein said host cell further encodes a separate EV cargo molecule.
147. The host cell of embodiment 146, wherein said EV cargo molecule comprises a nucleic acid.
148. The host cell of embodiment 147, wherein said nucleic acid comprises an RNA.
149. The host cell of embodiment 148, wherein said RNA comprises an mRNA, an miRNA, or an shRNA.
150. The host cell of embodiment 146, wherein said EV cargo molecule comprises a polypeptide.
151. A host cell comprising the nucleic acid as defined in any one of embodiments 113 to 118, the vector as defined in embodiment 120, the recombinant viral genome of any one of embodiments 125 to 128, or the viral particle as defined in any one of embodiments 130 to 136.
152. The host cell of embodiment 151, which is a prokaryotic cell.
153. The host cell of embodiment 151, which is a eukaryotic cell.
154. The host cell of embodiment 153, which is a yeast cell or an insect cell.
155. The host cell of embodiment 153, which is a mammalian cell.
156. The host cell of embodiment 155, which is a human cell.
157. The host cell of embodiment 151, which is an immune cell.
158. The host cell of embodiment 157, wherein said immune cell is a B cell, a T cell, a dendritic cell, a macrophage or a neutrophil.
159. The host cell of embodiment 158, which is a regulatory T cell or a cytotoxic T cell.
160. The host cell of any one of embodiments 151 to 159, wherein said host cell further encodes a separate EV cargo molecule.
161. The host cell of embodiment 160, wherein said EV cargo molecule comprises a nucleic acid.
162. The host cell of embodiment 161, wherein said nucleic acid comprises an RNA.
163. The host cell of embodiment 162, wherein said RNA comprises an mRNA, an miRNA, or an shRNA.
164. The host cell of embodiment 160, wherein said EV cargo molecule comprises a polypeptide.
165. Targeted extracellular vesicles (EVs) comprising the recombinant polypeptide as defined in any one of embodiments 1 to 22, 25 to 42, and 45 to 80.
166. The targeted EVs of embodiment 165, which further comprise a separate EV cargo molecule.
167. The targeted EVs of embodiment 166, wherein said EV cargo molecule comprises a nucleic acid.
168. The targeted EVs of embodiment 167, wherein said nucleic acid comprises an RNA.
169. The targeted EVs of embodiment 168, wherein said RNA comprises an mRNA, an miRNA, or an shRNA.
170. The targeted EVs of embodiment 167, wherein said EV cargo molecule comprises a polypeptide.
171. The targeted EVs of embodiment 166, wherein said EV cargo molecule comprises an API.
172. Targeted extracellular vesicles (EVs) comprising the recombinant polypeptide as defined in any one of embodiments 23, 24, 43, 44, and 81 to 106.
173. The targeted EVs of embodiment 172, which further comprise a separate EV cargo molecule.
174. The targeted EVs of embodiment 173, wherein said EV cargo molecule comprises a nucleic acid.
175. The targeted EVs of embodiment 174 wherein said nucleic acid comprises an RNA.
176. The targeted EVs of embodiment 175, wherein said RNA comprises an mRNA, an miRNA, or an shRNA.
177. The targeted EVs of embodiment 173, wherein said EV cargo molecule comprises a polypeptide.
178. The targeted EVs of embodiment 173, wherein said EV cargo molecule comprises an API.
179. The targeted EVs of any one of embodiments 165 to 178, which are exosomes.
180. The targeted EVs of any one of embodiments 165 to 178, which are microvesicles.
181. The targeted EVs of any one of embodiments 165 to 178, which are ectosomes.
182. The targeted EVs of any one of embodiments 165 to 178, which are apoptotic bodies.
183. A composition comprising the nucleic acid as defined in any one of embodiments 107 to 112, the vector as defined in embodiment 119, the recombinant viral genome of any one of embodiments 121 to 124, the viral particle as defined in any one of embodiments 129 to 132, or the targeted EVs of any one of embodiments 165 to 171; together with a pharmaceutically acceptable excipient, diluent, or carrier.
184. A composition comprising the nucleic acid as defined in any one of embodiments 113 to 117, the vector as defined in embodiment 120, the recombinant viral genome of any one of embodiments 125 to 129, the viral particle as defined in any one of embodiments 133 to 136, or the targeted EVs of any one of embodiments 172 to 176; together with a pharmaceutically acceptable excipient, diluent, or carrier.
185. A method of binding a targeting moiety to a target molecule of a target cell comprising contacting said target cell with the targeted EVs as defined in any one of embodiments 165 to 178.
186. A use of the targeted EVs as defined in any one of embodiments 165 to 178 for binding a targeting moiety to a target molecule of a target cell.
187. The targeted EVs as defined in any one of embodiments 165 to 178 for use in binding a targeting moiety to a target molecule of a target cell.
188. A method of delivering a payload molecule to a target cell comprising contacting said target cell with the targeted EVs as defined in any one of embodiments 172 to 178.
189. A use of the targeted EVs as defined in any one of embodiments 172 to 178 for delivering a payload molecule to a target cell.
190. The EVs as defined in any one of embodiments 172 to 178 for use in delivering a payload molecule to a target cell.
191. A method of delivering a cargo molecule to a target cell comprising contacting said target cell with the targeted EVs as defined in any one of embodiments 166 to 171 and 173 to 178.
192. A use of the targeted EVs as defined in any one of embodiments 166 to 171 and 173 to 178 for delivering a cargo molecule to a target cell.
193. The targeted EVs as defined in any one of embodiments 166 to 171 and 173 to 178 for use in delivering a cargo molecule to a target cell.
194. A method of stimulating an immune response to an antigen comprising administering to a subject the nucleic acid as defined in any one of embodiments 113 to 115, the vector as defined in embodiment 120, the recombinant viral genome of any one of embodiments 125 to 128, the viral particle as defined in any one of embodiments 133 to 136, or the targeted EVs of any one of embodiments 172 to 176, wherein said target cell comprises an immune cell, and wherein said at least one EV therapeutic payload polypeptide comprises an antigen.
195. The method of embodiment 194, wherein said antigen comprises a disease cell-specific antigen.
196. The method of embodiment 194, wherein said antigen comprises a tumor-specific antigen
197. The method of embodiment 194, wherein said antigen is from a pathogen.
198. The method of any one of embodiments 194 to 197, wherein said at least one EV therapeutic payload polypeptide further comprises an adjuvant.
199. A method of killing target cells comprising administering to a subject the nucleic acid as defined in any one of embodiments 113 to 118, the vector as defined in embodiment 120, the recombinant viral genome of any one of embodiments 125 to 129, the viral particle as defined in any one of embodiments 132 to 136, or the targeted EVs of any one of embodiments 172 to 184, wherein said at least one EV therapeutic payload polypeptide comprises a cytotoxic molecule.
200. The method of embodiment 199, wherein said cytotoxic molecule comprises human GZMB R201K, murine GZMB, diphtheria toxin, a PE38 domain from Pseudomonas exotoxin A, or human TRAIL.
201. The method of embodiment 199 or 200, wherein said target cell comprises a disease cell.
202. The method of embodiment 201, wherein said disease cell is a tumor cell.
203. A method of reprogramming immune cells comprising contacting the immune cells with the nucleic acid as defined in any one of embodiments 113 to 118, the vector as defined in embodiment 120, the recombinant viral genome of any one of embodiments 125 to 129, the viral particle as defined in any one of embodiments 132 to 136, or the targeted EVs of any one of embodiments 172 to 184, wherein the at least one EV therapeutic payload molecule comprises an immunomodulatory molecule.
204. The method of embodiment 203, wherein said immunomodulatory molecular comprises a STING or ERAdP pathway activator.
205. The method of embodiment 204, wherein said STING or ERAdP pathway activator comprises a bacterial dinucleotide cyclase.
206. The method of embodiment 205, wherein said bacterial dinucleotide cyclase comprises CdaA.
207. The method of any one of embodiments 203 to 206, wherein said immune cells comprise B cells, a T cells, NK cells, dendritic cells, macrophages, or neutrophils.
208. A method of directing an immune response to a target disease cell comprising administering to a subject the nucleic acid as defined in any one of embodiments 107 to 118, the vector as defined in embodiment 119 or 120, the recombinant viral genome of any one of embodiments 121 to 128, the viral particle as defined in any one of embodiments 129 to 136, or the targeted EVs of any one of embodiments 165 to 178, wherein said recombinant polypeptide comprises at least two targeting moieties which specifically bind, respectively, to at least two different target molecules, wherein said at least two different target molecules are expressed, respectively, by an immune cell and a disease cell.
209. The method of embodiment 208, wherein said disease cell comprises a tumor cell.
210. The method of embodiment 209, wherein one of said at least two different target molecules comprises a tumor-associated antigen.
211. The method of any one of embodiments 208 to 210, where said immune cell comprises a T-cell, a B-cell, a natural killer (NK) cell, a dendritic cell, a macrophage, or a neutrophil.
212. The method of embodiment 211, wherein said immune cell is a T cell.
213. The method of embodiment 212, wherein said immune cell is a regulatory T cell or a cytotoxic T cell.
214. The method of embodiment 211, wherein said immune cell is an NK cell.
215. A method of preparing therapeutic targeted EVs for a subject comprising:
   - contacting cells obtained from a subject with the nucleic acid as defined in any one of embodiments 107 to 118, the vector as defined in embodiment 119 or 120, the recombinant viral genome of any one of embodiments 121 to 128, or the viral particle as defined in any one of embodiments 129 to 136, and
   - collecting the targeted EVs.
216. The method of embodiment 215, wherein said cells are tumor cells.
217. The method of embodiment 215, wherein said cells are immune cells.
218. The method of embodiment 217, where said immune cells comprises T cells, B cells, natural killer (NK) cells, dendritic cells, macrophages, or neutrophils
219. A method of producing targeted EVs, wherein said method comprises:
   - expressing the nucleic acid as defined in any one of embodiments 107 to 118 in cells, or culturing the host cell as defined in any one of embodiments 137 to 164 to produce EVs, and
   - collecting EVs.

## Claims

1. A targeted extracellular vesicle (EV) comprising a recombinant polypeptide comprising:
- at least one targeting moiety for directing said EV to at least one target molecule expressed by at least one target cell,
- at least one EV-anchoring polypeptide, and
- at least one intravesicular polypeptide,
wherein the intravesicular polypeptide is selected from the group of: a polypeptide comprising a nucleic acid-binding domain; a polypeptide comprising a cytotoxic molecule; and a polypeptide comprising an immunomodulatory molecule such as a STING or ERAdP pathway activator.

2. The targeted EV of claim 1, wherein the intravesicular polypeptide comprises an RNA-binding motif such as an RNA-binding motif of Staufen-1.

3. The targeted EV of claim 1, wherein the intravesicular polypeptide comprises a cytotoxic molecule selected from human GZMB R201K, murine GZMB, diphtheria toxin, a PE38 domain from *Pseudomonas* exotoxin A, or human TRAIL.

4. The targeted EV of claim 1, wherein the intravesicular polypeptide comprises granzyme B.

5. The targeted EV of claim 1, wherein the intravesicular polypeptide comprises a STING pathway activator such as a bacterial dinucleotide cyclase.

6. The targeted EV of claim 5, wherein the bacterial dinucleotide cyclase comprises CdaA.

7. The targeted EV of any one of the preceding claims, wherein said at least one EV-anchoring polypeptide comprises an EV-directed transmembrane polypeptide linked to said at least one targeting moiety, optionally wherein said EV-directed transmembrane polypeptide comprises a transmembrane domain from LAMP2b, VSVG, CD81, CD82, a tetraspanin or LAMP1; or wherein said EV-directed transmembrane polypeptide optionally comprises a transmembrane domain from Junin virus glycoprotein, Lassa fever virus glycoprotein, LCMV (lymphocytic choriomeningitis virus) glycoprotein, SARS-CoV-2 glycoprotein, Tamiami virus glycoprotein, Guanarito virus glycoprotein, Paraná virus glycoprotein, Machupo virus glycoprotein, Sabia virus glycoprotein or CdaA.

8. The targeted EV of any one of the preceding claims, wherein said at least one target cell comprises a mammalian cell such as a tumor cell or an immune cell.

9. The targeted EV of any one of the preceding claims, wherein said at least one targeting moiety comprises a receptor ligand, an antibody or a functional fragment thereof, an scFv, a single domain antibody or a DARPin.

10. The targeted EV of any one of the preceding claims, wherein said intravesicular polypeptide is linked to said at least one targeting moiety via said EV-anchoring polypeptide, preferably wherein the intravesicular polypeptide comprises at least one EV therapeutic payload polypeptide.

11. The targeted EV of any one of the preceding claims, wherein said at least one targeting moiety comprises at least two targeting moieties, optionally wherein said at least two targeting moieties specifically bind to at least two different target molecules.

12. The targeted EV of any one of the preceding claims, wherein said EV comprises cargo separate from the recombinant polypeptide.

13. The targeted EV of claim 12, wherein the cargo comprises nucleic acid such as RNA selected from mRNA, miRNA or shRNA, wherein the RNA optionally binds to an RNA-binding motif on the recombinant polypeptide.

14. The targeted EV of claim 12, wherein the intravesicular polypeptide of the EV comprises an antigen such as a tumor-associated antigen or an antigen from a pathogen, and the cargo comprises an adjuvant such as a STING or ERAdP pathway activator.
